(19) Europäisches Patentamt
European Patent Office
Office européen des brevets

(11) EP 2 903 077 A1

(12) EUROPEAN PATENT APPLICATION
published in accordance with Art. 153(4) EPC

(43) Date of publication:
**05.08.2015 Bulletin 2015/32**

(21) Application number: **13842001.3**

(22) Date of filing: **10.09.2013**

(51) Int Cl.:
***H01M 14/00*** *(2006.01)* ***C09B 57/10*** *(2006.01)*
***C09B 67/44*** *(2006.01)* ***H01L 31/04*** *(2014.01)*

(86) International application number:
**PCT/JP2013/074295**

(87) International publication number:
**WO 2014/050528 (03.04.2014 Gazette 2014/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.09.2012 JP 2012218754**
**29.07.2013 JP 2013156800**

(71) Applicant: **FUJI-FILM Corporation**
**Minato-ku**
**Tokyo 106-8620 (JP)**

(72) Inventors:
- **NOMURA Kimiatsu**
**Ashigarakami-gun**
**Kanagawa 258-8577 (JP)**
- **SATO Hirotaka**
**Ashigarakami-gun**
**Kanagawa 258-8577 (JP)**
- **KOBAYASHI Katsumi**
**Ashigarakami-gun**
**Kanagawa 258-8577 (JP)**
- **WATANABE Kohsuke**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

# (54) PHOTOELECTRIC CONVERSION ELEMENT AND DYE-SENSITIZED SOLAR CELL

(57) A photoelectric conversion element includes a conductive substrate, a photosensitive layer containing an electrolyte, a charge transfer layer containing an electrolyte, and a counter electrode, in which the photosensitive layer contains semiconductor particles loaded with a metal complex dye represented by the following Formula (1). A dye-sensitized solar cell uses the photoelectric conversion element.

$$M(LD)(LA)(X)m \cdot CI \qquad \text{Formula (1)}$$

In the formula (1), M represents a divalent or trivalent metal ion; LD represents a bidentate or tridentate ligand having an aryl group or an heterocyclic group; and the ligand has 1 to 3 atoms that are coordinated with M by becoming an anion. LA represents a tridentate ligand represented by a specific formula; X represents a monodentate ligand; CI represents a counter ion necessary for neutralizing a charge; and m represents 0 or 1.

EP 2 903 077 A1

FIG. 1

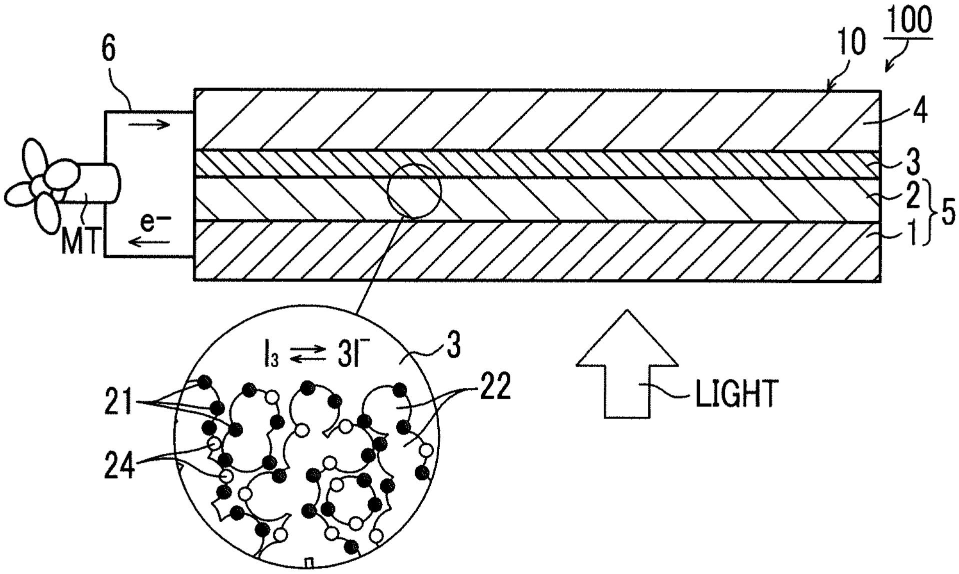

## Description

## BACKGROUND OF THE INVENTION

1. Field of the Invention

The present invention relates to a photoelectric conversion element and a dye-sensitized solar cell.

2. Description of the Related Art

Photoelectric conversion elements are used in various photosensors, copy machines, solar cells, and the like. The photoelectric conversion elements have been put to practical use in the form of photoelectric conversion elements adopting various modes, such as photoelectric conversion element using metals, photoelectric conversion elements using semiconductors, photoelectric conversion elements using organic pigments or dyes, or photoelectric conversion elements as a combination of these. Particularly, because solar cells using inexhaustible solar energy do not require fuels and use wasteless clean energy, full-scale commercialization of the solar cells is highly anticipated. Among the solar cells, silicon-based solar cells have been researched and developed for a long period of time, and are becoming increasingly popular by the political support of each country. However, because silicon is an inorganic material, the improvement of throughput, cost, and the like thereof is inevitably limited.

For this reason, dye-sensitized solar cells are being strenuously researched. Particularly, the research was fueled by the results of research conducted by Graetzel et al. of EPFL of Switzerland. By adopting a structure in which a sensitizing dye composed of a ruthenium complex is fixed to the surface of a thin film made of porous titanium oxide, Graetzel et al. realized a photoelectric conversion efficiency equivalent to that of amorphous silicon. As a result, the dye-sensitized solar cells that can be manufactured without using an expensive vacuum apparatus instantly drew attention of researchers all over the world.

Up to now, as metal complex dyes used in photoelectric conversion elements, dyes that are generally called N3, N719, Z907, and J2 have been developed. However, in many cases, the dye-sensitized solar cells in the related art generally have a low photoelectric conversion efficiency and poor durability.

Recently, a ruthenium metal complex dye has been developed which has excellent an absorption coefficient within a wavelength range of 450 nm to 550 nm, photoelectric conversion efficiency, and stability and contains terpyridin and 3-trifluoromethyl-5-[4-(p-substituted phenylethenyl)-2-pyridyl]pyrazol as ligands (see Chemical Communications, 2009, pp 5844-5846).

Furthermore, JP2012-36237A describes that if a specific substituent is introduced into a nitrogen-containing ring structure constituting a ligand of a metal complex dye, photoelectric conversion efficiency of a photoelectric conversion element is improved, and durability is further improved.

Moreover, KR10-2012-0035696A describes a ruthenium dye in which a specific electron-withdrawing substituent has been introduced into a tridentate ligand having a pyridine ring structure.

## SUMMARY OF THE INVENTION

Solar cells are increasingly drawing attention and being anticipated as an energy source alternative to nuclear power generation, and further improvement of performance is required for dye-sensitized solar cells. Photoelectric conversion elements using the metal complex dyes described in JP2012-36237A, KR10-2012-0035696A, and Chemical Communications, 2009, pp 5844-5846 are still unsatisfactory in terms of the photoelectric conversion efficiency and durability. An object of the present invention is to provide a photoelectric conversion element that makes a contribution to the further improvement of photoelectric conversion efficiency ($\eta$) and durability of a dye-sensitized solar cell, and a dye-sensitized solar cell using the photoelectric conversion element.

In order to achieve the above objects, the present inventors performed intensive examination. As a result, they found that in a metal complex dye which contains a ligand having a terpyridine structure having an adsorptive group to be adsorbed onto the surface of semiconductor particles and a specific bidentate or tridentate ligand having an aryl group or an heterocyclic group, if a specific electron-withdrawing group is introduced into the ligand having a terpyridine structure as a substituent, and at least one of the coordinating atoms in the bidentate or tridentate ligand having an aryl group or an heterocyclic group is turned into an anionic atom, photoelectric conversion efficiency ($\eta$) and durability of a dye-sensitized solar cell are improved. The present invention has been completed based on these findings.

That is, the objects of the present invention have been achieved by the following means.

[1] A photoelectric conversion element including a conductive substrate, a photosensitive layer containing an electrolyte, a charge transfer layer containing an electrolyte, and a counter electrode, in which the photosensitive layer contains semiconductor particles supporting a metal complex dye represented by the following Formula (1).

M(LD)(LA)(X)m·CI Formula (1)

In Formula (1), M represents a divalent or trivalent metal ion. LD represents a bidentate or tridentate ligand having an aryl group or an heterocyclic group, and the ligand has 1 to 3 atoms that are coordinated with M by becoming an anion. LA represents a tridentate ligand represented by the following Formula (2). X represents a monodentate ligand. CI represents a counter ion necessary for neutralizing a charge m represents 0 or 1.

[Chem. 1]

Formula (2)

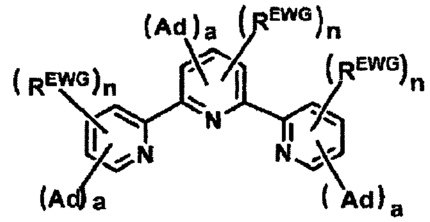

In Formula (2), Ad represents either an adsorptive group selected from among -COOH, $-SO_3H$, $-PO_3H_2$, -OH, -SH, and salts of these or a group having the adsorptive group. a represents an integer of 0 to 3, but all a do not represent 0 at the same time. $R^{EWG}$ represents an electron-withdrawing group selected from among $-NO_2$, $-SO_2R$, $-SO_3R$, -F, -Cl, -Br, -I, -CN, -COR, and a perfluoroalkyl group. R represents a group selected from among an alkyl group, an aryl group, an alkenyl group, an alkynyl group, a heterocyclic group, and an amino group. n represents an integer of equal to or greater than 0, but all n do not represent 0 at the same time.

[2] The photoelectric conversion element described in [1], in which LD in Formula (1) is a bidentate ligand represented by any of the following Formulae (2L-1) to (2L-4).

[Chem. 2]

Formula (2L-1)

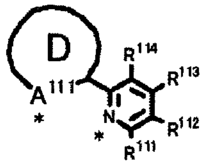

Formula (2L-2)

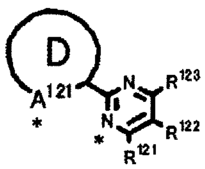

Formula (2L-3)

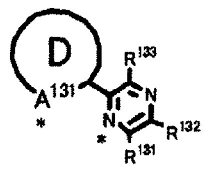

Formula (2L-4)

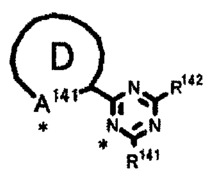

In Formulae (2L-1) to (2L-4), * represents a coordination position in which the ligand is coordinated with M. Each of $A^{111}$, $A^{121}$, $A^{131}$, and $A^{141}$ represents an anionic coordinating atom that is any of a nitrogen atom or a carbon atom. A ring D represents an aromatic hydrocarbon ring or a heterocyclic ring. Each of $R^{111}$ to $R^{114}$, $R^{121}$ to $R^{123}$, $R^{131}$ to $R^{133}$, and $R^{141}$ and $R^{142}$ independently represents a hydrogen atom or a substituent.

[3] The photoelectric conversion element described in [1], in which LD in Formula (1) represents a tridentate ligand represented by any of the following Formulae (3L-1) to (3L-6).

[Chem. 3]

Formula (3L-1)

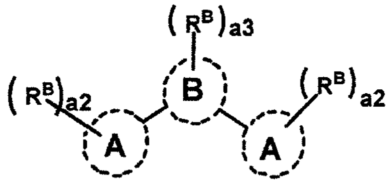

Formula (3L-2)

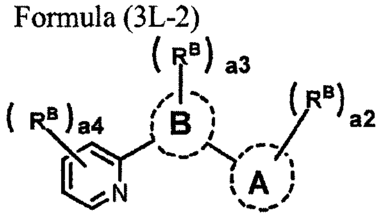

Formula (3L-3)

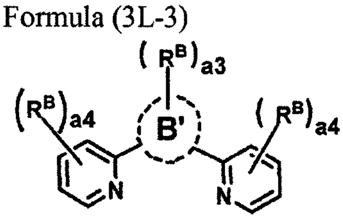

Formula (3L-4)

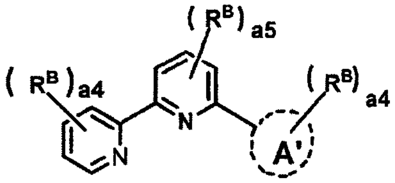

Formula (3L-5)

Formula (3L-6)

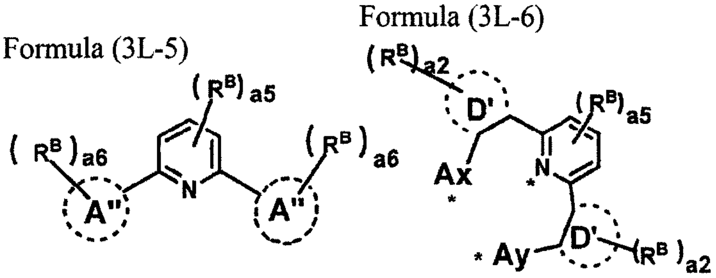

In Formulae (3L-1) to (3L-6), $R^B$ represents a substituent. Each of a2 and a3 independently represents an integer of equal to or greater than 0, and a4 represents an integer of 0 to 4. a5 represents an integer of 0 to 3. a6 represents an integer of 0 to 2.

A ring A represents a ring structure selected from among a pyrimidine ring, a pyrazine ring, a pyridazine ring, a triazine ring, a pyridine ring that is coordinated with M through a carbon atom, a thiophene ring that is coordinated with M through a carbon atom, a furan ring that is coordinated with M through a carbon atom, an imidazole ring, an oxazole ring, a thiazole ring, an oxadiazole ring, a thiadiazole ring, an isoxazole ring, an isothiazole ring, a triazole ring, a pyrazole ring, a pyrrole ring, and a benzene ring. A ring A' represents a ring structure selected from among a pyrimidine ring that is coordinated with M through a carbon atom, a pyrazine ring that is coordinated with M through a carbon atom, a pyridazine ring that is coordinated with M through a carbon atom, a pyridine ring that is coordinated with M through a carbon atom, a thiophene ring that is coordinated with M through a carbon atom, a furan ring that is coordinated with M through a carbon atom, an imidazole ring, an oxazole ring that is coordinated with M through a carbon atom, a thiazole ring that is coordinated with M through a carbon atom, an oxadiazole ring that is coordinated with M through a carbon atom, a thiadiazole ring that is coordinated with M through a carbon atom, an isoxazole ring that is coordinated with M through a carbon atom, an isothiazole ring that is coordinated with M through a carbon atom, a triazole ring, a pyrazole ring, a pyrrole ring, and a benzene ring. A ring A" represents a pyrazole ring.

A ring B represents a ring structure selected from among a pyrimidine ring, a triazine ring, an imidazole ring, an oxazole ring, a thiazole ring, an oxadiazole ring, a thiadiazole ring, a triazole ring, a pyrazole ring, a pyrrole ring, and a benzene ring. A ring B' represents a ring structure selected from among a pyrimidine ring that is coordinated with M through a carbon atom, an imidazole ring, an oxazole ring that is coordinated with M through a carbon atom, a thiazole ring that is coordinated with M through a carbon atom, an oxadiazole ring that is coordinated with M through a carbon atom, a thiadiazole ring that is coordinated with M through a carbon atom, a triazole ring, a pyrazole ring, a pyrrole ring, and a benzene ring.

A ring D' represents an aromatic hydrocarbon ring or a heterocyclic ring. Each of Ax and Ay independently represents a nitrogen atom, an oxygen atom, or a sulfur atom. Here, at least one of Ax and Ay is an anion.

Herein, two ring As present in Formula (3L-1) may be the same as or different from each other, and two ring D's present in Formula (3L-6) may be the same as or different from each other.

[4] The photoelectric conversion element described in any one of [1] to [3], in which the adsorptive group or the group having the adsorptive group and the electron-withdrawing group are present on different pyridine rings in Formula (2).

[5] The photoelectric conversion element described in any one of [1] to [4], in which the group having the adsorptive group is a group selected from the group consisting of an alkyl group and an amino group, and the group has the adsorptive group as a substituent.

[6] The photoelectric conversion element described in any one of [1] to [5], in which the adsorptive group is -COOH or a salt thereof.

[7] The photoelectric conversion element described in any one of [1] to [6], in which M in Formula (1) is $Ru^{2+}$, $Fe^{2+}$, or $Os^{2+}$.

[8] The photoelectric conversion element described in any one of [1] to [7], in which the semiconductor particles are loaded with a coadsorbent having at least one adsorptive group.

[9] The photoelectric conversion element described in [8], in which the coadsorbent is represented by the following Formula (CA).

[Chem. 4]

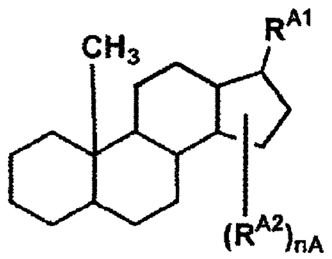

Formula (CA)

In Formula (CA), $R^{A1}$ represents a substituent having an adsorptive group. $R^{A2}$ represents a substituent. nA represents an integer of equal to or greater than 0.
[10] A dye-sensitized solar cell using the photoelectric conversion element described in any one of [1] to [9].
In the present specification, unless otherwise specified, regarding a carbon-carbon double bond, when an E-isomer and a Z-isomer are present in a molecule, unless otherwise specified, the double bond may form either the E-isomer or the Z-isomer, or form a mixture thereof.
In the present specification, when there are a plurality of substituents, linking groups, ligands, ring structures, and the like (hereinafter, referred to as "substituents and the like) marked with a specific sign, or when the plurality of substituents and the like are specified collectively or selectively, unless otherwise specified, the substituents and the like may be the same as or different from each other. The same will be applied to the case of specifying the number of the substituents and the like. Furthermore, when the plurality of substituents are close to each other (particularly, when they are adjacent to each other), unless otherwise specified, the substituents may form a ring by binding to each other. Moreover, rings, for example, an alicyclic ring, an aromatic ring, or a heterocyclic ring may form a condensed ring by being further condensed. In the present specification, the meaning of the term "ring" includes a condensed ring. In addition, in the present specification, examples of the "group having an adsorptive group" or the "substituent having an adsorptive group" include the groups described regarding Ad which will be described later.
In the present specification, unless otherwise specified, each of the substituents may further have a substituent.
The photoelectric conversion element and the dye-sensitized solar cell of the present invention have an excellent photoelectric conversion efficiency (η) and durability.
If the metal complex dye used in the present invention is used as a photosensitive layer of a photoelectric conversion element by being supported on the surface of semiconductor particles, the photoelectric conversion efficiency (η) and durability of the dye-sensitized solar cell using the photoelectric conversion element can be further improved.

## BRIEF DESCRIPTION OF THE DRAWINGS

Fig. 1 is a cross-sectional view schematically showing an embodiment of a photoelectric conversion element of the present invention, including an enlarged view showing the interior of the layer.
Fig. 2 is a cross-sectional view schematically showing a dye-sensitized solar cell prepared in Example 1.

## DESCRIPTION OF THE PREFERRED EMBODIMENTS

«Photoelectric conversion element and dye-sensitized solar cell»

The photoelectric conversion element of the present invention is formed by using semiconductor particles supporting the metal complex dye represented by Formula (1), which will be described later. The dye-sensitized solar cell of the present invention is formed by using the photoelectric conversion element, and includes a conductive substrate. On the conductive substrate, a photosensitive layer that contains semiconductor particles supporting the metal complex dye represented by Formula (1) which will be described later, a charge transfer layer that contains an electrolyte, and a counter electrode are disposed in this order. Each of the layers may be constituted with a single layer or multiple layers,

and if necessary, the dye-sensitized solar cell may further include layers other than each of the aforementioned layers.

For example, the photoelectric conversion element and the dye-sensitized solar cell of the present invention can be in the form of the embodiment shown in Fig. 1. A photoelectric conversion element 10 shown in Fig 1 includes a conductive substrate 1, a photosensitive layer 2 containing semiconductor particles that is disposed on the conductive substrate 1 and has been sensitized with a dye 21, a charge transfer layer 3, and a counter electrode 4. The conductive substrate 1, in which the photosensitive layer 2 is installed, functions as a working electrode in the photoelectric conversion element 10. In the present embodiment, the photoelectric conversion element 10 is described in the form of a dye-sensitized solar cell system 100 which can be obtained when the photoelectric conversion element 10 is used as a battery that makes operating means MT work through an external circuit 6.

In the present embodiment, a light-receiving electrode 5 is composed of the conductive substrate 1 and the photosensitive layer 2 that contains semiconductor particles 22 supporting (having adsorbed) the dye 21, and is provided on the conductive substrate 1 by coating. The photosensitive layer 2 is designed according to the purpose, and may be constituted with a single layer or multiple layers. A single photosensitive layer may contain one kind of the dye 21 or a mixture of plural kinds of the dye. However, at least one kind among the dyes used is the metal complex dye, which will be described later, used in the present invention. The light that has entered the photosensitive layer 2 excites the dye. The excited dye has electrons having high energy. The electrons are transferred to the conduction band of the semiconductor particles 22 from the dye 21 and then reach the semiconductor memory device 1 by diffusion. At this time, although the metal complex dye has turned into an oxidized dye, electrons on the electrode work in the external circuit 6, passes the counter electrode 4, and return to the photosensitive layer 2 in which the oxidized dye (metal complex dye) 21 and an electrolyte are present. In this way, the photoelectric conversion element functions as a solar cell.

Fig. 1 is a view for schematically illustrating the embodiment of the present invention. In the present invention, the way the dye is adsorbed, the constitution (single layer-multiple layers) of each layer, and the like are not limited to the constitution shown in Fig. 1.

In the present invention, as materials used in the photoelectric conversion element or the dye-sensitized solar cell and methods for preparing each of the members, except for the constitution of the metal complex dye, the materials and methods for preparing each of the members that are generally used for photoelectric conversion elements and dye-sensitized solar cells may be adopted. For example, US4927721A, US4684537A, US5084365A, US5350644A, US5463057A, US5525440A, JP1995-249790A (JP-H07-249790A), JP2004-220974A, and JP2008-135197A can be referred to.

Hereinafter, main members will be schematically described.

<Photosensitive layer>

The photosensitive layer contains an electrolyte which will be described later. It is a layer containing semiconductor particles supporting a sensitizing dye including the metal complex dye used in the present invention that will be described later.

(Metal complex dye)

In the present invention, a sensitizing dye is used for increasing photosensitivity of semiconductor particles by being supported on (adsorbed onto) the surface of the semiconductor particles. In the present invention, as the sensitizing dye, at least a metal complex dye represented by the following Formula (1) is used.

$$M(LD)(LA)(X)m\cdot CI \qquad \text{Formula (1)}$$

In Formula (1), M is a central metal and represents a divalent or trivalent metalion. M is preferably a metal ion that can be coordinated with a tetradentate ligand or a hexadentate ligand. Specific examples of metal atoms of the metal ion include Ru, Fe, Os, Cu, W, Cr, Mo, Ni, Pd, Pt, Co, Ir, Rh, Re, Mn, and Zn. The metal ion is preferably $Ru^{2+}$, $Os^{2+}$, or $Fe^{2+}$, particularly preferably $Ru^{2+}$ or $Os^{2+}$, and most preferably $Ru^{2+}$. Herein, the valency of the metal ion in a state of being incorporated into the photoelectric conversion element changes in some cases due to an oxidation-reduction reaction between M and the surrounding material.

In Formula (1), LA represents a tridentate ligand represented by the following Formula (2).

[Chem. 5]

Formula (2)

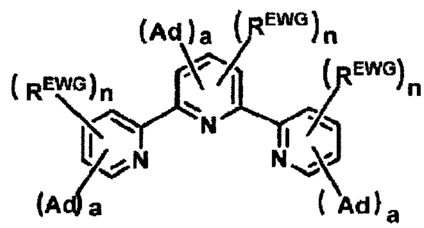

In Formula (2), Ad represents either a group (adsorptive group) that exhibits adsorptivity with respect to the surface of semiconductor particles or a group having the adsorptive group. The adsorptive group is selected from among -COOH, $-SO_3H$ $-PO_3H_2$, -OH, -SH, and salts of these. Ad is preferably the adsorptive group, and as Ad, -COOH or a salt thereof is suitable.

Each of as represents an integer of 0 to 3, and a is preferably 0 or 1. However, as do not represent 0 at the same time. In Formula (2), the total number of Ad is preferably 1 to 3, and more preferably 2. Particularly, among three pyridine rings, each of two pyridine rings adjacent to each other preferably has one Ad, and the remaining pyridine ring preferably do not have Ad.

$R^{EWG}$ represents an electron-withdrawing group selected from among $-NO_2$, $-SO_2R$,- $SO_3R$, -F, -Cl, -Br, -I, -CN, -COR, and a perfluoroalkyl group. Herein, R represents a group selected from among an alkyl group, an aryl group, an alkenyl group, an alkynyl group, a heterocyclic group, and an amino group.

Herein, the amino group includes an amino group, an alkylamino group, a N,N-dialkylamino group, an arylamino group, a N,N-diarylamino group, a N-alkyl-N-arylamino group, a heterocyclic amino group, a N,N-diheterocyclic amino group, a N-alkyl-N-heterocyclic amino group, and a N-aryl-N-heterocyclic amino group.

Each of ns represents an integer of equal to or greater than 0, but all n do not represent 0 at the same time. In Formula (2), the total number of $R^{EWG}$ is preferably 1 to 3, more preferably 1 or 2, and even more preferably 1.

In Formula (2), it is preferable for Ad and $R^{EWG}$ to be present on different pyridine rings.

$R^{EWG}$ is preferably $-NO_2$, $-SO_2R$, -COR, a perfluoroalkyl group, a cyano group, or a halogen atom, and more preferably a cyano group or a halogen atom.

In Formula (2), in a case in which Ad is the group having the adsorptive group (a case in which Ad is the adsorptive group is not included in this case), Ad is a group selected from among an alkyl group and an amino group, and preferably has the adsorptive group as a substituent.

Specific examples of LA will be shown below.

In the following specific examples, "Ac" represents acetyl, "Me" represents methyl, and "Et" represents ethyl.

[Chem. 6]

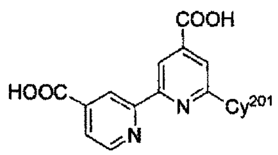

| | $Cy^{201}$ | | $Cy^{201}$ | | $Cy^{201}$ |
|---|---|---|---|---|---|
| LA-1-1 | 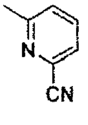 | LA-1-8 | 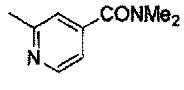 | LA-1-15 | 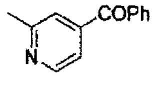 |
| LA-1-2 | 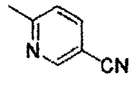 | LA-1-9 | 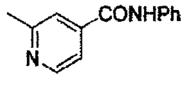 | LA-1-16 | 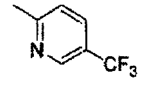 |

(continued)

| | Cy$^{201}$ | | Cy$^{201}$ | | Cy$^{201}$ |
|---|---|---|---|---|---|
| LA-1-3 | 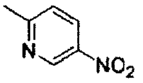 | LA-1-10 | 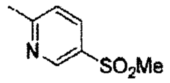 | LA-1-17 | 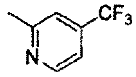 |
| LA-1-4 | 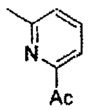 | LA-1-11 | 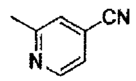 | LA-1-18 | 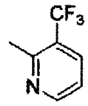 |
| LA-1-5 | 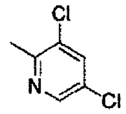 | LA-1-12 | 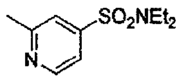 | LA-1-19 | 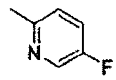 |
| LA-1-6 | 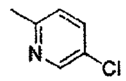 | LA-1-13 | 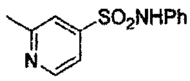 | LA-1-20 | 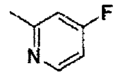 |
| LA-1-7 | 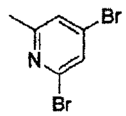 | LA-1-14 | 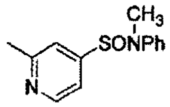 | LA-1-21 | 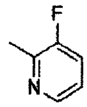 |

Examples of LA also include a triethylamine salt and a tetrabutylammonium salt of the above specific examples, in addition to the above specific examples.

In Formula (1), LD represents a bidentate or tridentate ligand having an aryl group or a heterocyclic group. The ligand has one to three atoms that are coordinated with the central metal M by becoming an anion, and preferably has one or two such atoms.

When LD is a bidentate ligand, it is preferably either a ligand represented by any of the following Formulae (2L-1) to (2L-4) or a ligand represented by Formula (2L-5) or (2L-6) which will be described later.

Particularly, a ligand represented by any of the following Formulae (2L-1) to (2L-4) is preferable, and a ligand represented by Formula (2L-1) or (2L-2) is more preferable.

[Chem. 7]

Formula (2L-1)

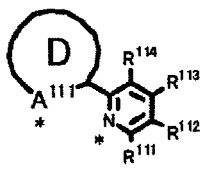

Formula (2L-2)

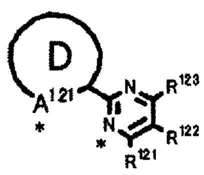

Formula (2L-3)

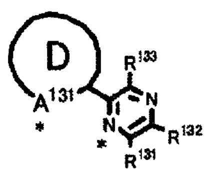

Formula (2L-4)

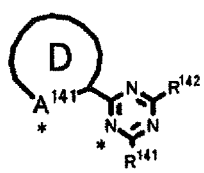

In Formulae (2L-1) to (2L-4), * represents a position in which the ligand is coordinated with the central metal M.

In Formulae (2L-1) to (2L-4), $A^{111}$, $A^{121}$, $A^{131}$, and $A^{141}$ are coordinating atoms coordinated with the central metal M, and are selected from among a nitrogen atom or a carbon atom. However, all of them are anionic. That is, the nitrogen atom and the carbon atom becoming the coordinating atoms carry a negative (-) charge. $A^{111}$, $A^{121}$ $A^{131}$, and $A^{141}$ are preferably anionic nitrogen atoms.

Herein, $A^{111}$, $A^{121}$, $A^{131}$, and $A^{141}$ are ring-constituting atoms of a ring D.

In Formulae (2L-1) to (2L-4), the ring D represents an aromatic hydrocarbon ring or a heterocyclic ring. The heterocyclic ring is preferably an aromatic heterocyclic ring.

The aromatic hydrocarbon ring is preferably a 6-membered ring, and may be condensed, and examples thereof include a benzene ring and a naphthalene ring.

The heterocyclic ring is preferably a 5-membered ring or a 6-membered ring, and hetero atoms constituting the ring may include an oxygen atom or a sulfur atom in addition to a nitrogen atom. The heterocyclic ring may be condensed with a benzene ring or a heterocyclic ring.

Specific examples of the ring D include a pyrrole ring, an imidazole ring, a pyrazole ring, a triazole ring, a benzene ring, a furan ring, a thiophene ring, an oxazole ring, a thiazole ring, a benzolog (benzene condensate) of these, and the like. Among these, a pyrrole ring, n imidazole ring, a pyrazole ring, a triazole ring, and a benzene ring are preferable, and an imdazole ring, a pyrazole ring, and a benzene ring are particularly preferable. These rings may have a substituent, and examples of the substituent include substituents selected from a substituent group T which will be described later. Among the substituents, an alkyl group is preferable, and a methyl group and a trifluoromethyl group are more preferable.

In Formulae (2L-1) to (2L-4), each of $R^{111}$ to $R^{114}$, $R^{121}$ to $R^{123}$, $R^{131}$ to $R^{133}$, and $R^{141}$ and $R^{142}$ independently represents a hydrogen atom or a substituent. Examples of the substituent include substituents selected from the substituent group T which will be described later. Among the substituents, an alkyl group, an aryl group, an alkenyl group, an alkynyl group, and a heterocyclic ring are preferable, and an aryl group, an alkenyl group, and a heterocyclic ring are particularly preferable. The number of carbon atoms of the substituent is preferably an integer of 0 to 30, more preferably an integer of 0 to 25, even more preferably an integer of 0 to 20, and particularly preferably an integer of 0 to 10.

Any one of $R^{111}$ to $R^{114}$, any one of $R^{121}$ to $R^{123}$, any one of $R^{131}$ to $R^{133}$, and any one of $R^{141}$ and $R^{142}$ is preferably a group represented by the following Formula (G).

[Chem. 8]

Formula (G)

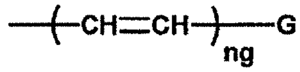

In Formula (G), G represents a group represented by the following Formula (G-1). ng represents 0 or 1.

[Chem. 9]

Formula (G-1)

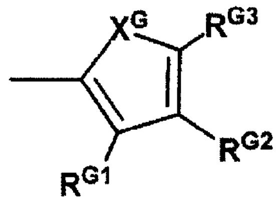

In Formula (G-1), $X^G$ represents an oxygen atom, a sulfur atom, $N(R^{g1})$, $C(R^{g1})(R^{g2})$, or $Si(R^{g1})(R^{g2})$. Herein, each of $R^{g1}$ and $R^{g2}$ independently represents a hydrogen atom, an alkyl group, or an aryl group. Each of $R^{G1}$, $R^{G2}$, and $R^{G3}$ independently represents a hydrogen atom or a substituent. $R^{G1}$ and $R^{G2}$, and $R^{G2}$ and $R^{G3}$ may form a ring by binding to each other.

ng is preferably 1.

$X^G$ is preferably an oxygen atom, a sulfur atom, $N(R^{g1})$, or $C(R^{g1})(R^{g2})$, more preferably an oxygen atom, a sulfur atom, or $C(R^{g1})(R^{g2})$, and particularly preferably a sulfur atom.

ALogP value of G is preferably 3.0 to 20.0.

The "LogP" refers to a common logarithm of a partition coefficient P. It is a value of physical properties showing how a certain chemical substance is partitioned in two-phase system including oil (generally, 1-octanol) and water that are in a state of equilibrium, by a quantitative numerical value. The LogP is represented by the following formula.

$$\mathrm{LogP} = \mathrm{Log}(C_{oil}/C_{water})$$

In the above formula, $C_{oil}$ represents molar concentration in an oil phase, and $C_{water}$ represents a molar concentration in a water phase. As the value of LogP increases in positive sense from 0, oil solubility is increased. Moreover, as the absolute value of LogP increases in negative sense, water solubility is increased. There is negative correlation between LogP and solubility of a chemical substance, and LogP is widely used as a parameter for estimating hydrophilicity

and hydrophobicity. Considering its definition, LogP needs to be measured by partition experiment in principle. However, because the experiment is cumbersome, it is effective to estimate the LogP value by using a structural formula.

Therefore, an estimated LogP value obtained by calculation is frequently used.

In the present invention, the LogP value is calculated by ChemDrawPro ver. 12.0 manufactured by CambridgeSoft. The LogP value is preferably 3.5 to 15.0, more preferably 4.0 to 14.0, even more preferably 4.2 to 12.0, particularly preferably 4.3 to 10.0, and most preferably 4.4 to 9.0.

From the viewpoint of photoelectric conversion efficiency and durability, G (group represented by Formula (G-1)) is preferably any of the following Embodiment A to Embodiment C, more preferably Embodiment A or Embodiment B, and even more preferably Embodiment A.

<Embodiment A>

Each of $R^{G1}$ and $R^{G2}$ is any of a hydrogen atom, an alkyl group, an amino group, an alkylthio group, an arylthio group, an alkenyl group, and an alkynyl group. Moreover, $R^{G3}$ represents any of a hydrogen atom, an alkyl group, an alkylamino group, an arylamio group, an alkoxy group, an aryloxy group, an alkylthio group, an arylthio group, an alkenyl group, a heteroaryl group, an alkenyl group, and an alkynyl group. Here, when ng is 0, and both the $R^{G1}$ and $R^{G2}$ are hydrogen atoms, $R^{G3}$ is any of a hydrogen atom, an alkylamino group, an arylamino group, an alkoxy group, an aryloxy group, an alkylthio group, an arylthio group, an alkenyl group, a heteroaryl group, an alkenyl group, and an alkynyl group.

Each of $R^{G1}$ and $R^{G2}$ is preferably a hydrogen atom, an alkyl group, an alkylthio group, an arylthio group, an alkenyl group, or an alkynyl group, more preferably a hydrogen atom, an alkyl group, an alkylthio group, or an arylthio group, and even more preferably a hydrogen atom, an alkyl group, or an alkylthio group.

$R^{G3}$ is preferably a hydrogen atom, an alkyl group, an alkoxy group, an aryloxy group, an alkylthio group, an arylthio group, an alkenyl group, or a heteroaryl group, more preferably a hydrogen atom, an alkyl group, an alkylthio group, an arylthio group, an alkenyl group, or a heteroaryl group, even more preferably a hydrogen atom, an alkyl group, an alkylthio group, an arylthio group, or a heteroaryl group, and particularly preferably a hydrogen atom, an alkyl group, an alkylthio group, or a heteroaryl group. When $R^{G1}$ is a substituent, $R^{G2}$ is preferably a hydrogen atom, and $R^{G3}$ is preferably a hydrogen atom, an alkoxy group, an alkylthio group, an arylthio group, or a heteroaryl group, and more preferably a hydrogen atom or a heteroaryl group. When $R^{G2}$ is a substituent, each of $R^{G1}$ and $R^{G3}$ is preferably a hydrogen atom.

Specific examples of G in Embodiment A and specific examples of -(CH=CH)ng-G will be shown below. Here, the scope of the present invention is not limited thereto.

Herein, in the position indicated by * in specific examples of G, G binds to a pyridine ring, a pyrimidine ring, a pyrazine ring, or a triazine ring of LD in Formula (1) substituted with the ring D.

[Chem. 10]

| No. | G | LogP value of G | ng |
|---|---|---|---|
| G-1A | 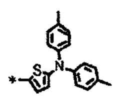 | 6.82 | 1 |
| G-2A | 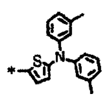 | 6.49 | 1 |
| G-3A | 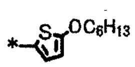 | 4.10 | 1 |
| G-4A | 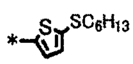 | 4.66 | 1 |
| G-5A | 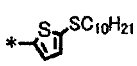 | 6.33 | 1 |
| G-6A | 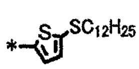 | 7.17 | 1 |

(continued)

| No. | G | LogP value of G | ng |
|---|---|---|---|
| G-7A | 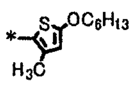 | 4.59 | 1 |
| G-8A | 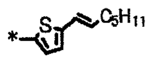 | 4.69 | 1 |
| G-9A | 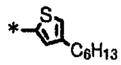 | 4.53 | 1 |
| G-10A | 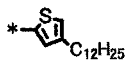 | 7.04 | 1 |
| G-11A | 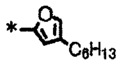 | 3.17 | 1 |
| G-12A | 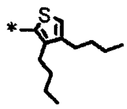 | 5.44 | 1 |
| G-13A | 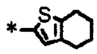 | 3.19 | 1 |
| G-14A | 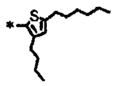 | 6.19 | 1 |
| G-15A | 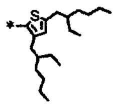 | 8.52 | 1 |
| G-16A | 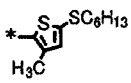 | 5.15 | 1 |
| G-17A | 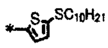 | 6.33 | 1 |
| G-18A | 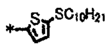 | 6.33 | 0 |
| G-19A | 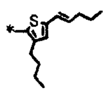 | 5.93 | 1 |
| G-20A | 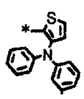 | 5.32 | 1 |
| G-21A | 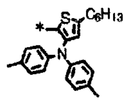 | 8.79 | 1 |
| G-22A | 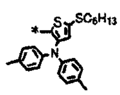 | 9.0 | 1 |

(continued)

| No. | G | LogP value of G | ng |
|---|---|---|---|
| G-23A | 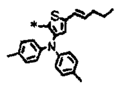 | 8.19 | 1 |
| G-24A | 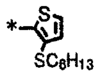 | 4.48 | 1 |

[Chem. 11]

| No. | G | LogP value of G | ng |
|---|---|---|---|
| G-25A | 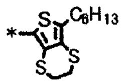 | 5.09 | 1 |
| G-26A | 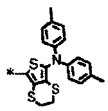 | 7.12 | 1 |
| G-27A | 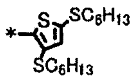 | 7.18 | 1 |
| G-2BA | 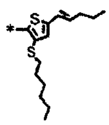 | 6.37 | 1 |
| G-29A | 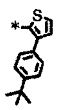 | 5.34 | 1 |
| G-30A | 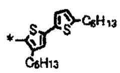 | 8.74 | 1 |
| G-31A | 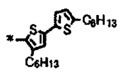 | 8.74 | 0 |
| G-32A | 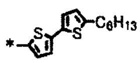 | 6.16 | 1 |
| G-33A | 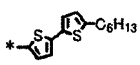 | 6.16 | 0 |
| G-34A | 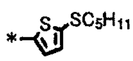 | 4.25 | 0 |
| G-35A | 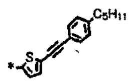 | 6.11 | 1 |
| G-36A | 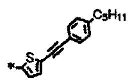 | 6.11 | 0 |

(continued)

| No. | G | LogP value of G | ng |
|---|---|---|---|
| G-37A | 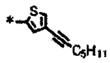 | 4.38 | 1 |
| G-38A | 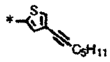 | 4.38 | 0 |
| G-39A | 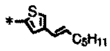 | 4.63 | 1 |
| G-40A | 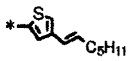 | 4.63 | 0 |
| G-41A | 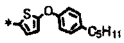 | 5.84 | 1 |
| G-42A | 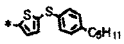 | 6.41 | 1 |

<Embodiment B>

Each of $R^{G1}$ and $R^{G2}$ is any of a hydrogen atom, an alkyl group, an amino group, an alkylthio group, and an arylthio group, and $R^{G3}$ is an aryl group. Each of $R^{G1}$ and $R^{G2}$ is preferably any of a hydrogen atom, an alkyl group, an alkylthio group, and an arylthio group, and $R^{G3}$ is preferably an aryl group. Each of $R^{G1}$ and $R^{G2}$ is more preferably any of a hydrogen atom and an alkyl group, and $R^{G3}$ is more preferably an aryl group.

Specific examples of G in Embodiment B and specific examples of -(CH=CH)ng-G will be shown below. Here, the scope of the present invention is not limited thereto.

[Chem. 12]

| No. | G | LopP value of G | ng |
|---|---|---|---|
| G-1B | 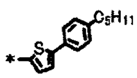 | 5.86 | 1 |
| G-2B | 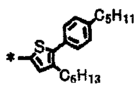 | 8.42 | 1 |
| G-3B | 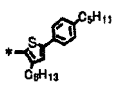 | 8.42 | 1 |
| G-4B | 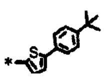 | 5.73 | 1 |
| G-5B | 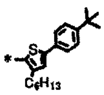 | 7.97 | 1 |
| G-6B | 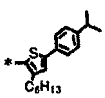 | 7.50 | 1 |
| G-7B | 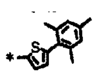 | 5.15 | 1 |

(continued)

| No. | G | LopP value of G | ng |
|---|---|---|---|
| G-8B | 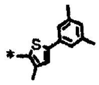 | 5.15 | 1 |
| G-9B | 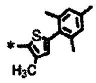 | 7.73 | 1 |
| G-10B | 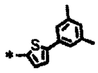 | 4.67 | 1 |
| G-11B | 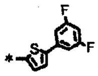 | 4.01 | 1 |
| G-12B | 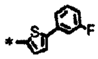 | 3.85 | 1 |
| G-13B | 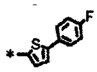 | 3.85 | 1 |
| G-14B | 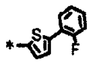 | 3.85 | 1 |
| G-15B | 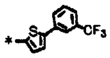 | 4.61 | 1 |
| G-16B | 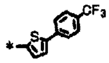 | 4.61 | 1 |
| G-17B | 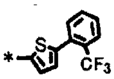 | 4.61 | 1 |
| G-18B | 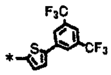 | 5.53 | 1 |
| G-19B | 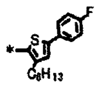 | 6.42 | 1 |
| G-20B | 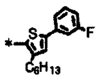 | 6.42 | 1 |
| G-21B | 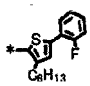 | 6.42 | 1 |
| G-22B | 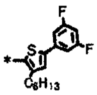 | 6.58 | 1 |
| G-23B | 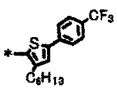 | 7.52 | 1 |
| G-24B | 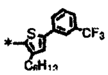 | 7.19 | 1 |

[Chem. 13]

| No. | G | LogP value of G | ng |
|---|---|---|---|
| G-25B | 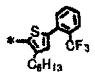 | 7.52 | 1 |
| G-26B | 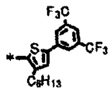 | 8.11 | 1 |
| G-27B | 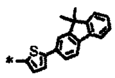 | 6.22 | 1 |
| G-28B | 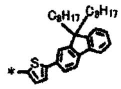 | 12.07 | 1 |
| G-29B | 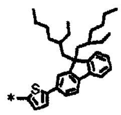 | 11.89 | 1 |
| G-30B | 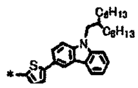 | 9.92 | 1 |

<Embodiment C>

Each of $R^{G1}$ and $R^{G2}$ is a chain-like alkoxy group or an aryloxy group, and $R^{G3}$ is any of a hydrogen atom, an alkyl group, an alkylamino group, an arylamino group, an alkoxy group, an aryloxy group, an alkylthio group, an arylthio group, an alkenyl group, an aryl group, and a heteroaryl group. The chain-like alkoxy group or aryloxy group represented by each of $R^{G1}$ and $R^{G2}$ is preferably a substituted or unsubstituted linear or branched alkyl group having 6 to 30 carbon atoms, a substituted or unsubstituted aryl group having 6 to 30 carbon atoms, a substituted or unsubstituted linear or branched alkyl group having 6 to 20 carbon atoms, or a substituted or unsubstituted aryl group having 6 to 20 carbon atoms, and more preferably a substituted or unsubstituted linear or branched alkyl group having 6 to 15 carbon atoms or a substituted or unsubstituted aryl group having 6 to 15 carbon atoms.

Specific examples of G in Embodiment C and specific examples of -(CH=CH)ng-G will be shown below. Here, the scope of the present invention is not limited thereto.

[Chem. 14]

| No. | G | LogP value of G | ng |
|---|---|---|---|
| G-1C | 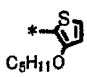 | 3.49 | 1 |
| G-2C | 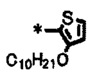 | 5.58 | 1 |
| G-3C | 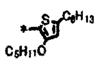 | 6.32 | 1 |

(continued)

| No. | G | LogP value of G | ng |
|---|---|---|---|
| G-4C | 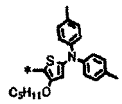 | 8.02 | 1 |
| G-5C | 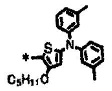 | 8.02 | 1 |
| G-6C | 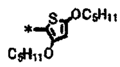 | 5.21 | 1 |
| G-7C | 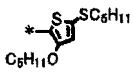 | 6.20 | 1 |
| G-8C | 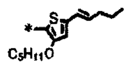 | 5.39 | 1 |
| G-9C | 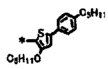 | 7.38 | 1 |
| G-10C | 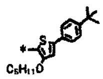 | 6.93 | 1 |
| G-11C | 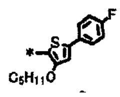 | 5.38 | 1 |
| G-12C | 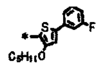 | 5.38 | 1 |
| G-13C | 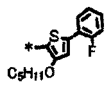 | 5.38 | 1 |
| G-14C | 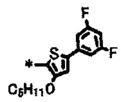 | 5.88 | 1 |
| G-15C | 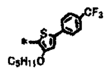 | 6.14 | 1 |
| G-16C | 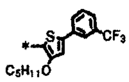 | 6.14 | 1 |
| G-17C | 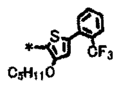 | 6.14 | 1 |
| G-18C | 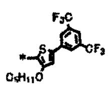 | 7.07 | 1 |
| G-19C | 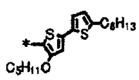 | 7.70 | 1 |

(continued)

| No. | G | LogP value of G | ng |
|---|---|---|---|
| G-20C | 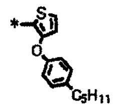 | 5.65 | 1 |

When LD is a bidentate ligand, in addition to Formulae (2L-1) to (2L-4), a ligand represented by the following Formula (2L-5) or (2L-6) is also preferable.

[Chem. 15]

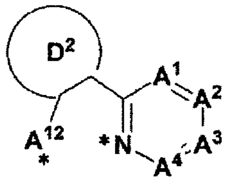

Formula (2L-5)

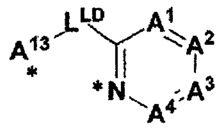

Formula (2L-6)

In Formulae (2L-5) and (2L-6), * represents a position in which the ligand is coordinated with M.
A ring $D^2$ represents an aromatic hydrocarbon ring or a heterocyclic aromatic ring.
Each of $A^{12}$ and $A^{13}$ independently represents $N^-R^L$, $O^-$, or $S^-$.
Each of $A^1$ to $A^4$ independently represents $C(R^{LD})$ or N, and at least one of $A^1$ to $A^4$ represents N.
$L^{LD}$ represents a divalent linking group selected from the group consisting of -C(=O)-, -C(=S)-, $-C(=NR^L)-$, $-C(R^L)_2-$, and $-C(=C(R^L)_2)-$.
Each of $R^L$ and $R^{LD}$ independently represents a hydrogen atom or a substituent.
The ring $D^2$ represents an aromatic hydrocarbon ring or a heterocyclic ring. The ring $D^2$ has the same definition as the ring D in Formulae (2L-1) to (2L-4), and preferable range thereof is also the same.
Examples of the substituent in $R^L$ and $R^{LD}$ include the substituents T which will be described later.
Specific examples of the bidentate ligand represented by LD will be shown below, but the present invention is not limited thereto.
Herein, "Me" is methyl, "t-Bu" is t-butyl, and "ph" is phenyl.

<Examples of bidentate ligand>

[Chem. 16]

| $R^{601}$-$R^{602}$ | | |
|---|---|---|
| LD No | $R^{601}$ | $R^{602}$ |
| LD-6-1 | 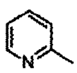 | 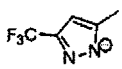 |
| LD-6-2 | 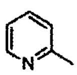 | 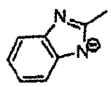 |
| LD-6-3 | 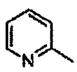 | 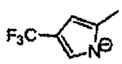 |

(continued)

| R601-R602 | | |
|---|---|---|
| LD No | $R^{601}$ | $R^{602}$ |
| LD-6-4 | 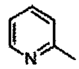 | 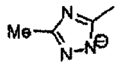 |
| LD-6-5 | 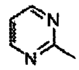 | 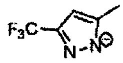 |
| LD-6-6 | 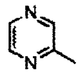 | 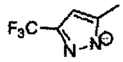 |
| LD-6-7 | 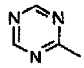 | 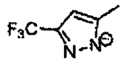 |
| LD-6-8 | 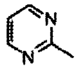 | 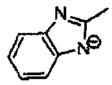 |
| LD-6-9 | 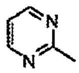 | 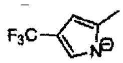 |
| LD-6-10 | 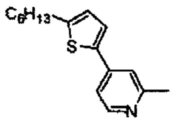 | 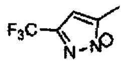 |
| LD-6-11 | 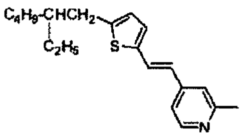 | 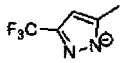 |
| LD-6-12 | 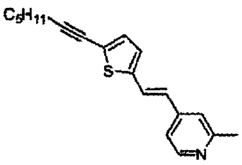 | 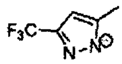 |
| LD-6-13 | 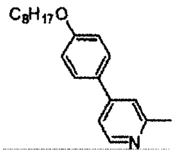 | 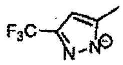 |
| LD-6-14 | 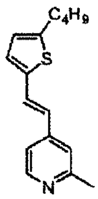 | 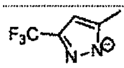 |
| LD-6-15 | 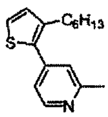 | 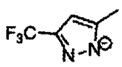 |

(continued)

| $R^{601}$-$R^{602}$ | | |
|---|---|---|
| LD No | $R^{601}$ | $R^{602}$ |
| LD-6-16 | 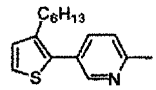 | 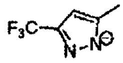 |
| LD-6-17 | 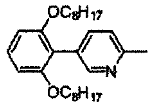 | 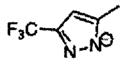 |

[Chem. 17]

| $R^{601}$-$R^{602}$ | | |
|---|---|---|
| LD No | $R^{601}$ | $R^{602}$ |
| LD-6-18 | 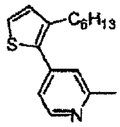 | 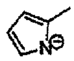 |
| LD-6-19 | 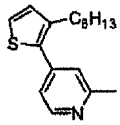 | 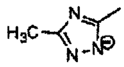 |
| LD-6-20 | 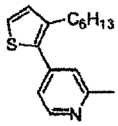 | 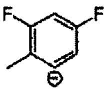 |
| LD-6-21 | 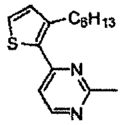 | 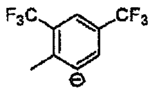 |
| LD-6-22 | 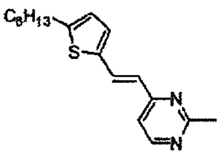 | 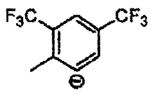 |
| LD-6-23 | 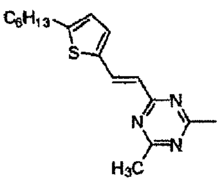 | 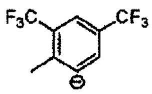 |
| LD-6-24 | 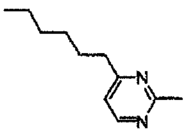 | 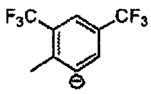 |

(continued)

| $R^{601}$-$R^{602}$ | | |
|---|---|---|
| LD No | $R^{601}$ | $R^{602}$ |
| LD-6-25 | 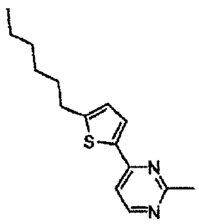 | 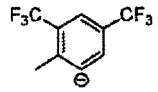 |
| LD-6-26 | 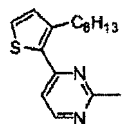 | 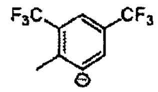 |
| LD-6-27 | 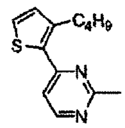 | 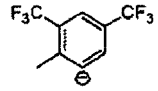 |
| LD-6-27 | 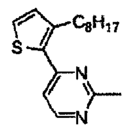 | 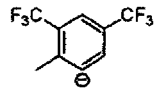 |
| LD-6-27 | 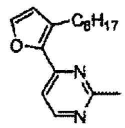 | 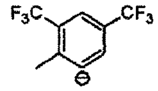 |

[Chem. 18]

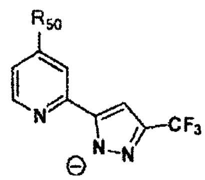

| | $R_{50}$ | | $R_{50}$ | | $R_{50}$ |
|---|---|---|---|---|---|
| L2ex1-1 | 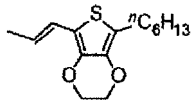 | L2ex1-8 | 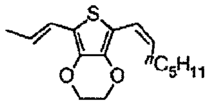 | L2ex1-14 | 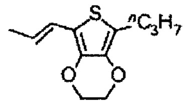 |
| L2ex1-2 | 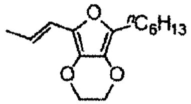 | L2ax1-9 | 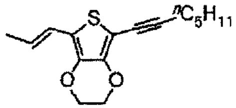 | L2ex1-15 | 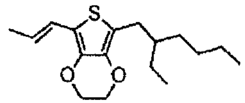 |
| L2ex1-3 | 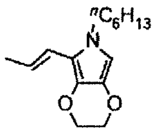 | L2ex1-10 | 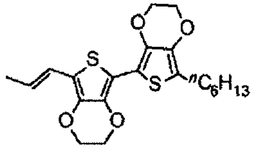 | L2ex1-16 | 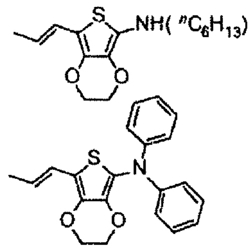 |
| L2ex1-4 | 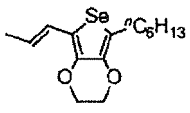 | L2ex1-11 | 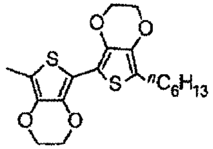 | L2ex1-17 | |
| L2ex1-5 | 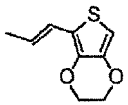 | L2ex1-12 | 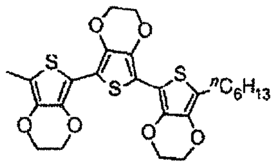 | L2ex1-18 | 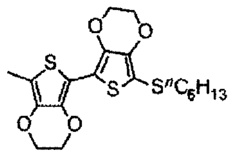 |
| L2ex1-6 | 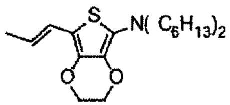 | | | | |
| L2ex1-7 | 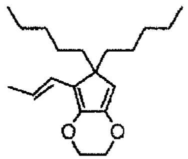 | L2ex1-13 | 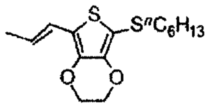 | L2ex1-19 | 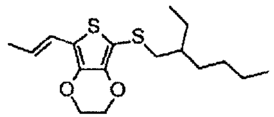 |

[Chem. 19]

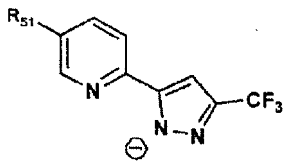

| | R51 | | R51 | | R51 |
|---|---|---|---|---|---|
| L2ex2-1 | 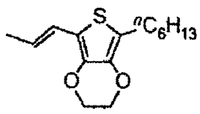 | L2ex2-6 | 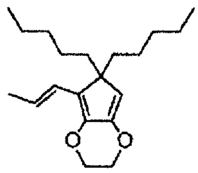 | L2ex2-10 | 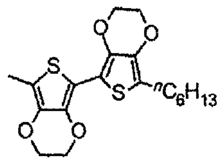 |
| L2ex2-2 | 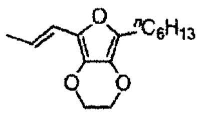 | L2ex2-7 | 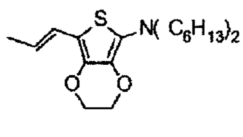 | L2ex2-11 | 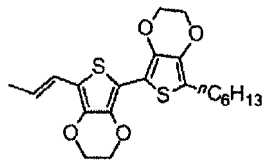 |
| LZex2-3 | 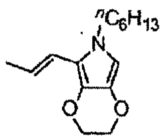 | | | | |
| L2ex2-4 | 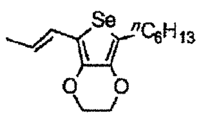 | L2ex2-8 | 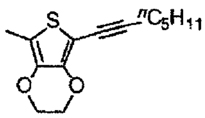 | L2ex2-12 | 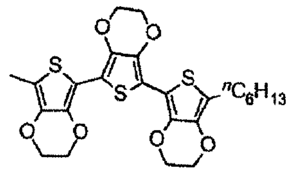 |
| L2ex2-5 | 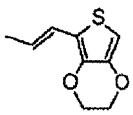 | L2ex2-9 | 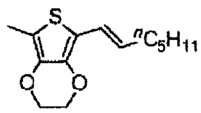 | L2ex1-13 | 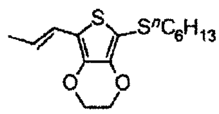 |

[Chem. 20]

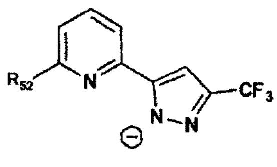

| | R52 | | R52 | | R52 |
|---|---|---|---|---|---|
| L2ex3-1 | 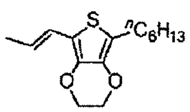 | L2ex3-6 | 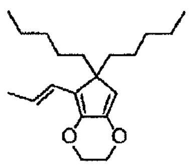 | L2ex3-10 | 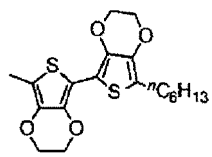 |

(continued)

| | R52 | | R52 | | R52 |
|---|---|---|---|---|---|
| 12ex3-2 | 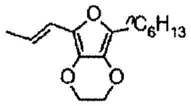 | L2ex3-7 | 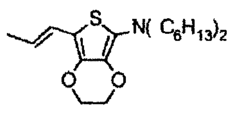 | L2ex3-11 | 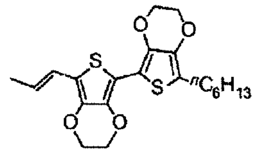 |
| L2ex3-3 | 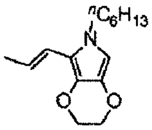 | | | | |
| L2ex3-4 | 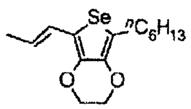 | L2ex3-8 | 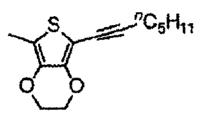 | L2ex3-12 | 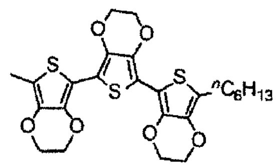 |
| L2ex3-5 | 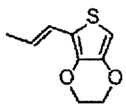 | L2ex3-9 | 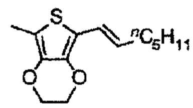 | L2ex1-13 | 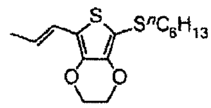 |

[Chem. 21]

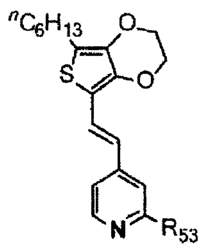

| | R53 | | R53 |
|---|---|---|---|
| L2ex4-1 | 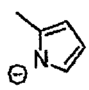 | L2ex4-7 | 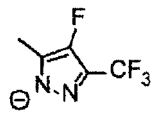 |
| L2ex4-2 | 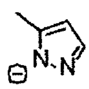 | L2ex4-8 | 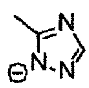 |
| L2ex4-3 | 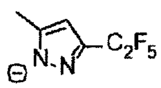 | L2ex4-9 | 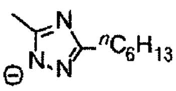 |
| L2ex4-4 | 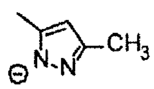 | L2ex4-10 | 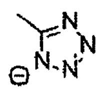 |
| L2ex4-5 | 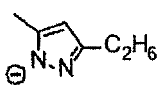 | L2ex4-11 | 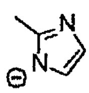 |

(continued)

| | $R_{53}$ | | $R_{53}$ |
|---|---|---|---|
| L2ex4-6 | 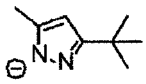 | L2ex4-12 | 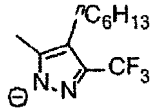 |

[Chem. 22]

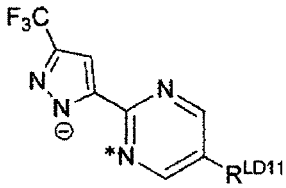

| | $-R^{LD11}$ | | $-R^{LD11}$ |
|---|---|---|---|
| LD-4-1 | $-n-C_8H_{17}$ | LD-4-16 | 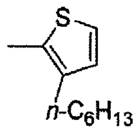 |
| LD-4-2 | $-i-C_3H_7$ | | |
| LD-4-3 | $-t-C_4H_9$ | | |
| LD-4-4 | 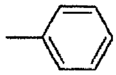 | LD-4-17 | 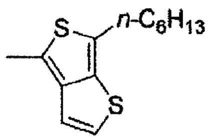 |
| LD-4-5 | 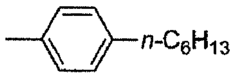 | | |
| LD-4-6 | 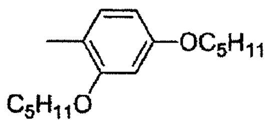 | LD-4-18 | 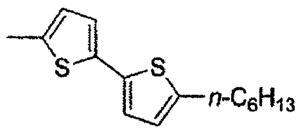 |
| LD-4-7 | 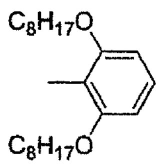 | LD-4-19 | 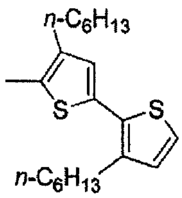 |
| LD-4-8 | 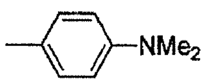 | LD-4-20 | 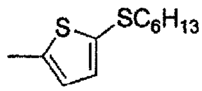 |
| LD-4-9 | 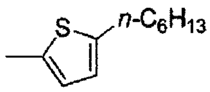 | LD-4-21 | 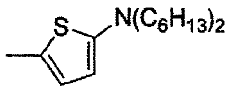 |
| LD-4-10 | 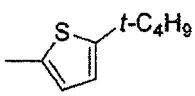 | LD-4-22 | 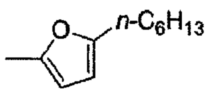 |
| LD-4-11 | 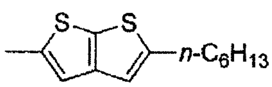 | LD-4-23 | 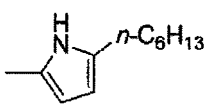 |

(continued)

| | $-R^{LD11}$ | | $-R^{LD11}$ |
|---|---|---|---|
| LD-4-12 | 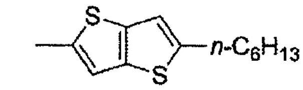 | LD-4-24 | 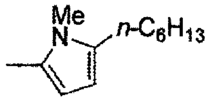 |
| LD-4-13 | 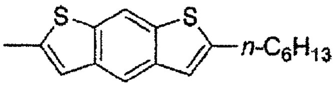 | LD-4-25 | 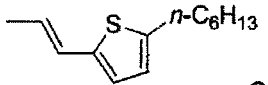 |
| LD-4-14 | 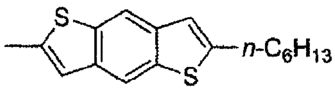 | LD-4-26 | 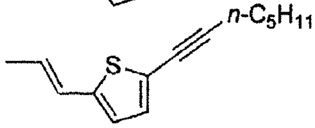 |
| LD-4-15 | 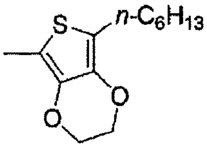 | LD-4-27 | 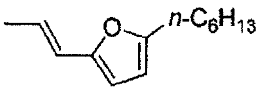 |

[Chem. 23]

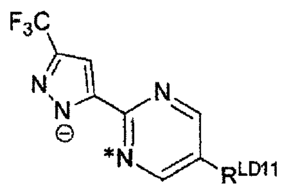

| | $-R^{LD11}$ | | -RLD11 |
|---|---|---|---|
| LD-4-1 | -*n*-$C_8H_{17}$ | | |
| LD-4-2 | -*i*-$C_3H_7$ | LD-4-16 | 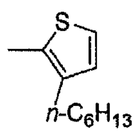 |
| LD-4-3 | -*t*-$G_4H_9$ | | |
| LD-4-4 | 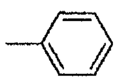 | LD-4-17 | 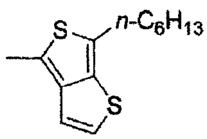 |
| LD-4-5 | 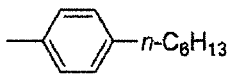 | | |
| LD-4-6 | 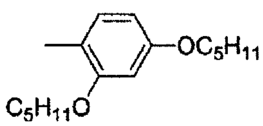 | LD-4-18 | 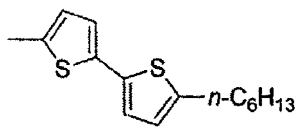 |
| LD-4-7 | 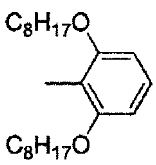 | LD-4-19 | 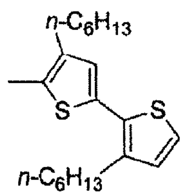 |

(continued)

| | $-R^{LD11}$ | | $-R^{LD11}$ |
|---|---|---|---|
| LD-4-8 | 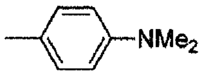 | LD-4-20 | 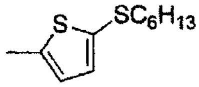 |
| LD-4-9 | 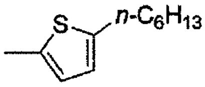 | LD-4-21 | 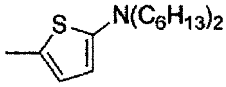 |
| LD-4-10 | 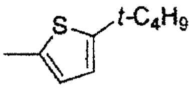 | LD-4-22 | 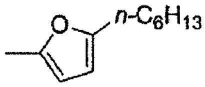 |
| LD-4-11 | 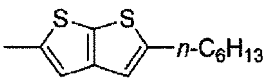 | LD-4-23 | 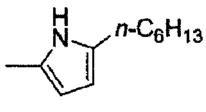 |
| LD-4-12 | 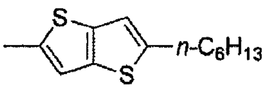 | LD-4-24 | 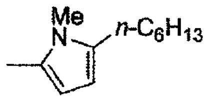 |
| LD-4-13 | 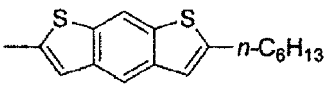 | LD-4-25 | 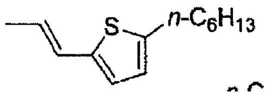 |
| LD-4-14 | 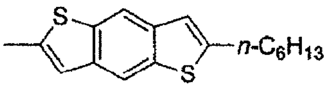 | LD-4-26 | 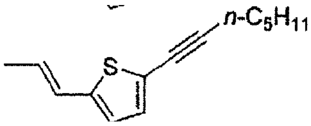 |
| LD-4-15 | 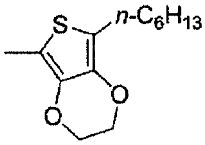 | LD-4-27 | 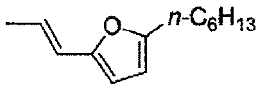 |

[Chem. 24]

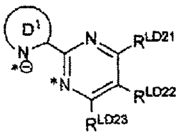

| | 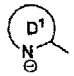 | $-R^{LD21}$ | $-R^{LD22}$ | $-R^{LD23}$ | | 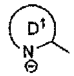 | $-R^{LD21}$ | $-R^{LD22}$ | $-R^{LD23}$ |
|---|---|---|---|---|---|---|---|---|---|
| LD-5-1 | 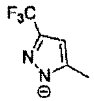 | -H | -H | -H | LD-5-11 | 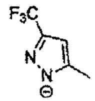 | $-CH_3$ | -H | $-CH_3$ |
| LD-5-2 | 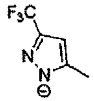 | $-CH_3$ | -H | -H | LD-5-12 | 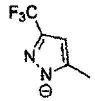 | $-CH_3$ | $-CH_3$ | $-CH_3$ |
| LD-5-3 | 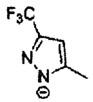 | $-n-C_8H_{17}$ | -H | -H | LD-5-13 | 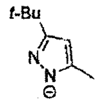 | 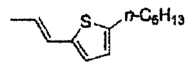 | -H | -H |
| LD-5-4 | 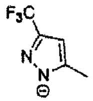 | 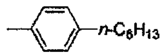 | -H | -H | LD-5-14 | 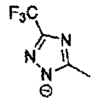 | 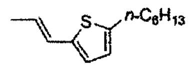 | -H | -H |
| LD-5-5 | 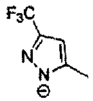 | 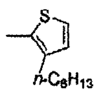 | -H | -H | LD-5-15 | 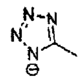 | 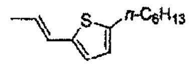 | -H | -H |
| LD-5-6 | 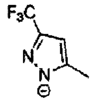 | 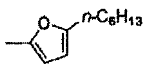 | -H | -H | LD-5-16 | 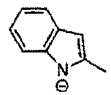 | 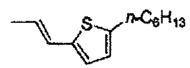 | -H | -H |
| LD-5-7 | 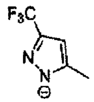 | 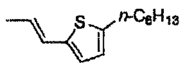 | -H | -H | LD-5-17 | 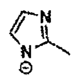 | 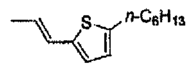 | -H | -H |
| LD-5-8 | 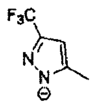 | 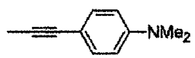 | -H | -H | LD-5-18 | 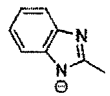 | 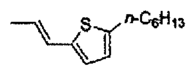 | -H | -H |
| LD-5-9 | 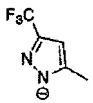 | $-CH_3$ | $-CH_3$ | -H | LD-5-19 | 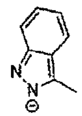 | 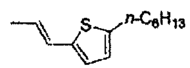 | -H | -H |

(continued)

| | 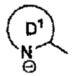 | $-R^{LD21}$ | $-R^{LD22}$ | $-R^{LD23}$ | 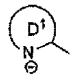 | $-R^{LD21}$ | $-R^{LD22}$ | $-R^{LD23}$ |
|---|---|---|---|---|---|---|---|---|
| LD-5-10 | 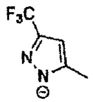 | 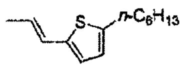 | $-CH_3$ | -H | | | | |

[Chem. 25]

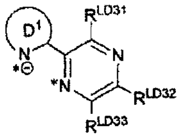

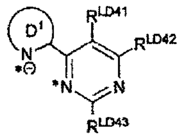

| | 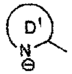 | $-R^{LD31}$ | $-R^{LD32}$ | $-R^{LD33}$ | | 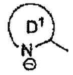 | $-R^{LD41}$ | $-R^{LD42}$ | $-R^{LD43}$ |
|---|---|---|---|---|---|---|---|---|---|
| LD-7-1 | 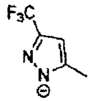 | -H | -H | -H | LD-8-1 | 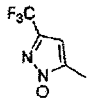 | -H | -H | -H |
| LD-7-2 | 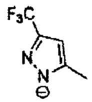 | $-CH_3$ | -H | -H | LD-8-2 | 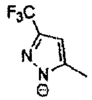 | $-CH_3$ | -H | -H |
| LD-7-3 | 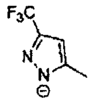 | -H | -H | $-CH_3$ | LD-8-3 | 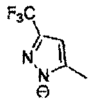 | -H | -H | $-CH_3$ |
| LD-7-4 | 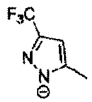 | -H | 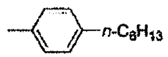 | -H | LD-8-4 | 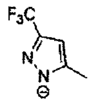 | -H | 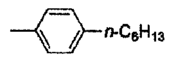 | -H |
| LD-7-5 | 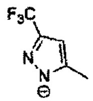 | -H | 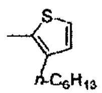 | -H | LD-8-6 | 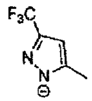 | -H | 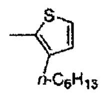 | -H |
| LD-7-6 | 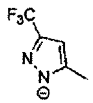 | -H | 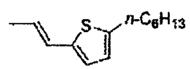 | -H | LD-8-6 | 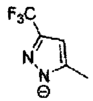 | -H | 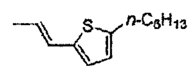 | -H |
| LD-7-7 | 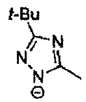 | -H | 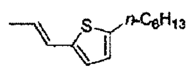 | -H | LD-8-7 | 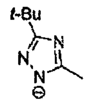 | -H | 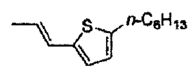 | -H |

[Chem. 26]

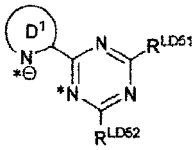

| | 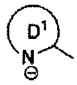 | $-R^{LD51}$ | $-R^{LD52}$ |
|---|---|---|---|
| LD-9-1 | 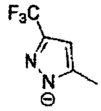 | -H | -H |
| LD-9-2 | 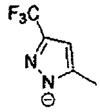 | -H | $-CH_3$ |
| LD-9-3 | 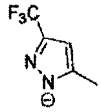 | 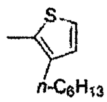 | -H |
| LD-9-4 | 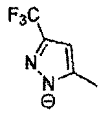 | 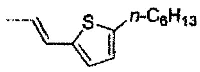 | -H |
| LD-9-5 | 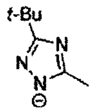 | 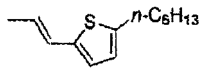 | -H |

[Chem. 27]

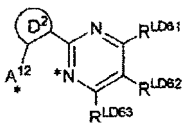

| | 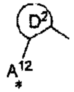 | $-R^{LD61}$ | $-R^{LD62}$ | $-R^{LD63}$ | | 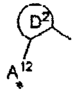 | $R^{LD61}$ | $-R^{LD62}$ | $-R^{LD63}$ |
|---|---|---|---|---|---|---|---|---|---|
| LD-10-1 | 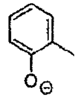 | -H | -H | -H | LD-10-9 | 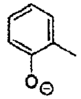 | -H | 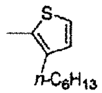 | -H |
| LD-10-2 | 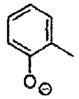 | $-CH_3$ | -H | -H | LD-10-10 | 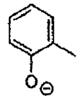 | -H | 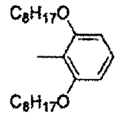 | -H |
| LD-10-3 | 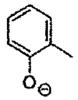 | $-n-C_8H_{17}$ | -H | -H | LD-10-11 | 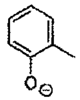 | -H | -H | $CH_3$ |
| LD-10-4 | 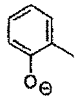 | 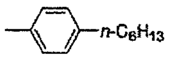 | -H | -H | LD-10-12 | 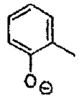 | $-CH_3$ | -H | $-CH_3$ |
| LD-10-5 | 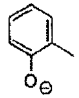 | 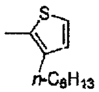 | -H | -H | LD-10-13 | 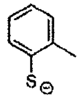 | 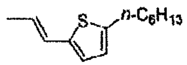 | -H | -H |
| LD-10-6 | 10-6 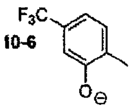 | 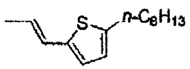 | -H | -H | LD-10-14 | 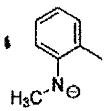 | 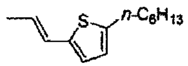 | -H | -H |
| LD-10-7 | 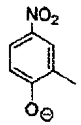 | 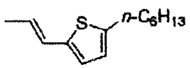 | -H | -H | LD-10-15 | 10-15 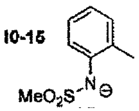 | 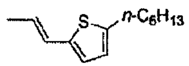 | -H | -H |
| LD-10-8 | 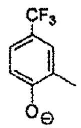 | 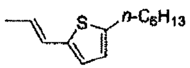 | -H | -H | LD-10-16 | 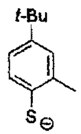 | 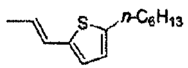 | -H | -H |

[Chem. 28]

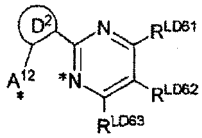

| | 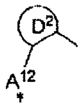 | $-R^{LD61}$ | $-R^{LD62}$ | $-R^{LD63}$ |
|---|---|---|---|---|
| LD-10-17 | 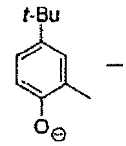 | 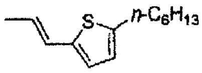 | -H | -H |
| LD-10-18 | 18 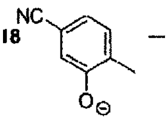 | 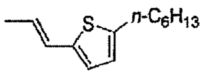 | -H | -H |
| D-10-19 | 19 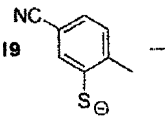 | 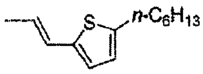 | -H | -H |
| LD-10-20 | ·20 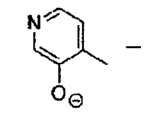 | 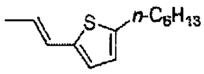 | -H | -H |
| LD-10-21 | 21 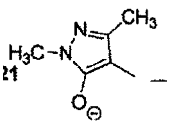 | 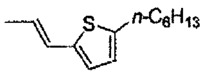 | -H | -H |
| LD-10-22 | 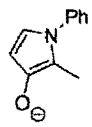 | 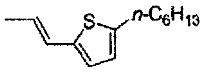 | -H | -H |
| LD-10-23 | 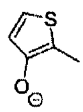 | 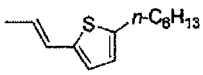 | -H | -H |
| LD-10-24 | 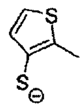 | 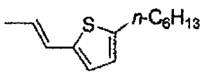 | -H | -H |

[Chem. 29]

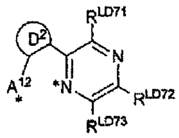

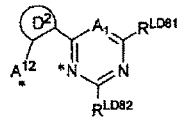

| | 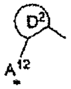 | $-R^{LD71}$ | $-R^{LD72}$ | $-R^{LD73}$ | | 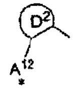 | $A_1$ | $-R^{LD81}$ | $-R^{LD62}$ |
|---|---|---|---|---|---|---|---|---|---|
| LD-11-1 | 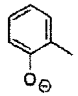 | -H | -H | -H | LD-12-1 | 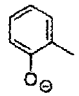 | CH | -H | -H |
| LD-11-2 | 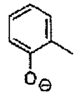 | $-CH_3$ | -H | -H | LD-12-2 | 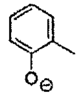 | CH | $-CH_3$ | -H |
| LD-11-3 | 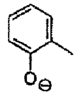 | -H | $-CH_3$ | -H | LD-12-3 | 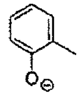 | CH | $-CH_3$ | $-CH_3$ |
| LD-11-4 | 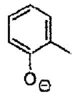 | -H | -H | $-CH_3$ | LD-12-4 | 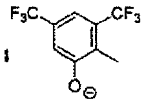 | CH | 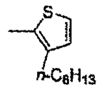 | -H |
| LD-11-5 | 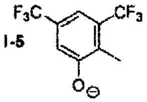 | -H | 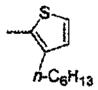 | -H | LD-12-5 | 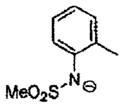 | CH | 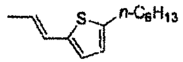 | -H |
| LD-11-6 | 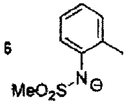 | -H | 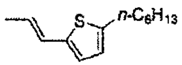 | -H | LD-12-6 | 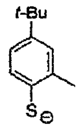 | CH | 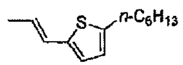 | -H |
| LD-11-7 | 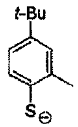 | -H | 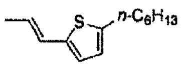 | -H | LD-12-7 | 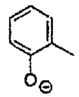 | N | -H | -H |
| LD-11-8 | 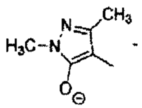 | -H | 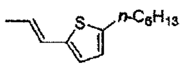 | -H | LD-12-8 | 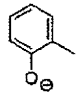 | N | 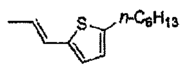 | -H |

[Chem. 30]

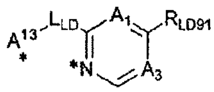

| | $A^{13}_{*}$– | -$L_{LD}$- | -$R_{LD91}$ | $A_1$ | $A_3$ |
|---|---|---|---|---|---|
| **LD-13-1** | 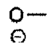 | 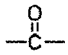 | -H | N | CH |
| **LD-13-2** | 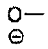 | 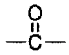 | -$CH_3$ | N | CH |
| **LD-13-3** | 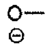 | 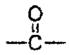 | 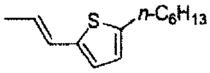 | N | CH |
| **LD-13-4** | 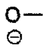 | 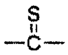 | 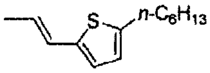 | N | CH |
| **LD-13-5** | 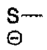 | 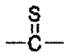 | 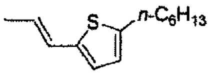 | N | CH |
| **LD-13-6** | 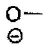 | 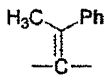 | 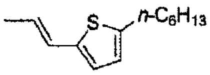 | N | CH |
| **LD-13-7** | 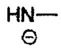 | 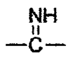 | 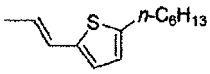 | N | CH |
| **LD-13-8** | 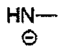 | 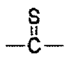 | 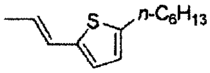 | N | CH |
| **LD-13-9** | 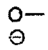 | 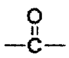 | -H | N | 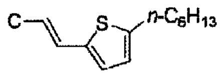 |
| **LD-13-10** | 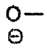 | 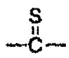 | -H | N | 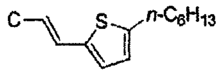 |
| **LD-13-11** | 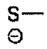 | 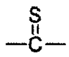 | -H | N | 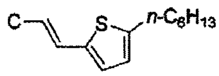 |
| **LD-13-12** | 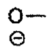 | 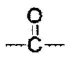 | -H | N | 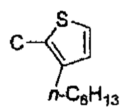 |
| **LD-13-13** | 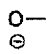 | 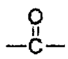 | -H | N | 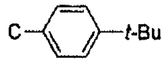 |

[Chem. 31]

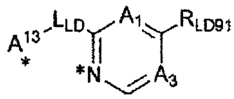

| | $\underset{*}{A^{13}}$– | -$L_{LD}$- | -$R_{LD91}$ | $A_1$ | $A_3$ |
|---|---|---|---|---|---|
| **LD-13-14** | 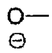 | 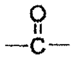 | -H | N | N |
| **LD-13-15** | 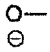 | 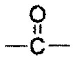 | -$CH_3$ | N | N |
| **LD-13-16** | 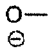 | 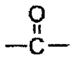 | 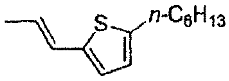 | N | N |
| **LD-13-17** | 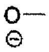 | 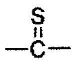 | 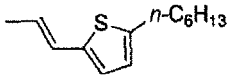 | N | N |
| **LD-13-18** | 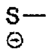 | 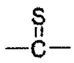 | 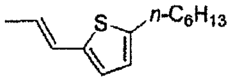 | N | N |
| **LD-13-19** | 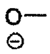 | 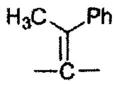 | 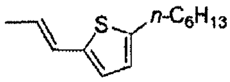 | N | N |
| **LD-13-24** | 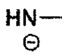 | 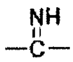 | 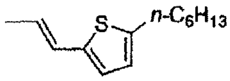 | N | N |
| **LD-13-21** | 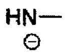 | 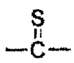 | 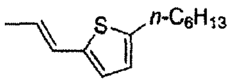 | N | N |
| **LD-13-22** | 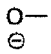 | 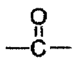 | 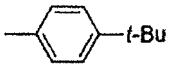 | N | N |
| **LD-13-23** | 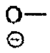 | 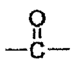 | 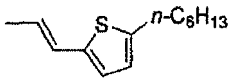 | CH | N |
| **LD-13-24** | 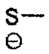 | 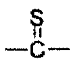 | 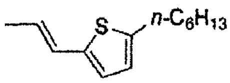 | CH | N |
| **LD-13-26** | 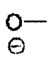 | 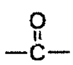 | -H | 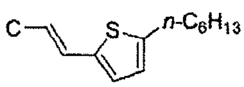 | N |

(continued)

| | $A^{13}$– | -$L_{LD}$- | -$R_{LD91}$ | $A_1$ | $A_3$ |
|---|---|---|---|---|---|
| **LD-13-26** | 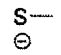 | 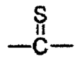 | -H | 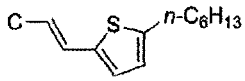 | N |

When LD is a tridentate ligand, the ligand is preferably a ligand represented by any of the following Formulae (3L-1) to (3L-6).

[Chem. 32]

Formula (3L-1)

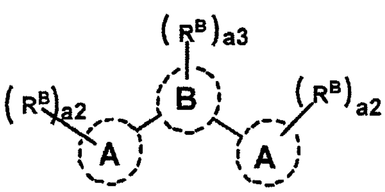

Formula (3L-2)

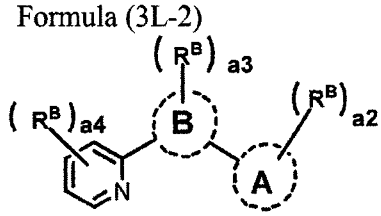

Formula (3L-3)

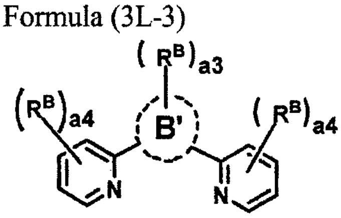

Formula (3L-4)

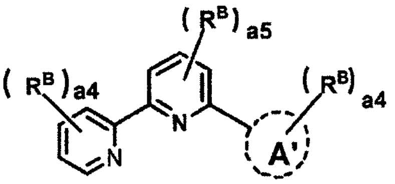

Formula (3L-5)

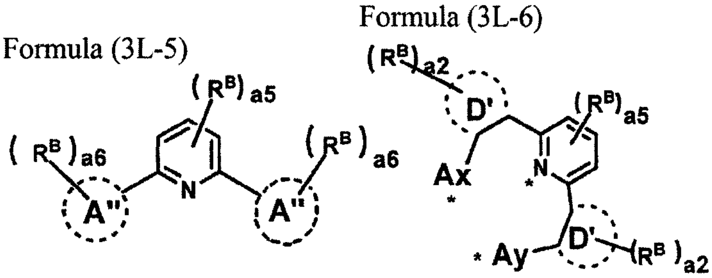

Formula (3L-6)

In Formulae (3L-1) to (3L-6), $R^B$ represents a substituent. Examples of the substituent include substituents selected from the substituent group T which will be described later. Among the substituents, a substituent selected from among an alkyl group, an aryl group, a heterocyclic group, an alkyloxy group, an aryloxy group, an arylthio group, an alkylthio group, a halogen atom, and an amino group is preferable; a substituent selected from among an alkyl group, an aryl group, a heterocyclic group, an alkyloxy group, an aryloxy group, and a halogen atom is more preferable; and a substituent selected from among an alkyl group, an aryl group, a heterocyclic group, and a halogen atom is even more preferable. The number of carbon atoms of the substituent is preferably an integer of 0 to 30, more preferably an integer of 0 to 25, even more preferably an integer of 0 to 20, and particularly preferably an integer of 0 to 10.

Each of a2 and a3 independently represents an integer of equal to or greater than 0, preferably represents an integer of 0 to 4, and more preferably represents an integer of 0 to 3. a4 represents an integer of 0 to 4. a5 represents an integer of 0 to 3. a6 represents an integer of 0 to 2.

When a single ring has a plurality of $R^B$S, the plurality of $R^B$S may form a ring by binding to each other.

A ring A represents any of a pyrimidine ring, a pyrazine ring, a pyridazine ring, a triazine ring, a pyridine ring that is coordinated with M through a carbon atom, a thiophene ring that is coordinated with M through a carbon atom, a furan ring that is coordinated with M through a carbon atom, an imidazole ring, an oxazole ring, a thiazole ring, an oxadiazole ring, a thiadiazole ring, an isoxazole ring, an isothiazole ring, a triazole ring, a pyrazole ring, a pyrrole ring, and a benzene ring. The ring A is preferably an imidazole ring, a tirazole ring, a pyrazole ring, a pyrrole ring, a pyridine ring that is coordinated with M through a carbon atom, or a thiophene ring that is coordinated with M through a carbon atom, and more preferably a pyrazole ring, a pyrrole ring, a pyridine ring that is coordinated with M through a carbon atom, or a thiophene ring that is coordinated with M through a carbon atom.

Herein, two ring As present in Formula (3L-1) may be the same as or different from each other.

Examples of the ring A will be shown below, but the present invention is not limited thereto. In the following specific examples of the ring A, * represents a position in which the ring A binds to a ring B. pr-1 to pr-6, pz-1 to pz-6, im-1 to im-3, and tz-1 to tz-3 are coordinated with M through a nitrogen atom having undergone proton dissociation.

Here, tz-1 to tz-3 may be coordinated with M through other nitrogen atoms in the form of an tautomer. hc-1 to hc-10 are coordinated with M through a carbon atom.

[Chem. 33]

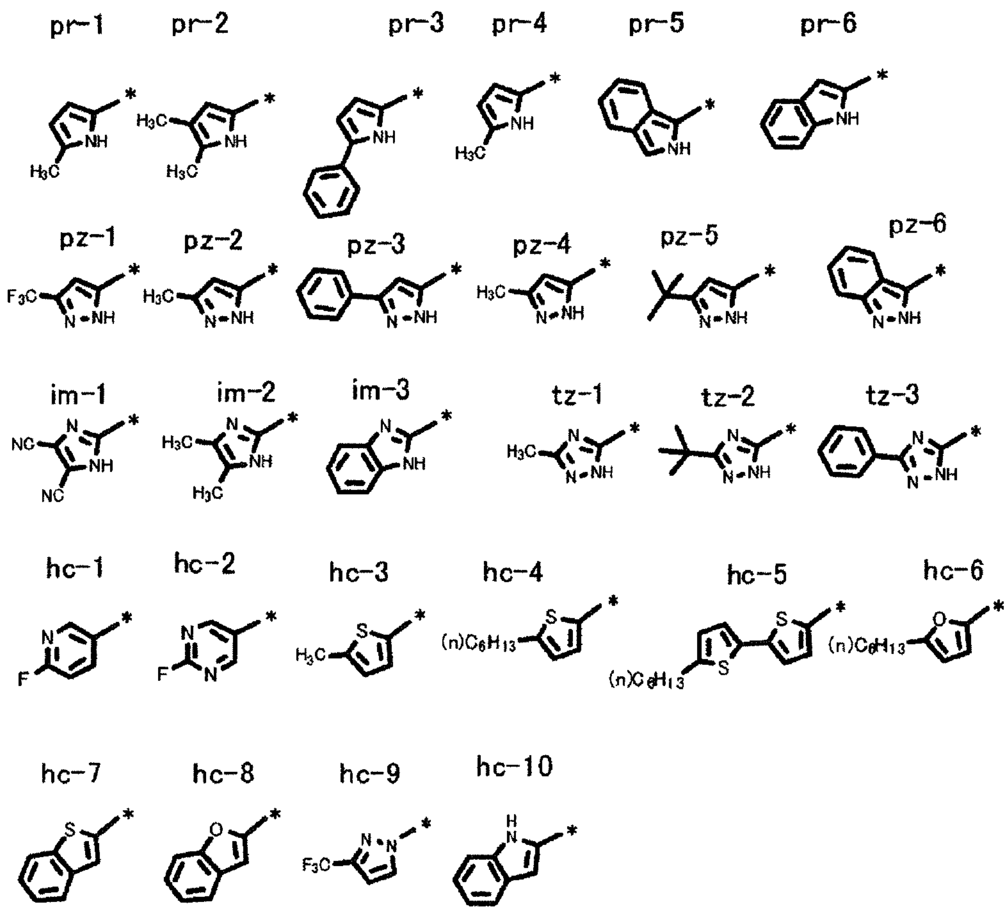

pr-1 pr-2 pr-3 pr-4 pr-5 pr-6

pz-1 pz-2 pz-3 pz-4 pz-5 pz-6

im-1 im-2 im-3 tz-1 tz-2 tz-3

hc-1 hc-2 hc-3 hc-4 hc-5 hc-6

hc-7 hc-8 hc-9 hc-10

A ring A' represents a ring structure selected from among a pyrimidine ring that is coordinated with M through a carbon atom, a pyrazine ring that is coordinated with M through a carbon atom, a pyridazine ring that is coordinated with M through a carbon atom, a pyridine ring that is coordinated with M through a carbon atom, a thiophene ring that is coordinated with M through a carbon atom, a furan ring that is coordinated with M through a carbon atom, an imidazole ring, an oxazole ring that is coordinated with M through a carbon atom, a thiazole ring that is coordinated with M through a carbon atom, an oxadiazole ring that is coordinated with M through a carbon atom, a thiadiazole ring that is coordinated with M through a carbon atom, an isoxazole ring that is coordinated with M through a carbon atom, an isothiazole ring that is coordinated with M through a carbon atom, a triazole ring, a pyrazole ring, a pyrrole ring, and a benzene ring.

Among these, the ring A' is preferably an imidazole ring, a triazole ring, a pyrazole ring, a pyrrole ring, a pyridine ring that is coordinated with M through a carbon atom, or a thiophene ring that is coordinated with M through a carbon atom, and more preferably a pyrazole ring, a pyrrole ring, an imidazole ring, or a triazole ring.

Examples of the ring A' include the rings exemplified for the ring A.

A ring A" represents a pyrazole ring.

A ring B represents a ring structure selected from among a pyrimidine ring, a triazine ring, an imidazole ring, an oxazole ring, a thiazole ring, an oxadiazole ring, a thiadiazole ring, a triazole ring, a pyrazole ring, a pyrrole ring, and a benzene ring.

The ring B is preferably a pyrimidine ring, a triazine ring, a thiazole ring, an oxazole ring, an imidazole ring, a pyrrole ring, a benzene ring, or a triazole ring, and more preferably a pyrrole ring, a benzene ring, an imidazole ring, or a triazole ring.

A ring B' represents a ring structure selected from among a pyrimidine ring that is coordinated with M through a carbon atom, an imidazole ring, an oxazole ring that is coordinated with M through a carbon atom, a thiazole ring that

is coordinated with M through a carbon atom, an oxadiazole ring that is coordinated with M through a carbon atom, a thiadiazole ring that is coordinated with M through a carbon atom, a triazole ring, a pyrazole ring, a pyrrole ring, and a benzene ring.

The ring B' is preferably a triazole ring that is coordinated with M through a nitrogen atom, an imidazole ring, a pyrrole ring, or a benzene ring that is coordinated with M through a carbon atom.

Examples of the ring B and the ring B' will be shown below, but the present invention is not limited thereto. In the following examples, * represents a position in which the ring B or the ring B' binds to the ring A or a pyridine ring. 5N-6, 5N-8, and 5N-10 bind to M through a nitrogen atom having undergone proton dissociation. 5N-7 binds to M through a carbon atom (carbene). pm-7 binds to M through a carbon anion.

Examples of the ring B include pm-1 to pm-7, ta-1 to ta-6, and 5N-1 to 5N-10, and examples of the ring B' include pm-7, 5N-6 to 5N-8, and SN-10.

Herein, "Et" represents ethyl.

[Chem. 34]

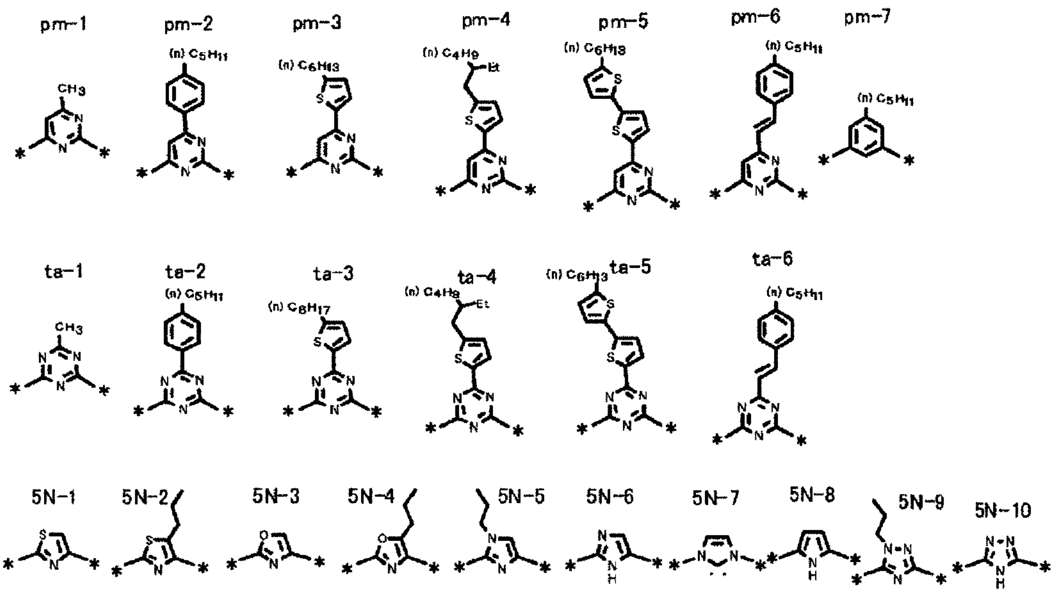

pm-1 pm-2 pm-3 pm-4 pm-5 pm-6 pm-7

ta-1 ta-2 ta-3 ta-4 ta-5 ta-6

5N-1 5N-2 5N-3 5N-4 5N-5 5N-6 5N-7 5N-8 5N-9 5N-10

A ring D' represents an aromatic hydrocarbon ring or a heterocyclic ring. The ring D' has the same definition as the ring D in Formulae (2L-1) to (2L-4), and a preferable range thereof is also the same. Herein, two ring D's present in Formula (3L-6) may be the same as or different from each other.

Each of Ax and Ay independently represents a nitrogen atom, an oxygen atom, or a sulfur atom. Herein, at least one of Ax and Ay is an anion.

Each of Ax and Ay is particularly preferably a residue remaining after active hydrogen is removed from a (substituted) amino group, a hydroxyl group, or a thiol group among functional groups in an aromatic carbon ring and a nitrogen-containing aromatic heterocyclic ring.

In the present invention, from the viewpoint of light fastness, the ligand LD is more preferably a ligand represented by any of Formulae (3L-4) and (3L-5). From the viewpoint of heat resistance, the ligand LD is more preferably a ligand represented by Formula (3L-1). From the viewpoint of resistance to heat cycle, the ligand LD is more preferably a ligand represented by Formula (3L-3). From the viewpoint of initial characteristics, the ligand LD is more preferably a ligand represented by any of Formulae (2L-1) to (2L-4).

Specific examples of LD in Formula (1) will be shown below, but the present invention is not limited thereto. In the following examples, "*" represents a linking site, "Me" represents methyl, and "Et" represents ethyl.

<Examples of LD which is a tridentate ligand having three ring structures including a coordinating atom as a ring-constituting atom and has two pyridine ring structures, in which the pyridine ring structures are adjacent to each other>

[Chem. 35]

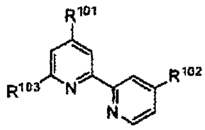

| LD No | $R^{103}$ | $R^{101}$ | $R^{102}$ |
|---|---|---|---|
| LD-1-1 | 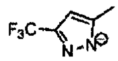 | H | H |
| LD-1-2 | 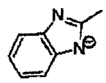 | H | H |
| LD-1-3 | 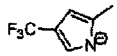 | H | H |
| LD-1-4 | 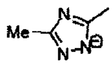 | H | H |
| LD-1-5 | 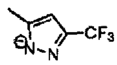 | H | H |
| LD-1-6 | 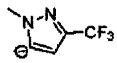 | H | H |
| LD-1-7 | 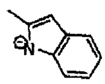 | H | H |
| LD-1-8 | 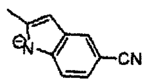 | H | H |
| LD-1-9 | 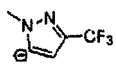 | $CH_3$ | $CH_3$ |
| LD-1-10 | 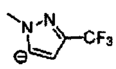 | 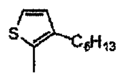 | H |
| LD-1-11 | 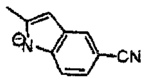 | 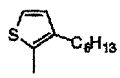 | $CH_3$ |

<Examples of LD which is a tridentate ligand having three ring structures including a coordinating atom as a ring-constituting atom and has one pyridine ring structure, in which the pyridine ring structure is positioned in the middle of the three ring structure>

[Chem. 36]

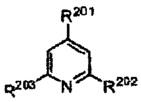

| LD No | $R^{203}$ | $R^{201}$ | $R^{202}$ |
|---|---|---|---|
| LD-2-1 | 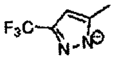 | H | 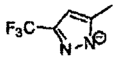 |
| LD-2-2 | 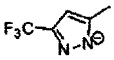 | 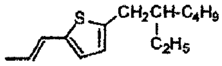 | 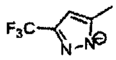 |
| LD-2-3 | 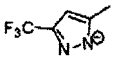 | 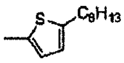 | 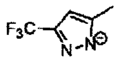 |
| LD-2-4 | 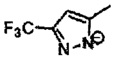 | 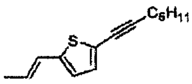 | 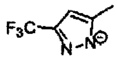 |
| LD-2-5 | 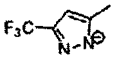 | 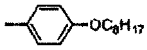 | 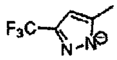 |
| LD-2-6 | 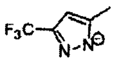 | 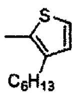 | 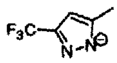 |
| LD-2-7 | 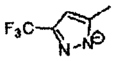 | 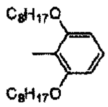 | 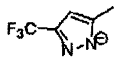 |
| LD-2-8 | 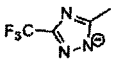 | 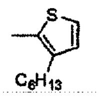 | 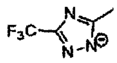 |
| LD-2-9 | 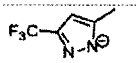 | 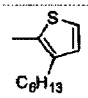 | 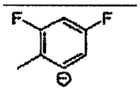 |
| LD-2-10 | 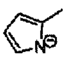 | 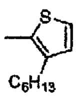 | 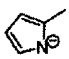 |
| LD-2-11 | 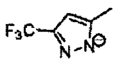 | 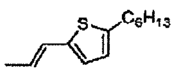 | 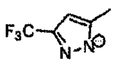 |
| LD-2-12 | 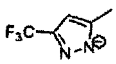 | 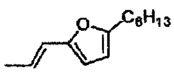 | 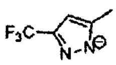 |

<Examples of LD which is a tridentate ligand having three ring structures including a coordinating atom as a ring-constituting atom, in which a 5-membered ring structure containing a heteroatom or a benzene ring structure is positioned in the middle of the three ring structures>

[Chem. 37]

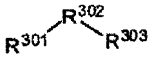

| LD No | $R^{301}$ | $R^{302}$ | $R^{303}$ |
|---|---|---|---|
| LD-3-1 | 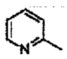 | 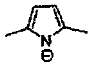 | 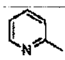 |
| LD-3-2 | 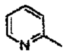 | 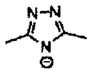 | 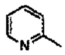 |
| LD-3-3 | 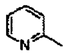 | 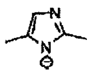 | 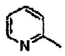 |
| LD-3-4 | 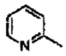 | 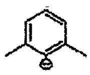 | 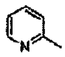 |
| LD-3-6 | 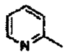 | 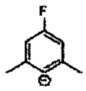 | 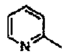 |
| LD-3-6 | 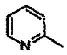 | 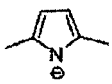 | 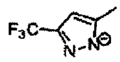 |
| LD-3-7 | 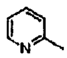 | 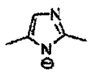 | 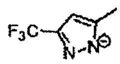 |
| LD-3-8 | 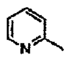 | 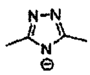 | 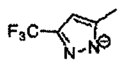 |
| LD-3-9 | 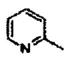 | 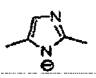 | 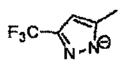 |
| LD-3-10 | 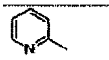 | 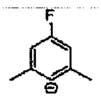 | 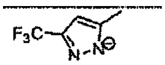 |
| LD-3-11 | 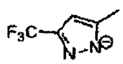 | 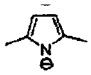 | 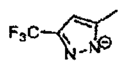 |
| LD-3-12 | 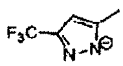 | 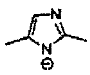 | 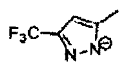 |
| LD-3-13 | 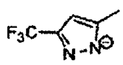 | 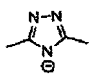 | 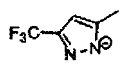 |
| LD-3-14 | | | |
| LD-3-15 | | | |

(continued)

| LD No | $R^{301}$ | $R^{302}$ | $R^{303}$ |
|---|---|---|---|
| LD-3-18 | 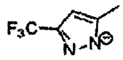 | 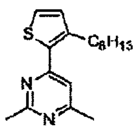 | 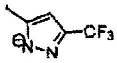 |
| LD-3-17 | 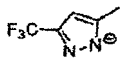 | 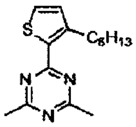 | 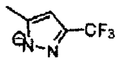 |
| LD-3-18 | 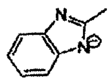 | 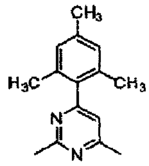 | 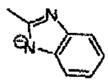 |
| LD-3-19 | 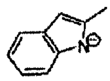 | 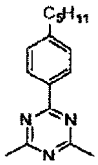 | 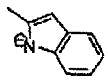 |

[Chem. 38]

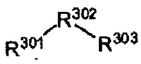

| LD No | $R^{301}$ | $R^{302}$ | $R^{303}$ |
|---|---|---|---|
| LD-3-20 | 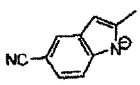 | 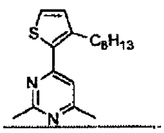 | 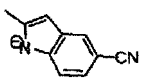 |
| LD-3-21 | 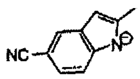 | 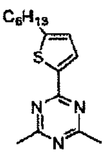 | 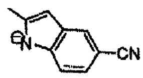 |
| LD-3-22 | 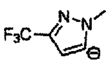 | 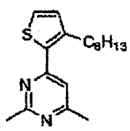 | 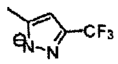 |
| LD-3-23 | 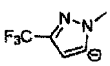 | 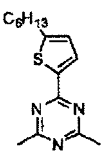 | 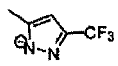 |

(continued)

| LD No | $R^{301}$ | $R^{302}$ | $R^{303}$ |
|---|---|---|---|
| LD-3-24 | 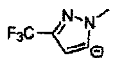 | 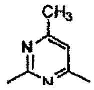 | 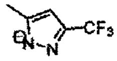 |
| LD-3-25 | 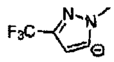 | 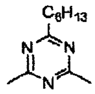 | 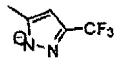 |
| LO-3-26 | 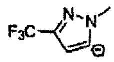 | 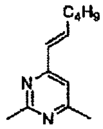 | 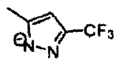 |
| LD-3-27 | 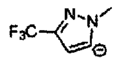 | 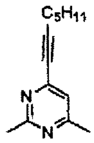 | 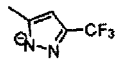 |
| LD-3-28 | 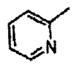 | 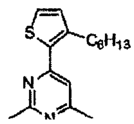 | 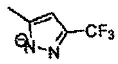 |
| LD-3-29 | 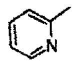 | 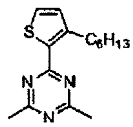 | 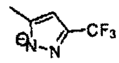 |
| LD-3-30 | 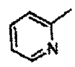 | 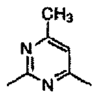 | 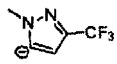 |
| LD-3-31 | 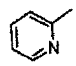 | 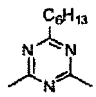 | 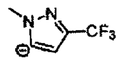 |
| LD-3-32 | 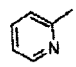 | 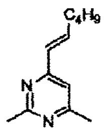 | 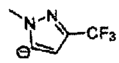 |

[Chem. 39]

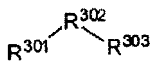

| LD No | $R^{301}$ | $R^{302}$ | $R^{303}$ |
|---|---|---|---|
| LD-3-33 | 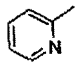 | 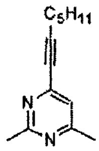 | 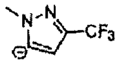 |
| LD-3-34 | 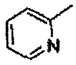 | 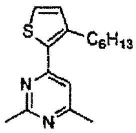 | 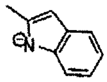 |
| LD-3-35 | 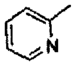 | 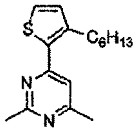 | 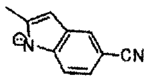 |
| LD-3-36 | 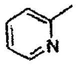 | 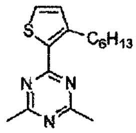 | 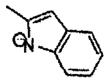 |
| LD-3-37 | 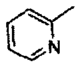 | 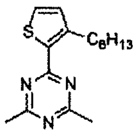 | 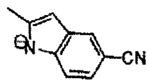 |
| LD-3-38 | 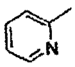 | 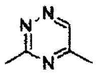 | 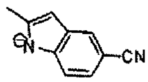 |

<Examples of the ligand represented by Formula (3L-6)>

[Chem. 40]

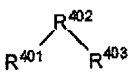

| LD No. | $R^{401}$ | $R^{402}$ | $R^{403}$ |
|---|---|---|---|
| LD-4-1 | 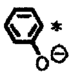 | 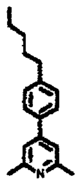 | 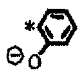 |

(continued)

| LD No. | $R^{401}$ | $R^{402}$ | $R^{403}$ |
|---|---|---|---|
| LD-4-2 | 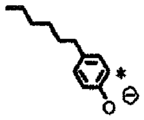 | 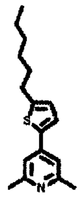 | 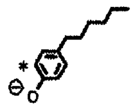 |
| LD-4-3 | 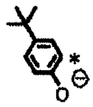 | 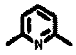 | 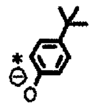 |
| LD-4-4 | 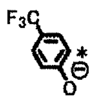 | 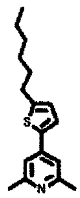 | 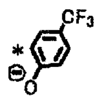 |
| LD-4-5 | 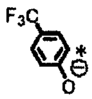 | 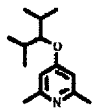 | 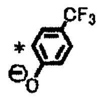 |
| LD-4-6 | 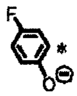 | 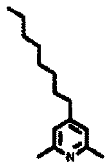 | 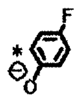 |
| LD-4-7 | 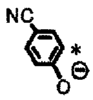 | 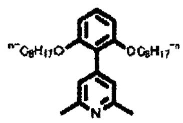 | 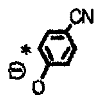 |
| LD-4-8 | 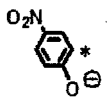 | 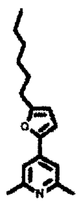 | 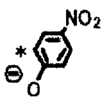 |
| LD-4-9 | 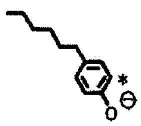 | 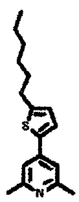 | 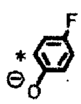 |
| LD-4-10 | 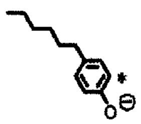 | 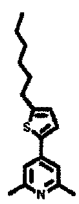 | 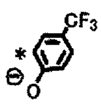 |

[Chem. 41]

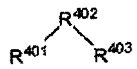

| LD No. | $R^{401}$ | $R^{402}$ | $R^{403}$ |
|---|---|---|---|
| LD-4-11 | 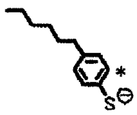 | 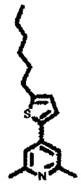 | 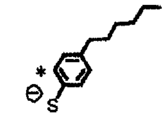 |
| LD-4-12 | 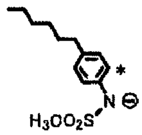 | 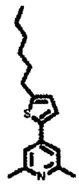 | 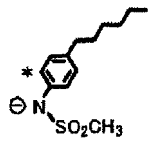 |
| LD-4-13 | 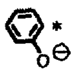 | 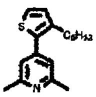 | 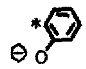 |
| LD-4-14 | 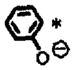 | 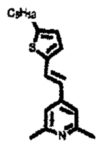 | 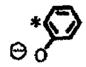 |

In Formula (1), X represents a monodentate ligand. X represents an anion, which is selected from the group consisting of an acyloxy anion, an acylthio anion, a thioacyloxy anion, a thioacylthio anion, an acylaminooxy anion, a thiocarbamate anion, a dithiocarbamate anion, a thiocarbonate anion, a dithiocarbonate anion, a trithiocarbonate anion, an acyl anion, a thiocyanate anion, an isothiocyanate anion, a cyanate anion, an isocyanate anion, a cyano anion, an alkylthio anion, an arylthio anion, an alkoxy anion, and an aryloxy anion, or a monodentate ligand coordinated through these groups. Alternatively, X represents a monodentate ligand selected from the group of anions, atoms, or compounds (including compounds in which a hydrogen atom has been substituted with an anion) consisting of a halogen atom, cyano, carbonyl, dialkylketone, carbonamide, thiocarbonamide, and thiourea. Herein, when the ligand X has an alkyl group, an alkenyl group, an alkynyl group, an alkylene group, and the like, these may be linear or branched or may be substituted or unsubstituted. In addition, when X has an aryl group, a heterocyclic group, a cycloalkyl group, and the like, these may be linear or branched or may be a monocyclic ring or a condensed ring.

In the present invention, X is preferably a cyanate anion, an isocyanate anion, a thioisocyanate anion, an isothiocyanate anion, a selenocyanate anion, or an isoselenocyanate anion, more preferably an isocyanate anion, an isothiocyanate anion, or an isoselenocyanate anion, and particularly preferably an isothiocyanate anion.

In Formula (1), m represents 0 or 1.

In Formula (1), CI represents a counter ion used when the counter ion is required for neutralizing a charge. Generally, whether the dye is a cation or an anion or whether a dye carries net ionic charge depends on the metal, ligand, and substituent in the metal complex dye.

When the substituent has a dissociable group, the metal complex dye represents Formula (1) may be dissociated and carry negative charge. In this case, the charge of the entire metal complex dye represented by Formula (1) becomes electrically neutral due to CI.

When the counter ion CI is a positive counter ion, the counter ion CI is, for example, an inorganic or organic ammonium ion (such as a tetraalkylammonium ion, a pyridinium ion, or the like), a phosphonium ion (such as a tetraalkylphosphonium ion, alkyltriphenyl phosphonium ion, or the like), an alkali metal ion, a metal complex ion, or a proton.

When the counter ion CI is a negative counter ion, the counter ion CI may be an inorganic anion or an organic anion. Examples thereof include a halogen anion (such as a fluoride ion, a chloride ion, a bromide ion, or an iodide ion), a substituted or unsubstituted arylsulfonate ion (such as a p-toluenesulfonate ion or a p-chlorobenzenesulfonate ion), an aryldisulfonate ion (such as a 1,3-benzenedisulfonate ion, a 1,5-naphthalenedisulfoante ion, or a 2,6-naphthalenedisulfonate ion), an alkylsulfate ion (such as methylsulfate ion), a sulfate ion, a thiocyanate ion, a perchlorate ion, a tetrafluoroborate ion, a hexafluorophosphate ion, a picrate ion, an acetate ion, a trifluoromethane sulfonate ion, and the like. Furthermore, as a charge balancing counter ion, either an ionic polymer or other dyes carrying charge opposite to the charge of the dye may be used. Moreover, it is possible to use a metal complex ion (such as bisbenzene-1,2-dithiolatonickel (III)).

In the present invention, CI is preferably an inorganic or organic ammonium ion, and particularly preferably a tetrabutyl ammonium ion, a sodium ion, or a proton.

Next, specific examples of the metal complex dye used in the present invention will be described. Before the specific examples will be described, how to denote the dyes will be described.

When LD is a tridentate ligand and has $Ru^{II}$ as a central metal, the structure of the metal complex dye used in the present invention can be represented by $Ru^{II}$(LA)(LD). For example, a metal complex dye in which LA is LA-1-1 and LD is LD-2-1 is denoted as $Ru^{II}$(LA-1-1)(LD-2-1). The structure of $Ru^{II}$(LA-1-1)(LD-2-1) will be shown below.

[Chem. 42]

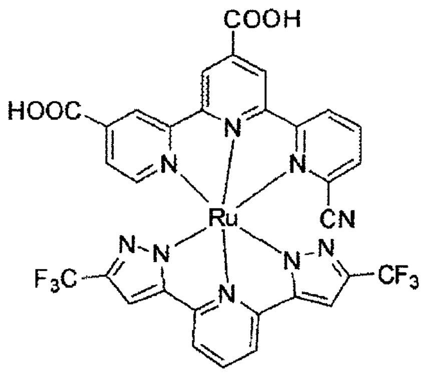

For a dye in which a proton has been substituted with a cation and become a salt, the cation is additionally denoted. For example, if the cation is a tetrabutylammonium salt, the dye is denoted as [$Ru^{II}$(LA-1-1)(LD-2-1)]N+$Bu_4$. The structure of [$Ru^{II}$(LA-1-1)(LD-2-1)]$^-$$N^+$$Bu_4$ will be shown below. Herein, "Bu" in the structural formula represents butyl.

[Chem. 43]

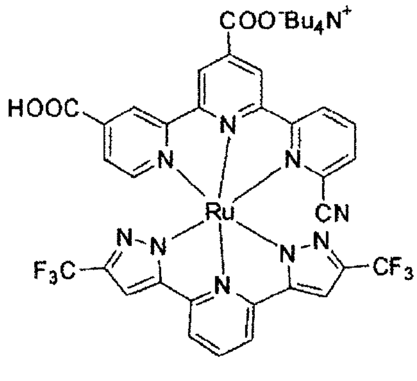

When the tridentate ligand LD is a monovalent anion, the whole complex becomes an anion. Therefore, a counter ion is additionally denoted. For example, a metal complex dye in which LA is LA-1-1 and LD is LD-1-1 is denoted by [$Ru^{II}$(LA-1-1)(LD-1-1)]Cl. Herein, Cl represents a chlorine ion. The structure of [$Ru^{II}$(LA-1-1)(LD-1-1)]Cl will be shown below.

[Chem. 44]

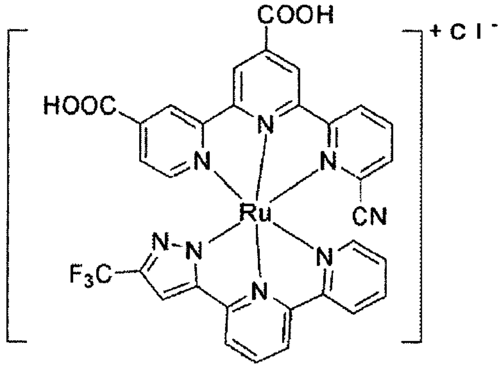

When LD is a bidentate ligand, and the central metal is $Ru^{II}$, the structure of the metal complex dye used in the present invention can be represented by $Ru^{II}$(LA)(LD)(X). For example, a metal complex dye, in which LA is LA-1-1, LD is LD-6-1, and X is an isothiocyanate, is denoted by $Ru^{II}$(LA-1-1)(LD-6-1)(NCS). The structure of such a dye will be shown below.

[Chem. 45]

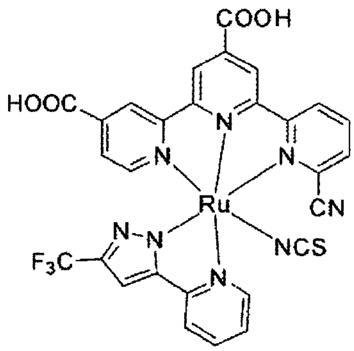

Specific examples of the metal complex used in the present invention will be described below according to the how to denote described above.

$Ru^{II}$(LA-1-1)(LD-1-1)Cl

$Ru^{II}$(LA-1-1)(LD-2-1)

$Ru^{II}$(LA-1-1)(LD-2-3)

$Ru^{II}$(LA-1-1)(LD-2-5)

$Ru^{II}$(LA-1-5)(LD-2-1)

$Ru^{II}$(LA-1-16)(LD-2-1)

$Ru^{II}$(LA-1-10)(LD-2-1)

$Ru^{II}$(LA-1-3)(LD-2-1)

$Ru^{II}$(LA-1-1)(LD-3-1)Cl

$Ru^{II}$(LA-1-4)(LD-3-2)Cl

$Ru^{II}$(LA-1-19)(LD-3-15)$(n\text{-}Bu)_4N^+$

$Ru^{II}$(LA-1-1)(LD-6-1)(NCS)

$Ru^{II}$(LA-1-1)(LD-6-10)(NCS)

$Ru^{II}$(LA-1-1)(LD-6-13)(NCS)

$Ru^{II}$(LA-1-1)(LD-4-16)(NCS)

$Ru^{II}$(LA-1-11)(LD-4-16)(NCS)

$Ru^{II}$(LA-1-1)(LD-5-1)(NCS)

$Ru^{II}$(LA-1-17)(LD-5-1)(NCS)

$Ru^{II}$(LA-1-1)(LD-7-1)(NCS)

$Ru^{II}$(LA-1-11)(LD-7-1)(NCS)

$Ru^{II}$(LA-1-1)(LD-8-1)(NCS)

$Ru^{II}$(LA-1-17)(LD-8-1)(NCS)

$Ru^{II}$(LA-1-1)(LD-9-1)(NCS)

$Ru^{II}$(LA-1-11)(LD-9-1)(NCS)

$Ru^{II}$(LA-1-1)(LD-10-1)(NCS)

$Ru^{II}$(LA-1-11)(LD-10-1)(NCS)

$Ru^{II}$(LA-1-1)(LD-11-1)(NCS)

$Ru^{II}$(LA-1-11)(LD-11-1)(NCS)

$Ru^{II}$(LA-1-1)(LD-12-1)(NCS)

$Ru^{II}$(LA-1-11)(LD-12-1)(NCS)

$Ru^{II}$(LA-1-1)(LD-13-1)(NCS)

$Ru^{II}$(LA-1-11)(LD-13-1)(NCS)

$Ru^{II}$(LA-1-1)(LD-14-1)

$Ru^{II}$(LA-1-11)(LD-14-1)

In the present invention, the metal complex dye used in the present invention may be concurrently used with other dyes.

Examples of the concurrently used dyes include the Ru complex dyes descried in JP1995-500630A (JP-H07-500630A) (particularly, the dyes synthesized in Examples 1 to 19 described in the $5^{th}$ line of left lower column on page 5 to the $7^{th}$ line of right upper column on page 7), the Ru complex dyes described in JP2002-512729A (particularly, the dyes synthesized in Examples 1 to 16 described in the $3^{rd}$ line from the bottom of page 20 to the $23^{rd}$ line on page 29), the Ru complex dyes described in JP2001-59062A (particularly, the dyes described in paragraphs [0087] to [0104]), the Ru complex dyes described in JP2001-6760A (particularly, the dyes described in paragraphs [0093] to [0102]), the Ru complex dyes described in JP2001-253894A (particularly, the dyes described in paragraphs [0009] and [0010]), the

Ru complex dyes described in JP2003-212851A (particularly, the dyes described in paragraph [0005]), the Ru complex dyes described in WO2007/91525A (particularly, the dyes described in paragraphs [0067]), the Ru complex dyes described in JP2001-291534A (particularly, the dyes described in paragraphs [0120] to [0144]), the Ru complex dyes described in JP2012-012570A (particularly, the dyes described in paragraphs [0095] to [0103]), the Ru complex dyes described in JP2013-084594A (particularly, the dyes described in paragraphs [0072] to [0081] and the like), the squarylium cyanine dyes described in JP1999-214730A (JP-H11-214730A) (particularly, the dyes described in paragraphs [0036] to [0047]), the squarylium cyanine dyes described in JP2012-144688A (particularly, the dyes described in paragraphs [0039] to [0046] and paragraphs [0054] to [0060]), the squarylium cyanine dyes described in JP2012-84503A (particularly, the dyes described in paragraphs [0066] to [0076] and the like), organic dyes described in JP2004-063274A (particularly, the dyes described in paragraphs [0017] to [0021]), the organic dyes described in JP2005-123033A (particularly, the dyes described in paragraphs [0021] to [0028]), the organic dyes described in JP2007-287694A (particularly, the dyes described in paragraphs [0091] to [0096]), the organic dyes described in JP2008-71648A (particularly, the dyes described in paragraphs [0030] to [0034]), the organic dyes described in WO2007/119525A (particularly, the dyes described in paragraph [0024]), the porphyrin dyes described in Angew. Chem. Int. Ed., 49, 1 to 5 (2010) and the like, and the phthalocyanine dyes described in Angew. Chem. Int. Ed., 46, 8358 (2007).

Preferable examples of the concurrently used dyes include Ru complex dyes, squarylium cyanine dyes, and organic dyes.

When the metal complex dye used in the present invention is concurrently used with other dyes, a mass ratio of the metal complex dye used in the present invention/other dyes is preferably 95/5 to 10/90, more preferably 95/5 to 50/50, even more preferably 95/5 to 60/40, particularly preferably 95/5 to 65/35, and most preferably 95/5 to 70/30.

(Semiconductor particles)

The semiconductor particles are preferably particles of chalcogenide (such as an oxide, a sulfide, or selenide) of a metal or particles of Perovskite. Preferable examples of the chalcogenide of a metal include titanium, tin, zinc, tungsten, zirconium, hafnium, strontium, indium, cerium, yttrium, lanthanum, vanadium, niobium, an oxide of tantalum, cadmium sulfide, cadmium selenide, and the like. Preferable examples of Perovskite include strontium titanate, calcium titanate, and the like. Among these, titanium oxide (titania), zinc oxide, tin oxide, and tungsten oxide are particularly preferable.

Examples of the crystal structure of titania includes an anatase type, a Brukite-type, and a rutile-type, and among these, an anatase-type and a Brukite-type are preferable. Titania nanotubes, titania nanowires, or titania rods may be mixed with titania particles or used as a semiconductor electrode.

The particle size of the semiconductor particles is a mean particle size obtained by using a diameter determined when the projected area of the particles is converted into a circle. The particle size is preferably 0.001 μm to 1 μm in terms of a primary particle size, and preferably 0.01 μm to 100 μm in terms of a mean particle size of a dispersion. Examples of the method of coating the conductive substrate with semiconductor particles include a wet method, a dry method, and other methods.

In order to prevent reverse current caused by direct contact between the electrolyte and the electrode, it is preferable to form a short circuit-preventing layer between the transparent conductive film and the semiconductor layer (photosensitive layer). Furthermore, a light-scattering layer may be provided. Moreover, in order to prevent contact between the photoelectrode and the counter electrode, it is preferable to use a spacer or a separator. It is preferable for the semiconductor particles to have a large surface area such that the particles can adsorb a large amount of dye. For example, in a state in which the support is coated with the semiconductor particles, the surface area is preferably not less than 10 times the projected area, and more preferably not less than 100 times the projected area. The upper limit thereof is not particularly limited, but usually, the surface area is about 5,000 times the projected area. Generally, the greater the thickness of the layer containing the semiconductor particles, the larger the amount of the dye that can be loaded per unit area, and consequentially, light absorption efficiency is improved. However, because the generated electrons are diffused farther away, the loss resulting from charge recombination also increases. Typically, the thickness of the photosensitive layer is preferably 0.1 μm to 100 μm, although the thickness varies with the use of the element. When the photoelectric conversion element is used as a dye-sensitized solar cell, the thickness is preferably 1 μm to 50 μm, and more preferably 3 μm to 30 μm. After the support is coated with the semiconductor particles, it is preferable to perform a firing treatment on the semiconductor particles. For example, the firing conditions can be set to be 100°C to 800°C and 10 minutes to 10 hours. The temperature at which the photosensitive layer is formed is not particularly limited. However, for example, when glass is used as the conductive substrate, it is preferable to form the layer at 60°C to 400°C.

The amount of the semiconductor particles used for coating is preferably 0.5 g to 500 g and more preferably 5 g to 100 g per 1 $m^2$ of the support. The amount of the dye used is preferably 0.01 mmol to 100 mmol, more preferably 0.1 mmol to 50 mmol, and particularly preferably 0.1 mmol to 10 mmol per 1 $m^2$ of the support. In this case, the amount

of the metal complex dye used in the present invention is preferably equal to or greater than 5 mol%, more preferably 60 mol% to 100 mol%, and even more preferably 85 mol% to 100 mol% with respect to the total amount of the dye. As the dye other than the metal complex dye used in the present invention, a dye that functions alone as a sensitizing dye of a dye-sensitized solar cell is preferable. Furthermore, the amount of the dye adsorbed onto the semiconductor particles is preferably 0.001 mmol to 1 mmol and more preferably 0.1 mmol to 0.5 mmol with respect to 1 g of the semiconductor particles. If the amount of the dye is within the above range, a sensitizing effect in the semiconductor particles is sufficiently obtained.

When the dye is a salt, the counter ion of the aforementioned specific metal complex dye is not particularly limited, and examples of the counter ion include an alkali metal ion, a quaternary ammonium ion, and the like.

In the present invention, for causing a dye to be adsorbed onto the semiconductor particles, it is preferable to use a dye solution containing a dye as described later. For example, by dipping a semiconductor electrode, in which a semiconductor layer (photosensitive layer) is formed on a support, into a dye solution obtained by dissolving a dye, the dye can be adsorbed onto the semiconductor particles.

After the dye is adsorbed onto the semiconductor particles, the surface of the semiconductor particles may be treated with amines. Preferable examples of the amines include pyridines (such as 4-tert-butylpyridine and polyvinylpyridine) and the like. When the amines are in the form of a liquid, they may be used as is or may be used by being dissolved in an organic solvent.

(Coadsorbent)

In the photoelectric conversion element of the present invention, a coadsorbent is preferably used together with the metal complex dye used in the present invention or the dye that is concurrently used if necessary. As the coadsorbent, coadsorbents having one or more acidic groups (preferably a carboxyl group or a group of a salt thereof) are preferable, and examples thereof include fatty acids and compounds having a steroid skeleton. The fatty acid may be saturated fatty acids or unsaturated fatty acids, and examples thereof include butanoic acid, hexanoic acid, octanoic acid, decanoic acid, hexadecanoic acid, dodecanoic acid, palmitic acid, stearic acid, oleic acid, linoleic acid, linolenic acid, and the like.

Examples of the compounds having a steroid skeleton include cholic acid, glycocholic acid, chenodeoxycholic acid, hyocholic acid, deoxycholic acid, lithocholic acid, ursodeoxycholic acid, and the like. Among these, cholic acid, deoxycholic acid, and chenodeoxycholic acid are preferable, and chenodeoxycholic acid is more preferable.

A compound represented by the following Formula (CA) is preferable as the coadsorbent.

[Chem. 46]

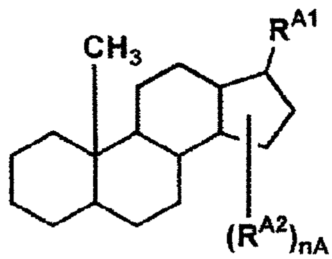

Formula (CA)

In the formula, $R^{A1}$ represents a substituent having the adsorptive group (that is, the adsorptive group or a substituent having the adsorptive group); $R^{A2}$ represents a substituent; and nA representing the number of $R^{A2}$ represents an integer of equal to or greater than 0.

The adsorptive group has the same definition as the aforementioned adsorptive group, and a preferable range thereof is also the same.

nA is preferably 2 to 4.

Among the above, $R^{A1}$ preferably represents an alkyl group substituted with a carboxyl group, a sulfo group, or a salt thereof, and more preferably represents $-CH(CH_3)CH_2CH_2CO_2H$ and $-CH(CH_3)CH_2CH_2CONHCH_2CH_2SO_3H$.

Examples of $R^{A2}$ include the substituents T which will be described later. Among the substituents T, an alkyl group, a hydroxy group, an acyloxy group, and an alkylaminocarbonyloxy group, an arylaminocarbonyloxy group are preferable, and an alkyl group, a hydroxy group, and an acyloxy group are more preferable.

Examples of these specific compounds include the compounds exemplified as the compounds having a steroid

skeleton.

The coadsorbent used in the present invention is adsorbed onto the semiconductor particles, and in this way, the coadsorbent exerts an effect of inhibiting inefficient intermixing of dyes and an effect of preventing electrons from moving back to the redox system in the electrolyte from the surface of the semiconductor particles. The amount of the coadsorbent used is not particularly limited. However, from the viewpoint of making the coadsorbent excellently exert the aforementioned effects, the amount thereof is preferably 1 mol to 200 mol, more preferably 10 mol to 150 mol, and particularly preferably 20 mol to 50 mol, with respect to 1 mol of the sensitizing dye.

<Conductive substrate>

The conductive substrate is preferably a metal support having conductivity. Alternatively, it is preferably a glass or plastic support having a conductive film layer on the surface. Examples of the plastic support include the transparent polymer film described in paragraph [0153] of JP2001-291534A. As the support, in addition to glass or plastic, ceramics (JP2005-135902A) and conductive resins (JP2001-160425A) may be used. A light management function may be added to the surface of the conductive substrate. For example, the conductive substrate may have an antireflection film described in JP2003-123859A that is obtained by alternately laminating a high-refractive index film and a low-refractive index oxide film, or may have a light guide function described in JP2002-260746A.

The thickness of the conductive film layer is preferably 0.01 μm to 30 μm, more preferably 0.03 μm to 25 μm, and particularly preferably 0.05 μm to 20 μm.

It is preferable for the conductive substrate to be substantially transparent. If the conductive substrate is "substantially transparent", it means that the light transmittance thereof is equal to or greater than 10%. The light transmittance is preferably equal to or greater than 50%, and particularly preferably equal to or greater than 80%. As the transparent conductive substrate, a support obtained by coating glass or plastic with a conductive metal oxide is preferable. As the metal oxide, tin oxide is preferable, and indium tin oxide and a fluorine-doped oxide are particularly preferable. At this time, the amount of the conductive metal oxide used for coating is preferably 0.1 g to 100 g per 1 $m^2$ of the glass or plastic support. When the transparent conductive substrate is used, it is preferable to allow light to enter from the support side.

<Charge transfer layer>

The charge transfer layer used in the photoelectric conversion element of the present invention is a layer that functions to supply electrons to the oxidized dye and is disposed between the light-receiving electrode and the counter electrode. The charge transfer layer contains an electrolyte. Examples of the electrolytes include a liquid electrolyte obtained by dissolving redox pairs in an organic solvent, so-called a gel electrolyte obtained by impregnating a polymer matrix with a solution obtained by dissolving redox pairs in an organic solvent, molten salts containing redox pairs, and the like. In order to improve the photoelectric conversion efficiency, it is preferable to use a liquid electrolyte. As the solvent of the liquid electrolyte, nitrile compounds, ether compounds, ester compounds, and the like are used. Among these, nitrile compounds are preferable, and acetonitrile and methoxypropionitrile are particularly preferable.

Examples of the redox pairs include a combination of iodine and an iodide (preferably an iodide salt or iodized ionic liquid including lithium iodide, tetrabutylammonium iodide, tetrapropylammonium iodide, and methyl propylimidazolium iodide), a combination of alkylviologen (such as methylviologen chloride, hexylviologen bromide, or benzylviologen tetrafluoroborate) and a reduced product thereof, a combination of polyhydroxybenzenes (such as hydroquinone and naphthohydroquinone) and a reduced product thereof, a combination of a divalent iron complex and a trivalent iron complex (such as a combination of potassium ferricyanide and potassium ferrocyanide), a combination of a divalent cobalt complex and a trivalent cobalt complex, and the like. Among these, a combination of iodine and an iodide and a combination of a divalent cobalt complex and a trivalent cobalt complex are preferable.

The cobalt complex is particularly preferably a complex represented by the following Formula (CC).

Co(LL)ma(X)mb·CI Formula (CC)

In Formula (CC), LL represents a bidentate or tridentate ligand; X represents a monodentate ligand; ma represents an integer of 0 to 3; mb represents an integer of 0 to 6; and CI represents a counter ion used when a counter ion is required for neutralizing a charge.

CI has the same definition as CI in Formula (1), and a preferable range thereof is also the same.

LL is preferably a ligand represented by the following Formula (LC).

[Chem. 47]

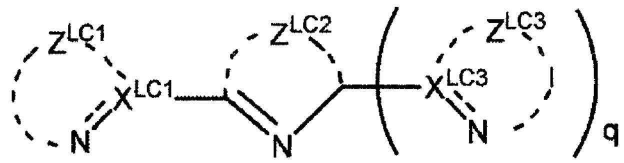

Formula (LC)

In Formula (LC), each of $X^{LC1}$ and $X^{LC3}$ independently represents a carbon atom or a nitrogen atom. Herein, when $X^{LC1}$ is a carbon atom, the bond between $X^{LC1}$ and a N atom is a double bond ($X^{LC1}$=N). When $X^{LC3}$ is a carbon atom, a bond between $X^{LC3}$ and a N atom represents a double bond ($X^{LC3}$=N). When $X^{LC1}$ is a nitrogen atom, the bond between $X^{LC1}$ and a N atom is a single bond ($X^{LC1}$-N). When $X^{LC3}$ is a nitrogen atom, the bond between $X^{LC3}$ and a N atom is a single bond ($X^{LC3}$-N).

Each of $Z^{LC1}$, $Z^{LC2}$, and $Z^{LC3}$ independently represents a non-metallic atomic group necessary for forming a 5- or 6-membered ring. $Z^{LC1}$, $Z^{LC2}$, and $Z^{LC3}$ may have a substituent and may form a closed ring via the substituent against a ring adjacent thereto. q represents 0 or 1. Examples of the substituent include the substituents T which will be described later. When q is 0, a hydrogen atom or a substituent other than a heterocyclic group formed by $Z^{LC3}$ binds to a carbon atom in a position in which $X^{LC3}$ binds to the 5- or 6-membered ring formed by $Z^{LC2}$.

X is preferably a halogen ion.

The ligand represented by Formula (LC) is more preferably a ligand represented by the following Formulae (LC-1) to (LC-4).

[Chem. 48]

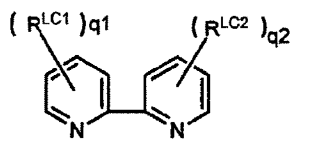

Formula (LC-1)

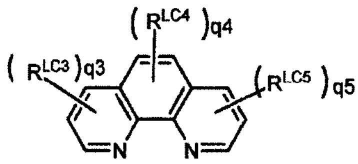

Formula (LC-2)

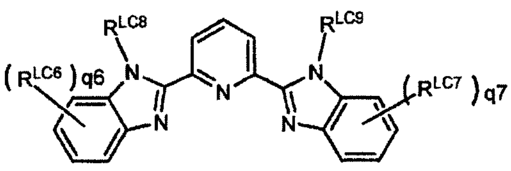

Formula (LC-3)

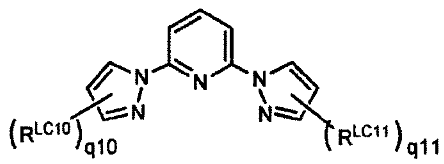

Formula (LC-4)

Each of $R^{LC1}$ to $R^{LC11}$ independently represents a substituent. Each of q1, q2, q6, and q7 independently represents an integer of 0 to 4. Each of q3, q5, q10, and q11 independently represents an integer of 0 to 3. q4 represents an integer of 0 to 2.

Specific examples of the substituents represented by $R^{LC1}$ to $R^{LC11}$ in Formulae (LC-1) to (LC-4) include an aliphatic group, an aromatic group, a heterocyclic group, and the like. Specific examples of the substituents include an alkyl group, an alkoxy group, an alkylthio group, an aryl group, an aryloxy group, an arylthio group, a heterocyclic group, and the like. Preferable examples thereof include an alkyl group (such as methyl, ethyl, n-butyl, n-hexyl, isobutyl, sec-butyl, t-butyl, n-dodecyl, cyclohexyl, or benzyl), an aryl group (such as phenyl, tolyl, or naphthyl), an alkoxy group (such as methoxy, ethoxy, isopropoxy, or butoxy), an alkylthio group (such as methylthio, n-butylthio, n-hexylthio, or 2-ethylhexylthio), an aryloxy group (such as phenoxy or naphtoxy), and arylthio group (such as phenylthio, nahthylthio, or 2-thienylthio), and a heterocyclic group (such as 2-thienyl or 2-furyl).

Specific examples of the cobalt complex having the ligand represented by Formula (LC) include the following compounds.

[Chem. 49]

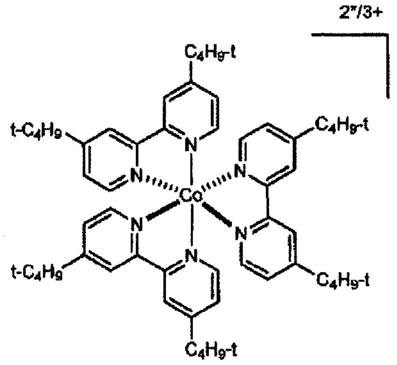

LL-1

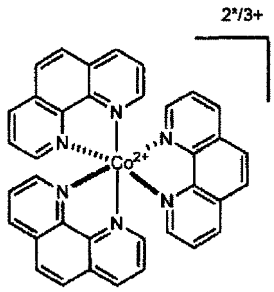

LL-2

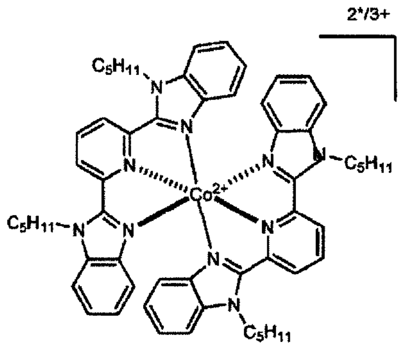

LL-3

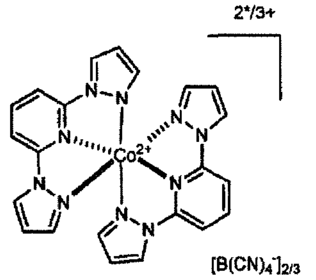

LL-4

When a combination of iodine and an iodide is used as an electrolyte, it is preferable to concurrently use an iodine salt of a nitrogen-containing aromatic cation of a 5- or 6-membered ring. Particularly, when the compound represented by Formula (1) is not an iodine salt, it is preferable to concurrently use an iodine salt such as a pyridinium salt, an imidazolium salt, and a triazolium salt described in WO95/18456A, JP1996-259543A (JP-H08-259543A), Electrochemistry, Vol. 65, No. 11, p.923 (1997), and the like.

As the organic solvent dissolving the redox pairs, nonprotonic polar solvents (such as acetonitrile, propylene carbonate, ethylene carbonate, dimethyl formamide, dimethyl sulfoxide, sulfolane, 1,3-dimethyl imidazolinone, and 3-methyl oxazolidionone) are preferable. Examples of the polymer used as the matrix of a gel electrolyte include polyacrylonitrile, polyvinylidene fluoride, and the like. Examples of the molten salt include those obtained by mixing polyethylene oxide with lithium iodide and at least one kind of another lithium salt (such as lithium acetate or lithium perchlorate) such that the resultant exhibit fluidity at room temperature. In this case, the amount of the polymer added is 1% by mass to 50% by mass. Furthermore, the electrolytic solution may contain $\gamma$-butyrolactone, and in this case, the iodide ion is more efficiently diffused, hence the conversion efficiency is improved.

As additives, an aminopyridine-based compound, a benzimidazole-based compound, an aminotriazole-based compound, an aminothiazole-based compound, an imidazole-based compound, an aminotriazine-based compound, a urea derivative, an amide compound, a pyrimidine-based compound, and a heterocyclic not containing nitrogen can be added to the electrolyte, in addition to the aforementioned 4-tert-butylpyridine.

Moreover, in order to improve the photoelectric conversion efficiency, a method of controlling water content in the electrolyte may be adopted. Preferable examples of the method of controlling water content include a method of controlling concentration and a method of adding a dehydrating agent to the electrolyte. In order to reduce toxicity of urea, a clathrate compound of urea and cyclodextrin may be used, or a method of consistently supplying water may be used. In addition, cyclic amidine may be used, or an antioxidant, a hydrolysis inhibitor, a stabilizing agent, or zinc iodide may be added.

Molten salts may be used as the electrolyte, and examples of preferable molten salts include an ionic liquid containing imidazolium or a triazolium-type cation, an oxazolium-based molten salt, a pyridinium-based molten salt, a guanidium-based molten salt, and a combination of these. These cationic molten salts may be combined with a specific anion. Furthermore, additives may be added to these molten salts, and the molten salts may have a liquid crystalline substituent. Moreover, quaternary ammonium salt-based molten salts may be used.

Examples of molten salts other than the above include those obtained by missing polyethylene oxide to lithium

iodide and at least one kind of another lithium salt (such as lithium acetate or lithium perchlorate) such that the resultant exhibits fluidity at room temperature.

A gellant may be added to the electrolytic solution composed of the electrolyte and a solvent so as to cause gelation and make the electrolyte into a quasi-solid. Examples of the gellant include an organic compound having a molecular weight of equal to or less than 1,000, a Si-containing compound having a molecular weight within a range of 500 to 5,000, an organic salt formed of specific acidic compound and basic compound, a sorbitol derivative, and polyvinylpyridine.

Furthermore, a method of trapping a matrix polymer, a crosslinked polymer compound or monomer, a crosslinking agent, an electrolyte, and a solvent in a polymer may be used.

Preferable examples of the matrix polymer include polymers having a nitrogen-containing heterocyclic ring in a repeating unit of a main chain or a side chain, crosslinked polymers obtained by reacting the aforementioned polymers with an electrophilic compound, polymers having a triazine structure, polymers having a uride structure, polymers containing a liquid crystalline compound, polymers having an ether bond, polyvinylidene fluoride-based polymers, polymers based on methacrylate or acrylate, thermosetting resins, crosslinked polysiloxane, PVA, clathrate compounds of polyalkylene glycol, dextrin, and the like, polymers to which a oxygen-containing polymer or a sulfur-containing polymer has been added, natural polymers, and the like. To these polymers, an alkali-swellable polymer, a polymer having a compound that can form a charge carrier complex composed of a cationic moiety and iodine in a single polymer, and the like may be added.

As the matrix polymer, polymers including a crosslinked polymer, which is obtained by reacting isocyanate having two or more functional groups with a functional group such as a hydroxyl group, an amino group, or a carboxyl group, may be used. Furthermore, a crosslinked polymer composed of a hydrosilyl group and a compound having a double bond may be used. In addition, a crosslinking method in which polysulfonic acid, polycarboxylic acid, or the like is reacted with a metallic ion compound having two or more functional groups may be used.

Examples of solvents that can be preferably used in combination with the aforementioned quasi-solidified electrolyte include a specific phosphoric acid ester, a mixed solvent containing ethylene carbonate, a solvent having a specific dielectric constant, and the like. Moreover, a method of keeping a solution of liquid electrolyte in a solid electrolyte membrane or in small pores, and preferable examples of such a method include methods using a conductive polymer film, a fibrous solid, or a cloth-like solid such as a filter.

Instead of the liquid electrolyte and the quasi-solidified electrolyte described above, a solid charge transport layer such as a p-type semiconductor or a hole transport material, for example, CuI, CuNSC, or the like can be used. Furthermore, the electrolyte described in Nature, vol. 486, p.487 (2012) and the like may be used. As the solid charge transport layer, an organic hole transport material may be used. Preferable examples of the hole transport layer include conductive polymers such as polythiophene, polyaniline, polypyrrole, and polysilane; spiro compounds in which two rings share a central element such as C or Si that forms a tetrahedral structure, aromatic amine derivatives such as triaryl amine, triphenylene derivatives, nitrogen-containing heterocyclic derivatives, and liquid crystalline cyano derivatives.

The redox pairs become electron carriers. Therefore, the total concentration thereof is preferably equal to or greater than 0.01 mol/L, more preferably equal to or greater than 0.1 mol/L, and particularly preferably equal to or greater than 0.3 mol/L. The upper limit of the total concentration of the redox pairs is not particularly limited, but is generally about 5 mol/L.

<Counter electrode>

The counter electrode functions as a positive electrode of the dye-sensitized solar cell (photoelectrochemical cell). Although the counter electrode generally has the same definition as the aforementioned conductive substrate, the support is not essentially required when the solar cell is constituted to sufficiently maintain the strength. It is preferable for the counter electrode to have a structure exerting a high current-collecting effect. In order to allow light to reach the photosensitive layer, at least one of the conductive substrate and the counter electrode should be substantially transparent. In the dye-sensitized solar cell of the present invention, it is preferable to make the conductive substrate transparent and to cause sunlight to enter from the side of the support. In this case, it is more preferable for the counter electrode to have a property of reflecting the light. As the counter electrode of the dye-sensitized solar cell, glass or plastic onto which a metal or a conductive oxide has been vapor-deposited is preferable, and glass onto which platinum has been vapor-deposited is particularly preferable.

In the photoelectric conversion element and the dye-sensitized solar cell of the present invention, in order to prevent evaporation of constituents thereof, it is preferable to seal the lateral surface of the cell side by using a polymer, an adhesive, or the like.

The present invention can be applied to the photoelectric conversion elements and the dye-sensitized solar cells described in JP4260494B, JP2004-146425A, JP2000-340269A, JP2002-289274A, JP2004-152613A, and

JP1997-27352A (JP-H09-27352A). Furthermore, the present invention can be applied to the photoelectric conversion elements and the dye-sensitized solar cells described in JP2004-152613A, JP2000-90989A, JP2003-217688A, JP2002-367686A, JP2003-323818A, JP2001-43907A, JP2000-340269A, JP2005-85500A, JP2004-273272A, JP2000-323190A, JP2000-228234A, JP2001-266963A, JP2001-185244A, JP2001-525108A, JP2001-203377A, JP2000-100483A, JP2001-210390A, JP2002-280587A, JP2001-273937A, JP2000-285977A, JP2001-320068A, and the like.

«Dye solution and manufacturing method of dye-adsorbed electrode and dye-sensitized solar cell using the dye solution»

In the present invention, it is preferable to manufacture dye-adsorbed electrodes (a photoelectrode and a dye-adsorbed conductor electrode) by using a dye solution containing the metal complex dye used in the present invention.

In such a dye solution, the metal complex dye used in the present invention is dissolved in a solvent, and if necessary, the dye solution may contain a coadsorbent or other components.

Examples of the solvent include the solvents described in JP2001-291534A, but the solvent is not particularly limited. In the present invention, an organic solvent is preferable, and alcohols, amides, nitriles, hydrocarbons, and a mixed solvent consisting of two or more kinds of these are more preferable. The mixed solvent is preferably a mixed solvent obtained by mixing alcohols with a solvent selected from among amides, nitriles, or hydrocarbons, more preferably a mixed solvent consisting of alcohols and amides or consisting of alcohols and hydrocarbons, and particularly preferably a mixed solvent consisting of alcohols and amides. Specifically, methanol, ethanol, propanol, butanol, dimethylformamide, and dimethylacetamide are preferable.

The dye solution preferably contains a coadsorbent. As the coadsorbent, the aforementioned coadsorbetns are preferable, and among these, the compound represented by Formula (CA) is preferable.

Herein, it is preferable to adjust the concentration of the metal complex dye or the coadsorbent in the dye solution used in the present invention, such that the solution can be used as is for manufacturing the photoelectric conversion element or the dye-sensitized solar cell. In the present invention, the dye solution preferably contains the metal complex dye used in the present invention, in an amount of 0.001% by mass to 0.1% by mass with respect to the total mass of the dye solution..

It is particularly preferable to adjust the water content in the dye solution. Therefore, in the present invention, it is preferable to adjust the water content to be 0% by mass to 0.1% by mass.

Likewise, in order to make the effects of the present invention excellently exerted, it is preferable to adjust the water content of the electrolyte in the photoelectric conversion element or the dye-sensitized solar cell. Therefore, it is preferable to adjust the water content of the electrolyte to be 0% by mass to 0.1% by mass. It is particularly preferable to use the dye solution to prepare the electrolytic solution.

(Substituent group T)

In the present specification, a "compound (including a complex and dye)" means not only the compound itself but also a salt, a complex, and an ion thereof. Furthermore, within a range that brings about intended effects, it also means a derivative obtained by changing a predetermined portion of the compound. In addition, in the present specification, when there is no description regarding whether or not a substituent is substituted or unsubstituted, it means that the substituent may have any substituent (the same will applied to a linking group and a ligand). Similarly, when there is not description regarding whether or not a compound is substituted or unsubstituted, the compound may have any substituent. Examples of preferable substituents include the following substituent group T.

Moreover, in the present specification, when a group is simply described as a "substituent", the substituent group T can be referred to for the "substituent". In addition, when a group is simply described as, for example, an "alkyl group", a preferable range and specific examples of groups corresponding to the substituent group T are applied to the group.

Examples of the substituent group T include the following.

An alkyl group (preferably having 1 to 20 carbon atoms, for example, methyl, ethyl, isopropyl, t-butyl, pentyl, heptyl, 1-ethylpentyl, benzyl, 2-ethoxyethyl, 1-carboxymethyl, or trifluoromethyl), an alkenyl group (preferably having 2 to 20 carbon atoms, for example, vinyl, allyl, or oleyl), an alkynyl group (preferably having 2 to 20 carbon atoms, for example, ethynyl, butadienyl, or phenylethynyl), a cycloalkyl group (preferably having 3 to 20 carbon atoms, for example, cyclopropyl, cyclopentyl, cyclohexyl, or 4-methylcyclohexyl), a cycloalkenyl group (preferably having 5 to 20 carbon atoms, for example, cyclopentenyl or cyclohexenyl), an aryl group (preferably having 6 to 26 carbon atoms, for example, phenyl, 1-naphthyl, 4-methoxyphenyl, 2-chlorophenyl, or 3-methylphenyl), a heterocyclic group (preferably having 2 to 20 carbon atoms; the heterocyclic group is more preferably a heterocyclic group in the form of a 5- or 6-membered ring having at least one oxygen atom, sulfur atom, or nitrogen atom in ring-constituting atoms, for example, 2-pyridyl, 4-pyridyl, 2-imidazolyl, 2-benzimidazolyl, 2-thiazolyl, or 2-oxazolyl), an alkoxy group (preferably having 1 to 20 carbon

atoms, for example, methoxy, ethoxy, isopropyloxy, or benzyloxy), an alkenyloxy group (preferably having 2 to 20 carbon atoms, for example, vinyloxy or allyloxy), an alkynyloxy group (preferably having 2 to 20 carbon atoms, for example, 2-propynyloxy or 4-butynyloxy), a cycloalkyloxy group (preferably having 3 to 20 carbon atoms, for example, cyclopropyloxy, cyclopentyloxy, cyclohexyloxy, or 4-methylcyclohexyloxy), an aryloxy group (preferably having 6 to 26 carbon atoms, for example, phenoxy, 1-naphthyloxy, 3-methylphenoxy, or 4-methoxyphenoxy), a heterocyclic oxy group (for example, imidazolyloxy, benzimidazolyloxy, thiazolyloxy, benzothiazolyloxy, triazinyloxy, or purinyloxy),

an alkoxycarbonyl group (preferably having 2 to 20 carbon atoms, for example, ethoxycarbonyl or 2-ethylhexyloxycarbonyl), a cycloalkoxycarbonyl group (preferably having 4 to 20 carbon atoms, for example, cyclopropyloxycarbonyl, cyclopentyloxycarbonyl, or cyclohexyloxycarbonyl), an aryloxycarbonyl group (preferably having 6 to 20 carbon atoms, for example, phenyloxycarbonyl or naphthyloxycarbonyl), an amino group (preferably having 0 to 20 carbon atoms, including an alkylamino group, an alkenylamino group, an alkynylamino group, a cycloalkylamino group, a cycloalkenylamino group, an arylamino group, and a heterocyclic amino group, for example, amino, N,N-dimethylamino, N,N-diethylamino, N-ethylamino, N-allylamino, N-(2-propynyl)amino, N-cyclohexylamino, N-cyclohexenylamino, aniline, pyridylamino, imidazolylamino, benzimidazolylamino, thiazolylamino, benzothiazolylamino, or triazinylamino), a sulfamoyl group (preferably having 0 to 20 carbon atoms; the sulfamoyl group is preferably a sulfamoyl group of alkyl, cycloalkyl, or aryl, for example, N,N-dimethylsulfamoyl, N-cyclohexylsulfamoyl, or N-phenylsulfamoyl), an acyl group (preferably having 1 to 20 carbon atoms, for example, acetyl, cyclohexylcarbonyl, or benzoyl), an acyloxy group (preferably having 1 to 20 carbon atoms, for example, acetyloxy, cyclohexylcarbonyloxy, or benzoyloxy), a carbamoyl group (preferably having 1 to 20 carbon atoms; the carbamoyl group is preferably a carbamoyl group of alkyl, cycloalkyl, or aryl, for example, N,N-dimethylcarbamoyl, N-cyclohexylcarbamoyl, or N-phenylcarbamoyl),

an acylamino group (preferably an acylamino group having 1 to 20 carbon atoms, for example, acetylamino, cyclohexylcarbonylamino, or benzoylamino), a sulfonamide group (preferably having 0 to 20 carbon atoms; the sulfonamide group is preferably a sulfonamide group of alkyl, cycloalkyl, or aryl, for example, methanesulfonamide, benzenesulfonamide, N-methylmethanesulfonamide, N-cyclohexylsulfonamide, or N-ethylbenzenesulfonamide), an alkylthio group (preferably having 1 to 20 carbon atoms, for example, methylthio, ethylthio, isopropylthio, or benzylthio), a cycloalkylthio group (preferably having 3 to 20 carbon atoms, for example, cyclopropylthio, cyclopentylthio, cyclohexylthio, or 4-methylcyclohexylthio), an arylthio group (preferably having 6 to 26 carbon atoms, for example, phenylthio, 1-naphthylthio, 3-methylphenylthio, or 4-methoxyphenylthio), an alkylsulfonyl group, a cycloalkylsulfonyl group, or an arylsulfonyl group (preferably having 1 to 20 carbon atoms, for example, methylsulfonyl, ethylsulfonyl, cyclohexylsulfonyl, or benzenesulfonyl),

a silyl group (preferably having 1 to 20 carbon atoms; the silyl group is preferably a silyl group substituted with alkyl, aryl, alkoxy, and aryloxy, for example, triethylsilyl, triphenylsilyl, diethylbenzylsilyl, or dimethylphenylsilyl), a silyloxy group (preferably having 1 to 20 carbon atoms; the silyloxy group is preferably a silyloxy group substituted with alkyl, aryl, alkoxy, and aryloxy, for example, triethylsilyloxy, triphenylsilyloxy, diethylbenzylsilyloxy, or dimethylphenylsilyloxy), a hydroxyl group, a cyano group, a nitro group, a halogen atom (such as a fluorine atom, a chlorine atom, a bromine atom, or an iodine atom), a carboxyl group, a sulfo group, a phosphonyl group, a phosphoryl group, and a boric acid group.

When a compound, a substituent, and the like have an alkyl group, an alkenyl group, and the like, these may be linear or branched or may be substituted or unsubstituted. Furthermore, when a compound, a substituent, and the like have an aryl group, a heterocyclic group, and the like, these may be monocyclic or polycyclic or may be substituted or unsubstituted.

## Examples

Hereinafter, the present invention will be more specifically described based on examples, but the present invention is not limited thereto.

<Synthesis example>

(Stannylation reaction)

A substrate in an amount of about 5 g substituted with halogen, bistributyltin in a molar quantity 1.2 times greater than that of the substrate, and tetrakis triphenylphosphine palladium in a molar quantity 0.05 times greater than that of the substrate were subjected to reflux in 100 ml of toluene in a nitrogen atmosphere. By thin layer chromatography, the state in which the reaction ended was confirmed. After being cooled to room temperature, the reaction liquid was filtered and concentrated. The obtained crude product was subjected to separation purification by using a fractionation column chromatography instrument (AI-580 manufactured by YAMAZEN CORPORATION) and a mixed solvent consisting of n-hexane, ethyl acetate, and methanol as an eluent, in a state in which the concentration gradient thereof was being controlled. By concentrating a target fraction, a stannylated product was obtained.

(Stille coupling)

A substrate in an amount of about 2 g substituted with halogen, the stannylated product in a molar quantity 1.3 times greater than that of the substrate, palladium (II) chloride in a molar quantity 0.05 times greater than that of the substrate, copper (I) iodide in a molar quantity 0.1 times greater than that of the substrate, cesium fluoride in a molar quantity 2 times greater than that of the substrate, and tri-t-butylphosphine in a molar quantity 0.1 times greater than that of the substrate were heated to 80°C in 100 ml of N,N-dimethylacetamide in a nitrogen atmosphere. By thin layer column chromatography, the state in which the reaction ended was confirmed. Thereafter, the reaction liquid was filtered, cooled to room temperature, and then concentrated. The obtained crude product was separation purification by using the same fractionation column chromatography instrument and eluent as described above. By concentrating a target fraction, a target substance was obtained.

-Synthesis of dimethyl ester of ligand LA-1-1 (LA-1-1Me)-

[Chem. 50]

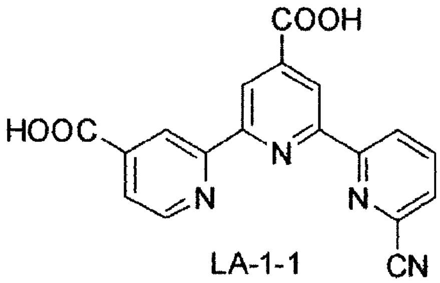

LA-1-1

5 g of 2-bromo-6-cyanopyridine was subjected to the aforementioned stannylation reaction, thereby obtaining stannylated LA-1-1A. Furthermore, 2 g of dimethyl 6-bromo-2,2'-bipyridine-4,4'-dicarboxylate and the stannylated LA-1-1A were subjected to the aforementioned Stille coupling, thereby obtaining LA-1-1Me as a dimethyl ester of LA-1-1.

-Synthesis of dimethyl ester of ligand LA-1-5 (LA-l-5Me)-

LA-1-5Me as a dimethyl ester of LA-1-5 was obtained in the same manner as in Synthesis of LA-1-1Me, except that 5 g of 2-bromo-6-cyanopyridine as a raw material in Synthesis of LA-1-1Me was replaced with equimolar 2-bromo-3,5-dichloropyridine.

-Synthesis of dimethyl ester of ligand LA-1-16 (LA-1-16Me)-

LA-1-16Me as a dimethyl ester of LA-1-16 was obtained in the same manner as in Synthesis of LA-1-1Me, except that 5 g of 2-bromo-6-cyanopyridine as a raw material in Synthesis of LA-1-1Me was replaced with equimolar 2-bromo-5-trifluoromethylpyridine.

-Synthesis of dimethyl ester of ligand LA-1-10 (LA-1-10Me)-

LA-1-10Me as a dimethyl ester of LA-1-10 was obtained in the same manner as in Synthesis of LA-1-1Me, except that 5 g of 2-bromo-6-cyanopyridine in Synthesis of LA-1-1Me was replaced with equimolar 2-bromo-5-methylsulfonylpyridine.

-Synthesis of dimethyl ester of ligand LA-1-3 (LA-1-3Me)-

LA-1-3Me as a dimethyl ester of LA-1-3 was obtained in the same manner as in Synthesis of LA-1-1Me, except that 5 g of 2-bromo-6-cyanopyridine in Synthesis of LA-1-1Me was replaced with equimolar 2-bromo-5-nitropyridine.

-Synthesis of dimethyl ester of ligand LA-1-4 (LA-1-4Me)-

LA-1-4Me as a dimethyl ester of LA-1-4 was obtained in the same manner as in Synthesis of LA-1-1Me, except that 5 g of 2-bromo-6-cyanopyridine in Synthesis of LA-1-1Me was replaced with equimolar 2-bromo-6-acetylpyridine.

-Synthesis of dimethyl ester of ligand LA-1-24 (LA-1-24Me)-

LA-1-24Me as a dimethyl ester of LA-1-24 was obtained in the same manner as in Synthesis of LA-1-1Me, except that 5 g of 2-bromo-6-cyanopyridine in Synthesis of LA-1-1Me was replaced with equimolar 2-bromo-5-fluoropyridine.

-Synthesis of ligand LD-1-1-

5 g of 6-acetyl-2,2'-bipyridine and trifluoroacetic acid ethyl ester in a molar quantity 1.2 times greater than that of the above compound were dissolved in 50 ml of tetrahydrofuran, and the resultant was cooled to 0°C. To the resultant, a sodium methoxide in methanol solution in a molar quantity 1.0 time greater than that of the above compound was added dropwise at 0°C, and the resultant was heated for 12 hours under reflux. 50 ml of toluene was added to the obtained reaction solution, and the resultant was washed with water and concentrated. Thereafter, the resultant was subjected to separation purification by using the aforementioned fractionation column chromatography instrument and eluent. By concentrating a target fraction, LD-1-1A as an intermediate of LD-1 was obtained. To the obtained LD-1-1A, an aqueous hydrazine solution at a moar ratio of 1.1 with respect to the LD-1-1A and 50 ml of ethanol were added, and the resultant was heated for 12 hours under reflux. After the reaction liquid was cooled, water was added thereto, and the resultant was subjected to extraction using methylene chloride and concentrated. Thereafter, the resultant was purified in the same manner as described above by using the fractionation column chromatography instrument, thereby obtaining a ligand LD-1-1.

-Synthesis of ligand LD-2-1-

A ligand LD-2-1 was obtained in the same manner as in Synthesis of LD-1-1, except that 5g of 6-acetyl-2,2'-bipyridine in Synthesis of LD-1-1 was replaced with 2,6-diacetylpyridine in a molar quantity 0.5 times greater than that of the above compound.

-Synthesis of ligand LD-2-3-

5 g of 4-chloro-2,6-pyridine dicarboxylic acid methyl ester and potassium t-butoxide in a molar quantity 2.1 times greater than that of the above compound were added to 50 ml of trifluoroacetic acid ethyl ester, and the resultant was heated for 5 hours under reflux. Thereafter, 50 ml of diluted hydrochloric acid was added thereto. The resultant was heated for 5 hours under reflux and cooled to room temperature. Then the resultant was washed with water and concentrated. The obtained crude product was subjected to separation purification by using the aforementioned fractionation column chromatography instrument and eluent. By concentrating a target fraction, LD-2-3A as an intermediate of LD-2-3 was obtained. To the obtained LD-2-3A, an aqueous hydrazine solution in a molar quantity 1.1 times greater than that of LD-2-3A and 50 ml of ethanol were added, and the resultant was heated for 12 hours under reflux. After the reaction liquid was cooled, water was added thereto, and the resultant was subjected to extraction using methylene chloride and concentrated. The obtained crude product was then purified in the same manner as described above by using the fractionation column chromatography instrument, thereby obtaining an intermediate LD-2-3B. 2g of the obtained LD-2-3B was subjected to the aforementioned stannylation reaction, thereby obtaining a stannylated product. 1g of the obtained stannylated product and 2-bromo-5-n-hexylthiophene in a molar quantity 1.3 times greater than that of the stannylated product were subjected to the aforementioned Stille coupling, thereby obtaining LD-2-3.

-Synthesis of ligand LD-2-5-

A ligand LD-2-5 was obtained in the same manner as in Synthesis of LD-2-3, except that 2-bromo-5-n-hexylthiophene in Synthesis of LD-2-3 was replaced with equimolar 1-bromo-4-n-octyloxybenzene.

-Synthesis of ligand LD-3-1-

1.5 g of 1,4-di(2-pyridyl)butane-1,4-dione and 5 g of ammonium acetate were heated and stirred for 2 hours at 125°C in nitrogen, and the resultant was cooled to room temperature. Thereafter, the resultant was diluted with methylene

chloride, washed with water, and concentrated, and the residue was purified in the same manner as described above by using the fractionation column chromatography instrument, thereby obtaining a ligand LD-3-1.

-Synthesis of ligand LD-3-2-

2g of 3,5-bipyridin-2-yl-[1,2,4]triazol-4-ylamine and nitric acid in a molar quantity 1.2 times greater than that of the above compound were added to 50 ml of water, and the resultant was cooled to 0°C. To the resultant, an aqueous sodium nitrate solution in a molar quantity 1.1 times greater than that of the resultant was added dropwise. Thereafter, the resultant was stirred for 30 minutes, and then heated for 1 hour under reflux. After being cooled, the resultant was diluted with methylene chloride, washed with water, and concentrated, and the residue was purified in the same manner as described above by using a fractionation column chromatography instrument, thereby obtaining a ligand LD-3-2.

-Synthesis of ligand LD-3-15-

5 g of 1,3-diacetylbenzene and potassium t-butoxide in a molar quantity 2.1 times greater than that of the above compound were added to 50 ml of trifluoroacetic acid ethyl ester, and the resultant was heated for 5 hours under reflux. Thereafter, 50 ml of diluted hydrochloric acid was added thereto. The resultant was heated for 5 hours under reflux and cooled to room temperature. Then the resultant was washed with water and concentrated. The obtained crude product was subjected to separation purification by using the aforementioned fractionation column chromatography instrument and eluent. By concentrating a target fraction, LD-3-15A as an intermediate of LD-3-15 was obtained. Herein, to the obtained LD-3-15A, an aqueous hydrazine solution in a molar quantity 1.1 times greater than that of LD-3-15A and 50 ml of ethanol were added, and the resultant was heated for 12 hours under reflux. After being cooled, the reaction liquid was purified in the same manner as described above by using the fractionation column chromatography instrument, thereby obtaining a lagnd LD-3-15.

-Synthesis of ligand LD-6-1-

A ligand LD-6-1 was obtained in the same manner as in Synthesis of LD-1-1, except that 5 g of 6-acetyl-2,2'-bipyridine in Synthesis of ligand LD-1-1 was replaced with equimolar 2-acetylpyridine.

-Synthesis of ligand LD-6-10-

A ligand LD-6-10 was obtained in the same manner as in Synthesis of LD-2-3, except that 5 g of 4-chloro-2,6-pyridinedicarboxylic acid methyl ester in Synthesis of ligand LD-2-3 was replaced with 4-bromo-2-pyridinecarboxylic acid methyl ester in a molar quantity 2 times greater than that of the above compound.

-Synthesis of ligand LD-6-13-

A ligand LD-6-13 was obtained in the same manner as in Synthesis of LD-2-3, except that, in Synthesis of ligand LD-2-3, 5 g of 4-chloro-2,6-pyridinedicarboxylic acid methyl ester was replaced with 4-bromo-2-pyridinecarboxylic acid methyl ester in a molar quantity 2 times greater than that of the above compound, and 2-bromo-5-n-hexylthiophene was replaced with equimolar 1-bromo-4-n-octyloxybenzene.

(Purification and identification of complx)

The complexes synthesized as below were purified by using a fractionation column chromatography instrument (AI-580 manufactured by YAMAZEN CORPORATION) and a mixed solvent consisting of n-hexane, ethyl acetate, and methanol as an eluent, in a state in which the concentration gradient thereof was being controlled. By concentrating a target fraction, a purified complex was obtained. The complex was subjected to 1H-NMR spectrometry and mass spectrometery, and a peak corresponding to the molecular weight thereof was observed, thereby identifying the complex.

-Synthesis of $Ru^{II}$(LA-1-1)(LD-1-1)Cl-

To 0.5 g of ruthenium (III) chloride and N,N-dimethylacetamide, the ligand LA-1-1Me in a molar quantity 1 time greater than that of the above compounds were added, and the resultant was heated and stirred for 3 hours at 80°C in nitrogen in a state of being protected from light. Thereafter, to the resultant, the equimolar ligand LD-1-1 and tributylamine in a molar quantity 10 times greater than that of the resultant were added, and the resultant was heated and stirred for 8 hours at 140°C in nitrogen in a state of being protected from light. The reaction liquid was diluted with methylene

chloride, washed with purified water, and concentrated. Then the residue was dispersed in 20 ml of methanol, and an aqueous solution of 1 N sodium hydroxide in a molar quantity 10 times greater than that of the resultant was added thereto, and the resultant was stirred overnight at room temperature. To the solution, a 1% aqueous hydrochloric acid solution was added dropwise until crystals were precipitated. The obtained solids were separated by filtration and then purified by the aforementioned method, thereby obtaining $Ru^{II}$(LA-1-1)(LD-1-1)Cl.

-Synthesis of $Ru^{II}$(LA-1-1)(LD-2-1)-

To 0.5 g of ruthenium (III) chloride and N,N-dimethylacetamide, the ligand LA-1-1Me in a molar quantity 1 time greater than that of the above compounds were added, and the resultant was heated and stirred for 3 hours at 80°C in nitrogen in a state of being protected from light. Thereafter, to the resultant, the equimolar ligand LD-2-1 and tributylamine in a molar quantity 10 times greater than that of the resultant were added, and the resultant was heated and stirred for 8 hours at 140°C in nitrogen in a state of being protected from light. The reaction liquid was diluted with methylene chloride, washed with purified water, and concentrated. Then the residue was dispersed in 20 ml of methanol, and an aqueous solution of 1 N sodium hydroxide in a molar quantity 10 times greater than that of the resultant was added thereto, and the resultant was stirred overnight at room temperature. To the solution, a 1% aqueous trifluoroacetic acid solution was added dropwise until crystals were precipitated. The obtained solids were separated by filtration and then purified by the aforementioned method, thereby obtaining $Ru^{II}$(LA-1-1)(LD-2-1).

-Synthesis of $Ru^{II}$(LA-1-5)(LD-2-1)-

$Ru^{II}$(LA-1-5)(LD-2-1) was synthesized in the same manner as in Synthesis of $Ru^{II}$(LA-1-1)(LD-2-1), except that LA-1-1Me in Synthesis of $Ru^{II}$(LA-1-1)(LD-2-1) was replaced with LA-1-5Me.

-Synthesis of $Ru^{II}$(LA-1-16)(LD-2-1)-

$Ru^{II}$(LA-1-16)(LD-2-1) was synthesized in the same manner as in Synthesis of $Ru^{II}$(LA-1-1)(LD-2-1), except that LA-1-1Me in Synthesis of $Ru^{II}$(LA-1-1)(LD-2-1) was replaced with LA-1-16Me.

-Synthesis of $Ru^{II}$(LA-1-10)(LD-2-1)-

$Ru^{II}$(LA-1-10)(LD-2-1) was synthesized in the same manner as in Synthesis of $Ru^{II}$(LA-1-1)(LD-2-1), except that LA-1-1Me in Synthesis of $Ru^{II}$(LA-1-1)(LD-2-1) was replaced with LA-1-10Me.

-Synthesis of $Ru^{II}$(LA-1-3)(LD-2-1)-

$Ru^{II}$(LA-1-3)(LD-2-1) was synthesized in the same manner as in Synthesis of Ru(LA-1-1)(LD-2-1), except that LA-1-1Me in Synthesis of $Ru^{II}$(LA-1-1)(LD-2-1) was replaced with LA-1-3Me.

-Synthesis of $Ru^{II}$(LA-1-1)(LD-2-3)-

$Ru^{II}$(LA-1-1)(LD-2-3) was synthesized in the same manner as in Synthesis of Ru(LA-1-1)(LD-2-1), except that LD-2-1 in Synthesis of $Ru^{II}$(LA-1-1)(LD-2-1) was replaced with LD-2-3.

-Synthesis of $Ru^{II}$(LA-1-1)(LD-2-5)-

$Ru^{II}$(LA-1-1)(LD-2-5) was synthesized in the same manner as in Synthesis of Ru(LA-1-1)(LD-2-1), except that LD-2-1 in Synthesis of $Ru^{II}$(LA-1-1)(LD-2-1) was replaced with LD-2-5.

-Synthesis of $Ru^{II}$(LA-1-1)(LD-3-1)Cl-

$Ru^{II}$(LA-1-1)(LD-3-1)Cl was synthesized in the same manner as in Synthesis of Ru(LA-1-1)(LD-1-1)Cl, except that LD-1-1 in Synthesis of Ru(LA-1-1)(LD-1-1)Cl was replaced with LD-3-1.

-Synthesis of $Ru^{II}$(LA-1-4)(LD-3-2)Cl-

$Ru^{II}$(LA-1-4)(LD-3-2)Cl was obtained in the same manner as in Synthesis of Ru(LA-1-1)(LD-1-1)Cl, except that, in Synthesis of $Ru^{II}$(LA-1-1)(LD-1-1)Cl, LA-1-1Me was replaced with LA-1-4Me, and LD-1-1 was replaced with LD-3-2.

-Synthesis of $Ru^{II}$(LA-1-19)(LD-3-15)$(n\text{-}Bu)_4$N-

To 0.5 g of ruthenium (III) chloride and N,N-dimethylacetamide, the ligand LA-1-24Me in a molar quantity 1 time greater than that of the above compounds were added, and the resultant was heated and stirred overnight at 70°C in nitrogen in a state of being protected from light. Thereafter, to the resultant, the equimolar ligand LD-3-15 and tributylamine in a molar quantity 10 times greater than that of the resultant were added, and the resultant was heated and stirred for 8 hours at 140°C in nitrogen in a state of being protected from light. During the reaction, a small amount of lithium diisopropylamine was added thereto. The reaction liquid was diluted with methylene chloride, washed with purified water, and concentrated. Then the residue was dispersed in 20 ml of methanol, and a 10% tetrabutylammonium hydroxide methanol solution in a molar quantity 10 times greater than that of the resultant was added thereto, and the resultant was stirred overnight at room temperature. The solution was concentrated, and the obtained solids were separated by filtration and purified in the aforementioned manner, thereby obtaining $Ru^{II}$(LA-1-19)(LD-3-15)$(n\text{-}Bu)_4N^+$.

-Synthesis of $Ru^{II}$(LA-1-1)(LD-6-1)(NCS)-

To 0.5 g of ruthenium (III) chloride and N,N-dimethylacetamide, the ligand LA-1-1Me in a molar quantity 1 time greater than that of the above compounds were added, and the resultant was heated and stirred for 4 hours at 80°C in nitrogen in a state of being protected from light. Thereafter, to the resultant, the equimolar ligand LD-6-1 and tributylamine in a molar quantity 10 times greater than that of the resultant were added, and the resultant was heated and stirred overnight at 140°C in nitrogen in a state of being protected from light. Moreover, potassium thiocyanate in a molar quantity 50 times greater than that of the resultant was added thereto, and the resultant was heated and stirred for 8 hours at 115°C. After being cooled to room temperature, the reaction liquid was diluted with methylene chloride, washed with pure water, and concentrated. Then the residue was dispersed in 20 ml of methanol, an aqueous solution of 1 N sodium hydroxide in a molar quantity 10 times greater than that of the resultant was added thereto, and the resultant was stirred overnight at room temperature. To the solution, diluted nitric acid was added until crystals were precipitated. The obtained solids were separated by filtration and then purified by the aforementioned method, thereby obtaining $Ru^{II}$(LA-1-1)(LD-6-1)(NCS).

-Synthesis of $Ru^{II}$(LA-1-1)(LD-6-10)(NCS)-

$Ru^{II}$(LA-1-1)(LD-6-10)(NCS) was synthesized in the same manner as in Synthesis of Ru(LA-1-1)(LD-6-1)(NCS), except that LD-6-1 in Synthesis of $Ru^{II}$(LA-1-1)(LD-6-1)(NCS), LD-6-1 was replaced with LD-6-10.

-Synthesis of $Ru^{II}$(LA-1-1)(LD-6-13)(NCS)-

$Ru^{II}$(LA-1-1)(LD-6-13)(NCS) was synthesized in the same manner as in Ru (LA-1-1)(LD-6-1)(NCS), except that LD-6-1 in Synthesis of $Ru^{II}$(LA-1-1)(LD-6-1)(NCS), LD-6-1 was replaced with LD-6-13.

<Example 1>

Various pastes for forming a semiconductor layer or a light-scattering layer of a semiconductor electrode constituting a photoelectrode were prepared. By using the pastes, dye-sensitized solar cells were prepared.

(Preparation of paste)

-Paste A-

Spherical $TiO_2$ particles (anatase; mean particle size: 25 nm; hereinafter, referred to as "spherical $TiO_2$ particles A") were added to a nitric acid solution, and the solution was stirred, thereby preparing titania slurry. Then a cellulose-based binder was added as a thickener to the titania slurry, and the resultant was kneaded, thereby preparing a paste A.

-Paste 1-

The spherical $TiO_2$ particles A and spherical $TiO_2$ particles (anatase; mean particle size: 200 nm; hereinafter, referred to as "spherical $TiO_2$ particles B) were added to a nitric acid solution, and the solution was stirred, thereby preparing titania slurry. Then a cellulose-based binder was added as a thickener to the titania slurry, and the resultant was kneaded, thereby preparing a paste 1 (mass of $TiO_2$ particles A:mass of $TiO_2$ particles B = 30:70).

-Paste 2-

The paste A was mixed with rod-shaped $TiO_2$ particles (anatase; diameter; 100 nm; aspect ratio: 5; hereinafter, referred to as "rod-shaped $TiO_2$ particles C), thereby preparing a paste 2 (mass of rod-shaped $TiO_2$ particles:mass of paste A = 30:70).

(Preparation of dye-sensitized solar cell)

According to the following procedure, a photoelectrode was prepared which had the same constitution as the photoelectrode 12 shown in Fig. 5 described in JP2002-289274A. Furthermore, by using the photoelectrode, a 10 mm × 10 mm dye-sensitized solar cell 1 was prepared which had the same constitution as the dye-sensitized solar cell 20 except for the photoelectrode shown in Fig. 3 of the same document. The specific constitution thereof is shown in Fig. 2. In Fig. 2, "41" indicates a transparent electrode, "42" indicates a semiconductor electrode, "43" indicates a transparent conductive film, "44" indicates a substrate, "45" indicates a semiconductor layer, "46" indicates a light-scattering layer, "40" indicates a photoelectrode, "20" indicates a dye-sensitized solar cell, "CE" indicates a counter electrode, "E" indicates an electrolyte, and "S" indicates a spacer.

A fluorine-doped $SnO_2$ conductive film (film thickness: 500 nm) was formed on a glass substrate, thereby preparing a transparent electrode. Thereafter, the paste 1 was printed on the $SnO_2$ conductive film by screen printing and then dried. Then the resultant was calcined under the conditions of 450°C under an atmosphere. In addition, the screen printing and calcining were repeated twice by using the paste 1, and the screen printing and calcining were repeated by using the paste 2. In this way, a semiconductor electrode (area of light-receiving surface: 10 mm × 10 mm; layer thickness: 10 μm, thickness of the semiconductor layer (resulting from paste 1): 6 μm; thickness of the light-scattering layer (resulting from paste 2): 2 μm; amount of the rod-shaped $TiO_2$ particles C contained in the light-scattering layer: 30% by mass) having the same constitution as the semiconductor electrode 42 shown in Fig. 2 was formed on the $SnO_2$ conductive film.

Thereafter, the metal complex dye used in the present invention was adsorbed onto the semiconductor electrode. A mixture containing anhydrous t-butanol, which was dehydrated over magnesium ethoxide, and dimethylformamide at a ratio of 1:1 (volume ratio) was used as a solvent, and the metal complex dyes listed in the following Table 1 were dissolved in the solvent such that the concentration became $3 \times 10^{-4}$ mol/L. Furthermore, as a coadsorbent, an equimolar mixture of chenodeoxyglycholic acid and cholic acid was added to the solution, in an amount of 20 mol with respect to 1 mol of each of the metal complex dyes, thereby preparing dye solutions. As a result of measuring the water content in the dye solutions by Karl Fischer titration, it was confirmed that the water content was less than 0.01% by mass. Thereafter, the semiconductor electrode was dipped in each of the dye solutions under conditions of 10 hours at 40°C, pulled up, and then dried at 50°C. In this way, photoelectrodes 40 were completed in which the dye was adsorbed onto the semiconductor electrode in a proportion of about $2 \times 10^{-7}$ mol/cm$^2$.

Subsequently, as a counter electrode, a platinum electrode (thickness of thin Pt film: 100 nm) having the same size and shape as the aforementioned photoelectrode was prepared, and as an electrolyte, a iodide-based redox propionitrile solution containing 0.1 M of iodide, 0.05 M of lithium iodide, and 0.25 M of 4-t-butylpyridine was prepared. Moreover, a spacer S (trade name: "Surlyn") manufactured by DuPont having a shape matching with the size of the semiconductor electrode was prepared. Thereafter, the photoelectrode 40 and the counter electrode CE were caused to face each other across the spacer S and subjected to thermocompression, and the inside thereof was filled with the electrolyte. The electrolyte inlet thereof was sealed with a cut thin glass plate, by using a resin XNR-5516 manufactured by Nagese ChemteX Corporation. The periphery of the cell was also sealed by using the aforementioned resin and cured. In this way, dye-sensitized solar cells (specimen Nos. 101 to 114 and c11 and c12) were completed.

<Test example>

The dye-sensitized solar cells prepared as above were subjected to the following test, and the performance thereof was evaluated.

(Evaluation 1) Evaluation of initial photoelectric conversion efficiency (initial characteristics)

For each of the dye-sensitized solar cells, a short-circuit current density (Jsc, unit: mA/cm$^2$), an open-circuit voltage (Voc, unit: v), and a fill factor (FF) were calculated, and the battery output was divided by incident energy, thereby measuring the photoelectric conversion efficiency (η(%)). In this test, a solar simulator (trade name: PEC-L12, manufactured by Peccell Technologies, Inc.) was used. The current-voltage characteristics were evaluated by using an I-V characteristic analyzer (trade name: PECK2400-N).

-Evaluation criteria of initial photoelectric conversion efficiency-

A: The photoelectric conversion efficiency is equal to or greater than 1.3 times the photoelectric conversion efficiency of c11.
B: The photoelectric conversion efficiency is equal to or greater than 1.1 times and less than 1.3 times the photoelectric conversion efficiency of c11.
C: The photoelectric conversion efficiency is less than 1.1 times the photoelectric conversion efficiency of c11.

(Evaluation 2) Durability test-1 (light fastness test)

The prepared dye-sensitized solar cells were subjected to a light fastness test by being put into a merry-go-round type light fastness tester (manufactured by Eagle Engineering Inc., Cell Tester III, equipped with WG320 filter manufactured by SCHOTT AG). The dye-sensitized solar cells having not yet been subjected to the light fastness test and the dye-sensitized solar cells left for 168 hours after the light fastness test were evaluated in terms of current. The decrease in a current value after the light fastness test was divided by a current value before the light fastness test, and the thus obtained value was used as a rate of deterioration for evaluation.

-Evaluation criteria-

A: The rate of deterioration is less than 0.60 times the rate of deterioration of c11.
B: The rate of deterioration is equal to or greater than 0.60 times and less than 0.90 times the rate of deterioration of c11.
C: The rate of deterioration is equal to or greater than 0.90 times the rate of deterioration of c11.

(Evaluation 3) Durability test-2 (heat resistance test)

The prepared dye-sensitized solar cells were subjected to a heat resistance test by being put into a thermostatic bath at 60°C. The dye-sensitized solar cells having not yet been subjected to the heat resistance test and the dye-sensitized solar cells left for 12 hours after the heat resistance test were evaluated in terms of current. The decrease in a current value after the heat resistance test was divided by a current value before the heat resistance test, and the thus obtained value was taken as a rate of deterioration.

-Evaluation criteria-

A: The rate of deterioration is less than 0.70 times the rate of deterioration of c11.
B: The rate of deterioration is equal to or greater than 0.70 times and less than 0.90 times the rate of deterioration of c11.
C: The rate of deterioration is equal to or greater than 0.90 times the rate of deterioration of c11.

The prepared dye-sensitized solar cells were repeatedly cooled and heated by being alternately put into a freezer at -20°C and then into a thermostatic bath at 40°C every two hours. In this way, a heat cycle test was performed. The dye-sensitized solar cells having not yet been subjected to the heat cycle test and the dye-sensitized solar cell left for 24 hours after the heat cycle test were evaluated in terms of current. The decrease in a current value after the heat cycle test was divided by a current value before the heat cycle test, and the obtained value was taken as a rate of deterioration.

-Evaluation criteria-

A: The rate of deterioration is less than 0.80 times the rate of deterioration of c11.

B: The rate of deterioration is equal to or greater than 0.80 times and less than 0.95 times the rate of deterioration of c11.

C: The rate of deterioration is equal to or greater than 0.95 times the rate of deterioration of c11.

The results of the Evaluations 1 to 4 are shown in the following Table 1.

[Table 1]

| Specimen No. | Metal complex dye | Durability test-1 | Durability test-2 | Durability test-3 | Initial characteristics | Note |
|---|---|---|---|---|---|---|
| 101 | $Ru^{II}$(LA-1-1)(LD-1-1)Cl | A | B | B | B | Present invention |
| 102 | $Ru^{II}$(LA-1-1)(LD-2-1) | A | B | B | B | Present invention |
| 103 | $Ru^{II}$(LA-1-5)(LD-2-1) | A | B | B | B | Present invention |
| 104 | $Ru^{II}$(LA-1-16)(LD-2-1) | A | B | B | B | Present invention |
| 105 | $Ru^{II}$(LA-1-10)(LD-2-1) | A | B | B | B | Present invention |
| 106 | $Ru^{II}$(LA-1-3)(LD-2-1) | A | B | B | B | Present invention |
| 107 | $Ru^{II}$(LA-1-1)(LD-2-3) | A | B | B | B | Present invention |
| 108 | $Ru^{II}$(LA-1-1)(LD-2-5) | A | B | B | B | Present invention |
| 109 | Ru(LA-1-1)(LD-3-1)Cl | B | B | A | B | Present invention |
| 110 | $Ru^{II}$(LA-1-4)(LD-3-2)Cl | B | B | A | B | Present invention |
| 111 | $Ru^{II}$(LA-1-19)(LD-3-15)$(n\text{-}Bu)_4N^+$ | B | A | B | B | Present invention |
| 112 | $Ru^{II}$(LA-1-1)(LD-6-1)(NCS) | B | B | B | A | Present invention |
| 113 | $Ru^{II}$(LA-1-1)(LD-6-10)(NCS) | B | B | B | A | Present invention |
| 114 | $Ru^{II}$(LA-1-1)(LD-6-13)(NCS) | B | B | B | A | Present invention |
| e11 | Comparative compound (1) | C | C | C | C | Comparative example |
| c12 | Comparative compound (2) | C | C | C | C | Comparative example |
| | | | | | | |

[Chem. 51]

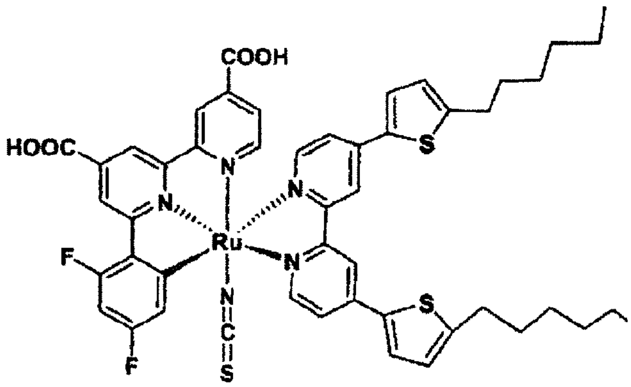

Comparative compound (1)

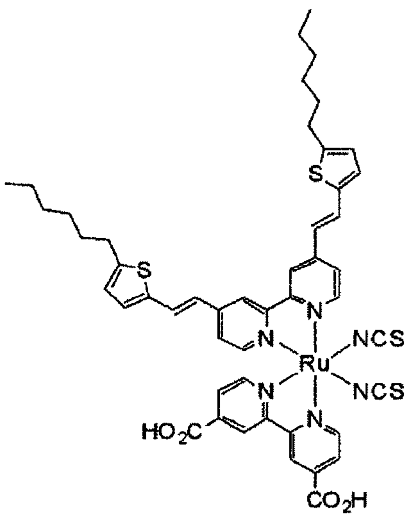

Comparative compound (2)

As is evident from Table 1, the photoelectric conversion element and the dye-sensitized solar cell of the present invention has an excellent photoelectric conversion efficiency, and exhibits excellent durability under various conditions.

## Claims

**1.** A photoelectric conversion element comprising,
a conductive substrate;
a photosensitive layer containing an electrolyte;
a charge transfer layer containing an electrolyte; and
a counter electrode,
wherein the photosensitive layer contains semiconductor particles loaded with a metal complex dye represented by the following Formula (1),

M(LD)(LA)(X)m·CI Formula (1)

in Formula (1), M represents a divalent or trivalent metal ion; LD represents a bidentate or tridentate ligand having an aryl group or an heterocyclic group; the ligand has 1 to 3 atoms that are coordinated with M by becoming an anion; LA represents a tridentate ligand represented by the following Formula (2); X represents a monodentate ligand; CI represents a counter ion necessary for neutralizing a charge; and m represents 0 or 1,

Formula (2)

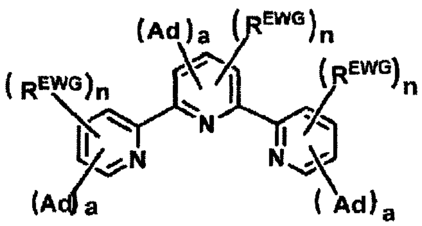

in Formula (2), Ad represents either an adsorptive group selected from among -COOH, $-SO_3H$, $-PO_3H_2$, -OH, -SH, and salts of these or a group having the adsorptive group; a represents an integer of 0 to 3; all a do not represent

0 at the same time; $R^{EWG}$ represents an electron-withdrawing group selected from among $-NO_2$, $-SO_2R$, $-SO_3R$, -F, -Cl, -Br, -I, -CN, -COR, and a perfluoroalkyl group; R represents a group selected from among an alkyl group, an aryl group, an alkenyl group, an alkynyl group, a heterocyclic group, and an amino group; n represents an integer of equal to or greater than 0; and all n do not represent 0 at the same time.

**2.** The photoelectric conversion element according to claim 1,
wherein LD in Formula (1) is a bidentate ligand represented by any of the following Formulae (2L-1) to (2L-4),

Formula (2L-1)

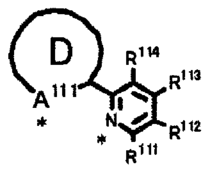

Formula (2L-2)

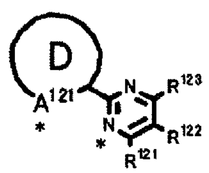

Formula (2L-3)

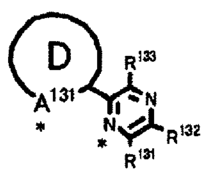

Formula (2L-4)

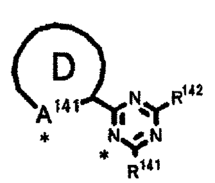

in Formulae (2L-1) to (2L-4), * represents a coordination position in which the ligand is coordinated with M. Each of $A^{111}$, $A^{121}$, $A^{131}$, and $A^{141}$ represents an anionic coordinating atom that is any of a nitrogen atom or a carbon atom, a ring D represents an aromatic hydrocarbon ring or a heterocyclic ring, each of $R^{111}$ to $R^{114}$, $R^{121}$ to $R^{123}$, $R^{131}$ to $R^{133}$, and $R^{141}$ and $R^{142}$ independently represents a hydrogen atom or a substituent.

**3.** The photoelectric conversion element according to claim 1,
wherein LD in Formula (1) represents a tridentate ligand represented by any of the following Formulae (3L-1) to (3L-6),

Formula (3L-1)

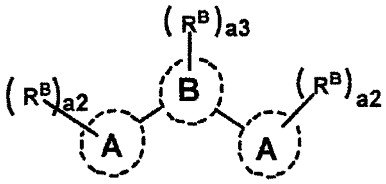

Formula (3L-2)

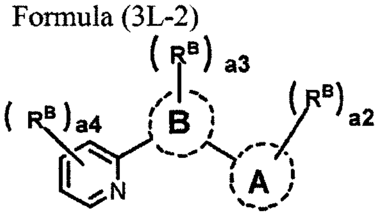

Formula (3L-3)

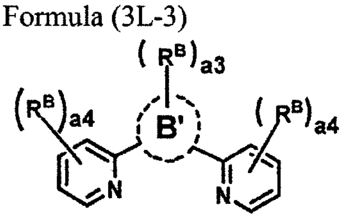

Formula (3L-4)

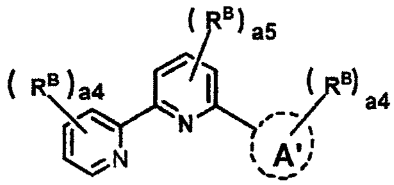

Formula (3L-5)

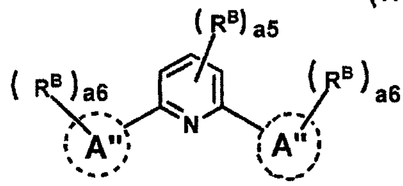

Formula (3L-6)

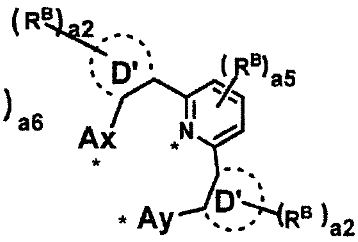

in Formulae (3L-1) to (3L-6), $R^B$ represents a substituent; each of a2 and a3 independently represents an integer of equal to or greater than 0; a4 represents an integer of 0 to 4; a5 represents an integer of 0 to 3; and a6 represents an integer of 0 to 2,
a ring A represents a ring structure selected from among a pyrimidine ring, a pyrazine ring, a pyridazine ring, a triazine ring, a pyridine ring that is coordinated with M through a carbon atom, a thiophene ring that is coordinated with M through a carbon atom, a furan ring that is coordinated with M through a carbon atom, an imidazole ring, an oxazole ring, a thiazole ring, an oxadiazole ring, a thiadiazole ring, an isoxazole ring, an isothiazole ring, a triazole ring, a pyrazole ring, a pyrrole ring, and a benzene ring; a ring A' represents a ring structure selected from among a pyrimidine ring that is coordinated with M through a carbon atom, a pyrazine ring that is coordinated with M through a carbon atom, a pyridazine ring that is coordinated with M through a carbon atom, a pyridine ring that is coordinated with M through a carbon atom, a thiophene ring that is coordinated with M through a carbon atom, a furan ring that is coordinated with M through a carbon atom, an imidazole ring, an oxazole ring that is coordinated with M through a carbon atom, a thiazole ring that is coordinated with M through a carbon atom, an oxadiazole ring that is coordinated

with M through a carbon atom, a thiadiazole ring that is coordinated with M through a carbon atom, an isoxazole ring that is coordinated with M through a carbon atom, an isothiazole ring that is coordinated with M through a carbon atom, a triazole ring, a pyrazole ring, a pyrrole ring, and a benzene ring; and a ring A" represents a pyrazole ring, a ring B represents a ring structure selected from among a pyrimidine ring, a triazine ring, an imidazole ring, an oxazole ring, a thiazole ring, an oxadiazole ring, a thiadiazole ring, a triazole ring, a pyrazole ring, a pyrrole ring, and a benzene ring; a ring B' represents a ring structure selected from among a pyrimidine ring that is coordinated with M through a carbon atom, an imidazole ring, an oxazole ring that is coordinated with M through a carbon atom, a thiazole ring that is coordinated with M through a carbon atom, an oxadiazole ring that is coordinated with M through a carbon atom, a thiadiazole ring that is coordinated with M through a carbon atom, a triazole ring, a pyrazole ring, a pyrrole ring, and a benzene ring,
a ring D' represents an aromatic hydrocarbon ring or a heterocyclic ring; each of Ax and Ay independently represents a nitrogen atom, an oxygen atom, or a sulfur atom; and at least one of Ax and Ay is an anion,
two ring As present in Formula (3L-1) may be the same as or different from each other; and two ring D's present in Formula (3L-6) may be the same as or different from each other.

**4.** The photoelectric conversion element according to any one of claims 1 to 3,
wherein in Formula (2), the adsorptive group or the group having the adsorptive group, and the electron-withdrawing group are present on different pyridine rings.

**5.** The photoelectric conversion element according to any one of claims 1 to 4,
wherein the group having the adsorptive group is a group selected from the group consisting of an alkyl group and an amino group, and the group has the adsorptive group as a substituent.

**6.** The photoelectric conversion element according to any one of claims 1 to 5,
wherein the adsorptive group is -COOH or a salt thereof.

**7.** The photoelectric conversion element according to any one of claims 1 to 6,
wherein M in Formula (1) is $Ru^{2+}$, $Fe^{2+}$, or $Os^{2+}$.

**8.** The photoelectric conversion element according to any one of claims 1 to 7,
wherein the semiconductor particles are loaded with a coadsorbent having at least one adsorptive group.

**9.** The photoelectric conversion element according to claim 8,
wherein the coadsorbent is represented by the following Formula (CA),

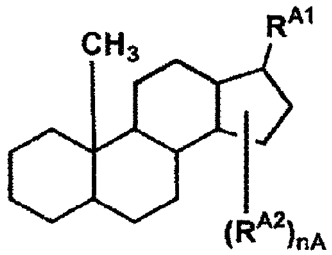

Formula (CA)

in Formula (CA), $R^{A1}$ represents a substituent having an adsorptive group; $R^{A2}$ represents a substituent; and nA represents an integer of equal to or greater than 0.

**10.** A dye-sensitized solar cell using the photoelectric conversion element according to any one of claims 1 to 9.

FIG. 1

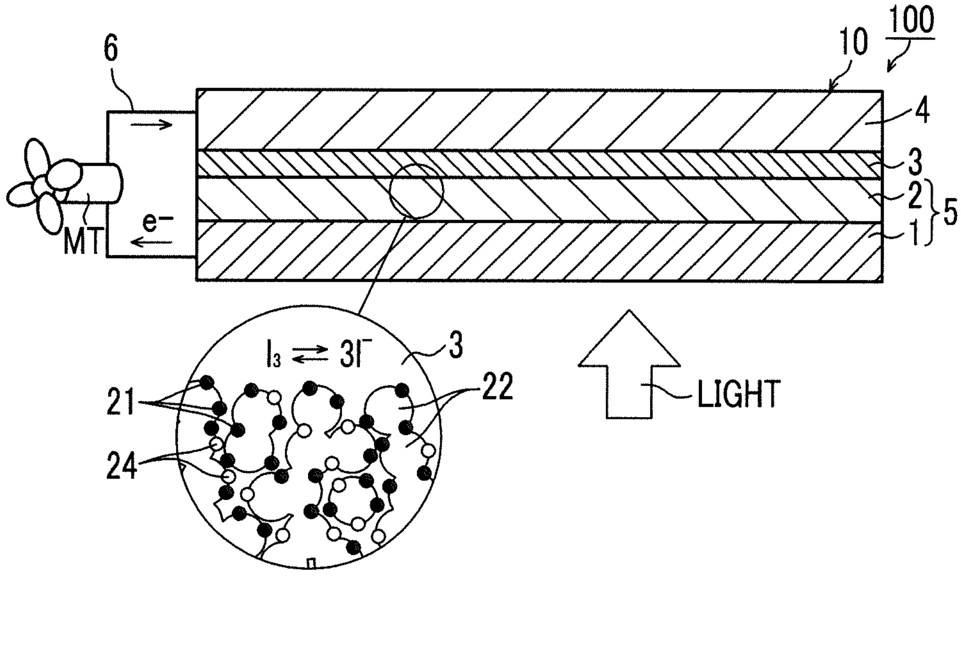

FIG. 2

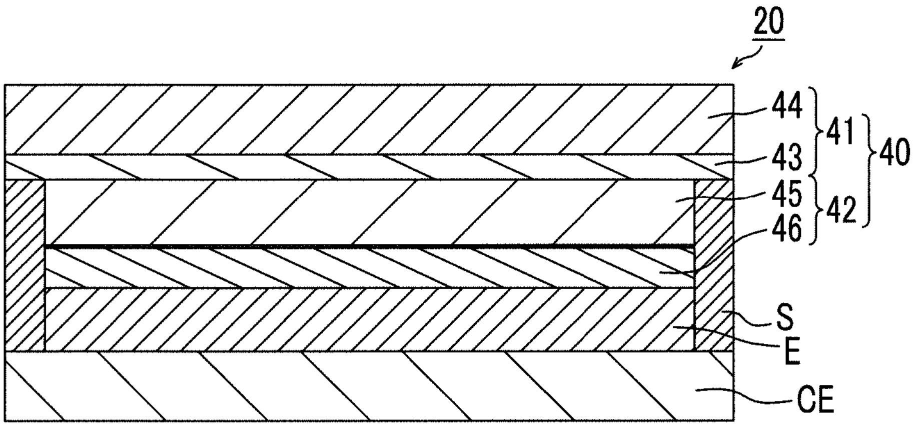

**INTERNATIONAL SEARCH REPORT**

International application No.
PCT/JP2013/074295

A. CLASSIFICATION OF SUBJECT MATTER
*H01M14/00*(2006.01)i, *C09B57/10*(2006.01)i, *C09B67/44*(2006.01)i, *H01L31/04*(2006.01)i

According to International Patent Classification (IPC) or to both national classification and IPC

B. FIELDS SEARCHED

Minimum documentation searched (classification system followed by classification symbols)
H01M14/00, C09B57/10, C09B67/44, H01L31/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
Scopus

C. DOCUMENTS CONSIDERED TO BE RELEVANT

| Category* | Citation of document, with indication, where appropriate, of the relevant passages | Relevant to claim No. |
|---|---|---|
| A | JP 2012-53983 A (Fujifilm Corp.), 15 March 2012 (15.03.2012), claims; paragraphs [0058] to [0060], [0146], [0147], [0168]; examples (Family: none) | 1-10 |
| A | US 2012/0073660 A1 (CHI et al.), 29 March 2012 (29.03.2012), entire text & US 2013/0018189 A1 & TW 201213309 A | 1-10 |
| A | Jen-Fu Yin et al., Structure optimization of ruthenium photosensitizers for efficient dye-sensitized solar cells - A goal toward a "bright" future, Coordination Chemistry Reviews, 2012.07.13, 256, 3008-3035, Available online 13 July 2012 | 1-10 |

☒ Further documents are listed in the continuation of Box C.　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>27 September, 2013 (27.09.13) | Date of mailing of the international search report<br>08 October, 2013 (08.10.13) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2013/074295

C (Continuation). DOCUMENTS CONSIDERED TO BE RELEVANT

| Category* | Citation of document, with indication, where appropriate, of the relevant passages | Relevant to claim No. |
|---|---|---|
| A | Anders Hagfeldt et al., Dye-Sensitized Solar Cells, Chemical reviews, 2010, Volume 110, 6595-6663 | 1-10 |
| P,X | WO 2013/088898 A1 (Fujifilm Corp.), 20 June 2013 (20.06.2013), claims; paragraphs [0131], [0174] to [0195], [0212] to [0276], [0285] to [0294]; examples (Family: none) | 1-10 |
| E,X<br>E,A | WO 2013/137221 A1 (Fujifilm Corp.), 19 September 2013 (19.09.2013), claims; paragraphs [0060] to [0065], [0074] to [0109], [0133] to [0137], [0155]; examples (Family: none) | 1,4-10<br>2,3 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012036237 A
- KR 1020120035696 A
- US 4927721 A
- US 4684537 A
- US 5084365 A
- US 5350644 A
- US 5463057 A
- US 5525440 A
- JP 7249790 A
- JP H07249790 A
- JP 2004220974 A
- JP 2008135197 A
- JP 7500630 A
- JP H07500630 A
- JP 2002512729 A
- JP 2001059062 A
- JP 2001006760 A
- JP 2001253894 A
- JP 2003212851 A
- WO 200791525 A
- JP 2001291534 A
- JP 2012012570 A
- JP 2013084594 A
- JP 11214730 A
- JP H11214730 A
- JP 2012144688 A
- JP 2012084503 A
- JP 2004063274 A
- JP 2005123033 A
- JP 2007287694 A
- JP 2008071648 A
- WO 2007119525 A
- JP 2005135902 A
- JP 2001160425 A
- JP 2003123859 A
- JP 2002260746 A
- WO 9518456 A
- JP 8259543 A
- JP H08259543 A
- JP 4260494 B
- JP 2004146425 A
- JP 2000340269 A
- JP 2002289274 A
- JP 2004152613 A
- JP 9027352 A
- JP H0927352 A
- JP 2000090989 A
- JP 2003217688 A
- JP 2002367686 A
- JP 2003323818 A
- JP 2001043907 A
- JP 2005085500 A
- JP 2004273272 A
- JP 2000323190 A
- JP 2000228234 A
- JP 2001266963 A
- JP 2001185244 A
- JP 2001525108 A
- JP 2001203377 A
- JP 2000100483 A
- JP 2001210390 A
- JP 2002280587 A
- JP 2001273937 A
- JP 2000285977 A
- JP 2001320068 A


### Non-patent literature cited in the description


- *Chemical Communications,* 2009, 5844-5846
- *Angew. Chem. Int. Ed.,* 2010, vol. 49, 1-5
- *Angew. Chem. Int. Ed.,* 2007, vol. 46, 8358
- *Electrochemistry,* 1997, vol. 65 (11), 923
- *Nature,* 2012, vol. 486, 487